(19) Europäisches Patentamt
European Patent Office
Office européen des brevets

(11) EP 1 058 552 B1

(12) EUROPEAN PATENT SPECIFICATION

(45) Date of publication and mention of the grant of the patent: **16.06.2004 Bulletin 2004/25**

(21) Application number: **98909028.7**

(22) Date of filing: **06.03.1998**

(51) Int Cl.[7]: **A61K 31/56**, C07J 41/00, A61K 31/57, A61K 31/575

(86) International application number: **PCT/US1998/004489**

(87) International publication number: **WO 1999/044616 (10.09.1999 Gazette 1999/36)**

# (54) STEROID DERIVED ANTIBIOTICS

ANTIBIOTISCHE STEROIDDERIVATE

DERIVES ANTIBIOTIQUES DE STEROIDES

(84) Designated Contracting States:
**AT BE CH DE DK ES FI FR GB GR IE IT LI LU MC NL PT SE**

(43) Date of publication of application:
**13.12.2000 Bulletin 2000/50**

(73) Proprietor: **BRIGHAM YOUNG UNIVERSITY
Provo, UT 84602-1231 (US)**

(72) Inventors:
- **SAVAGE, Paul, B.
Springville, UT 84663 (US)**
- **LI, Chunhong
Provo, UT 84601 (US)**

(74) Representative: **Stevens, Ian Edward et al
Eric Potter Clarkson,
Park View House,
58 The Ropewalk
Nottingham NG1 5DD (GB)**

(56) References cited:

| | |
|---|---|
| **WO-A-94/20520** | **WO-A-95/24415** |
| **WO-A1-95/19567** | **US-A- 3 519 714** |
| **US-A- 4 192 871** | **US-A- 5 834 453** |

- **JONES S R ET AL: "THE SYNTHESIS AND CHARACTERIZATION OF ANALOGS OF THE ANTIMICROBIAL COMPOUND SQUALAMINE: 6BETA-HYDROXY-3-AMINOSTERLS SYNTHESIZED FROM HOYDEOXYCHOLIC ACID" STEROIDS: STRUCTURE, FUNCTION, AND REGULATION,US,ELSEVIER SCIENCE PUBLISHERS, NEW YORK, NY, vol. 61, no. 10, 1 October 1996 (1996-10-01), pages 565-571, XP002062882 ISSN: 0039-128X**
- **NESTLER H.P., "Sequence-Selective Nonmacrocyclic Two-Armed Receptors for Peptides", MOLECULAR DIVERSITY, October 1996, Vol. 2, No. 1-2, pages 35-40, XP002910828**
- **CHENG Y. et al., "Sequence-Selective Peptide Binding with a Peptido-A,B-Trans-Steroidal Receptor Selected from an Encoded Combinatorial Receptor Library", JOURNAL OF THE AMERICAN CHEMICAL SOCIETY, February 1996, Vol. 118, No. 7, pages 1813-1814, XP002910829**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 058 552 B1

## Description

The invention relates to novel steroid derivatives and salts thereof and the processes and intermediates for their preparation.

Some compounds that associate strongly with the outer membrane of Gram-negative bacteria are known to disrupt the outer membrane and increase permeability. The increased permeability can lead directly to cell death or can increase the susceptibility of Gram-negative bacteria to other antibiotics. The best studied of this type of compound are the polymyxin antibiotics. For an example of a study involving the binding of polymyxin B to the primary constituent of the outer membrane of Gram-negative bacteria (lipid A) see: D. C. Morrison and D. M. Jacobs, *Binding of Polymixin B to The Lipid a Portion of Bacterial Lipopolysaccharides*, Immunochemistry 1976, vol. 13, 813-819. For an example of a study involving the binding of a polymyxin derivative to Gram-negative bacteria see: M. Vaara and P. Viljanen, *Binding of Polymyxin B Nonapeptide to Gram-negative Bacteria*, Antimicrobial Agents and Chemotherapy, 1985, vol. 27, 548-554.

Membranes of Gram-negative bacteria are semipermeable molecular "sieves" which restrict access of antibiotics and host defense molecules to their targets within the bacterial cell. Thus, cations and polycations which access the self-promoted uptake system are, by virtue of their ability to interact with and break down the outer membrane permeability barrier, capable of increasing the susceptibility of Gram-negative pathogenic bacteria to antibiotics and host defense molecules. Hancock and Wong demonstrated that a broad range of peptides could overcome the permeability barrier and coined the name "permeabilizers" to describe them (Hancock and Wong, Antimicrob. Agents Chemother., 26:48, 1984).

US 3, 519, 714 discloses gona-1,3,5 (10)-trienes, their synthesis and their hormonal effects. Jones et al, Steroids, 61 : 565-571, 1996 describes the synthesis and characterisation of analogs of the antimicrobial compound squalamine. The compounds are 6β-hydroxy-3-aminosterols synthesized from hyodeoxycholic acid.

SUMMARY OF THE INVENTION

In a first aspect, the present invention features compounds of the formula I

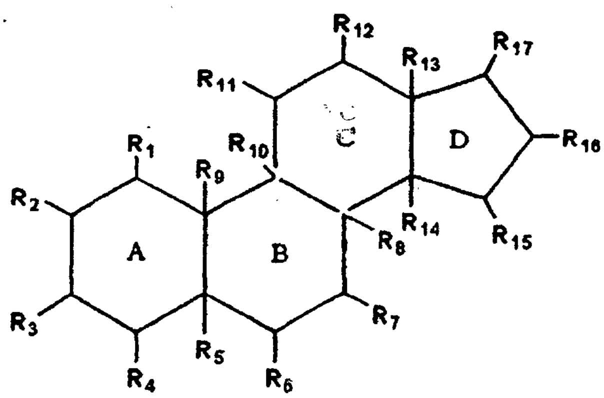

I

wherein:

fused rings A, B, C, and D are independently saturated or fully or partially unsaturated; and

$R_1$ through $R_4$, $R_6$, $R_7$, $R_{11}$, $R_{12}$, $R_{15}$, $R_{16}$, and $R_{17}$ is each independently selected from the group consisting of hydrogen, hydroxyl, (C1-C10) alkyl, (C1-C10) hydroxyalkyl, (C1-C10) alkyloxy-(C1-C10) alkyl, a substituted or unsubstituted (C1-C10) aminoalkyl wherein the substituents are selected from the group consisting of hydroxyl, protected hydroxyl, amino, protected amino, carboxy, protected carboxy, cyano, methylsulfonylamino, alkoxy, alkyl, aryl, N-benzyl, acyloxy, nitro, C1-C10 haloalkyl, C2-C6 alkenyl, C2-C6 alkynyl, oxo, a (C1-C10)alkyloxy-(C1-C10) alkyl linking group attached to a second steroid of formula I, (C1-C10) aminoalkyloxy, (C1-C10) aminoalkylcarboxy, (C1-C10) aminoalkylaminocarbonyl, H2N-HC(Q5)-C(O)-O-, H2N-HC(Q5)-C(O)-N(H)-, (C1-C10) azidoalkyloxy, (C1-C10) cyanoalkyloxy, P.G.-HN-CH(Q5)-C(O)-O-, (C1-C10) guanidinoalkyl oxy, and (C1-C10) guanidinoalkyl carboxy, where Q5 is H, P.G. is an amino protecting group, and

$R_5$, $R_8$, $R_9$, $R_{10}$, $R_{13}$, and $R_{14}$ is each independently:

deleted when one of fused rings A, B, C, or D is unsaturated so as to complete the valency of the carbon atom at that site, or

selected from the group consisting of hydrogen, hydroxyl, (C1-C10) alkyl, (C1-C10) hydroxyalkyl, (C1-C10) alkyloxy-(C1-C10) alkyl, a substituted or unsubstituted (C1-C10) aminoalkyl wherein the substituents are selected from the group consisting of hydroxyl, protected hydroxyl, amino, protected amino, carboxy, protected carboxy, cyano, methylsulfonylamino, alkoxy, alkyl, aryl, N-benzyl, acyloxy, nitro, C1-C10 haloalkyl, C2-C6 alkenyl, C2-C6 alkynyl, oxo, a (C1-C10)alkyloxy-(C1-C10)alkyl linking group attached to a second steroid of formula I, (C1-C10) aminoalkyloxy, (C1-C10) aminoalkylcarboxy, (C1-C10) aminoalkylaminocarbonyl, H2N-HC(Q5)-C(O)-O-, H2N-HC(Q5)-C(O)-N(H)-, (C1-C10) azidoalkyloxy, (C1-C10) cyanoalkyloxy, P.G. -HN-CH(Q5)-C(O)-O-, (C1-C10) guanidinoalkyloxy, and (C1-C10) guanidinoalkylcarboxy, where Q5 is H, P. G. is an amino protecting group, and

provided that at least two of $R_1$ through $R_{14}$ are independently selected from the group consisting of (C1-C10) aminoalkyloxy, (C1-C10) aminoalkylcarboxy, (C1-C10) aminoalkylaminocarbonyl, H2N-HC(Q5)-C(O)-O-, H2N-HC(Q5)-C(O)-N(H)-, (C1-C10) azidoalkyloxy, (C1-C10) cyanoalkyloxy, P.G.-HN-CH(Q5)-C(O)-O-, (C1-C10) guanidinoalkyloxy, and (C1-C10) guanidinoalkylcarboxy; or a pharmaceutically acceptable salt thereof. The compound has at least one of the features (a) to (c) as defined in Claim 1.

The term fused ring used herein can be heterocyclic or carbocyclic, preferably.

The term "saturated" used herein refers to the fused ring of formula I having each atom in the fused ring either hydrogenated or substituted such that the valency of each atom is filled.

The term "unsaturated" used herein refers to the fused ring of formula I where the valency of each atom of the fused ring may not be filled with hydrogen or other substituents. For example, adjacent carbon atoms in the fused ring can be doubly bound to each other. Unsaturation can also include deleting at least one of the following pairs and completing the valency of the ring carbon atoms at these deleted positions with a double bond; such as $R_5$ and $R_9$; $R_8$ and $R_{10}$; and $R_{13}$ and $R_{14}$.

The term "unsubstituted" used herein refers to a moiety having each atom hydrogenated such that the valency of each atom is filled.

The term "halo" used herein refers to a halogen atom such as fluorine, chlorine, bromine, or iodine.

Examples of amino acid side chains include but are not limited to H (glycine), methyl (alanine), - $(CH_2-(C=O)-NH_2$ (asparagine), $-CH_2-SH$ (cysteine), and $-CH(OH)CH_3$ (threonine).

An alkyl group is a branched or unbranched hydrocarbon that may be substituted or unsubstituted. Examples of branched alkyl groups include isopropyl, sec-butyl, isobutyl, tert-butyl, sec-pentyl, isopentyl, tert-pentyl, isohexyl. Substituted alkyl groups may have one, two, three or more substituents, which may be the same or different, each replacing a hydrogen atom. Substituents are halogen (e.g., F, Cl, Br, and I), hydroxyl, protected hydroxyl, amino, protected amino, carboxy, protected carboxy, cyano, methylsulfonylamino, alkoxy, acyloxy, nitro, and lower haloalkyl.

The term "substituted" used herein refers to moieties having one, two, three or more substituents, which may be the same or different, each replacing a hydrogen atom. Examples of substituents include but are not limited to halogen (e.g., F, Cl, Br, and I), hydroxyl, protected hydroxyl, amino, protected amino, carboxy, protected carboxy, cyano, methylsulfonylamino, alkoxy, alkyl, acryl, acyloxy, nitro, and lower haloalkyl.

An aryl group is a $C_{6-20}$ aromatic ring, wherein the ring is made of carbon atoms (e.g., $C_{6-14}$, $C_{6-10}$ aryl groups). Examples of haloalkyl include fluoromethyl, dichloromethyl, trifluoromethyl, 1,1-difluoroethyl, and 2,2-dibromoethyl.

Numerous amino-protecting groups are well-known to those in the art. In general, the species of protecting group is not critical, provided that it is stable to the conditions of any subsequent reaction(s) on other positions of the compound and can be removed at the appropriate point without adversely affecting the remainder of the molecule. In addition, a protecting group may be substituted for another after substantive synthetic transformations are complete. Clearly, where a compound differs from a compound disclosed herein only in that one or more protecting groups of the disclosed compound has been substituted with a different protecting group, that compound is within the invention. Further examples and conditions are found in T.W. Greene, *Protective Groups in Organic Chemistry*, (1st ed., 1981, 2nd ed., 1991).

The present invention also includes methods of synthesizing compounds of formula I of the first aspect of the invention where at least two of $R_1$ through $R_{14}$ are independently selected from the group consisting of a substituted or unsubstituted (C1-C10) aminoalkyloxy. The method includes the step of contacting a compound of formula IV,

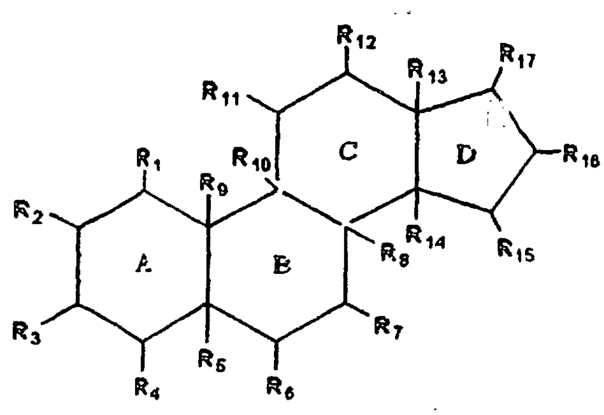

IV

where at least two of $R_1$ through $R_{14}$ are hydroxyl, and the remaining moieties on the fused rings A, B, C, and D are defined for formula I, with a protected amino acid to produce a protected amino acid compound of formula IV, wherein at least two of $R_1$ through $R_{14}$ are (P.G.-HN-HC(Q5)-C(O)-O-), where Q5 is H and P.G is an amino protecting group ; and removing the protecting group of the protected amino acid compound to form a compound of formula I.

Further aspects of the invention are defined in Claims 2 to 6.

The present invention also includes pharmaceutical compositions of matter that are useful as antibacterial agents, sensitizers of bacteria to other antibiotics and disrupters of bacterial membranes. The pharmaceutical compositions can be used to treat humans and animals having a bacterial infection. The pharmaceutical compositions can include an effective amount of the steroid derivative alone or in combination with other antibacterial agents.

Without wishing to be bound to any particular theory, the steroid derivatives act as bacteriostatic and bactericidal agents by binding to the outer cellular membrane of bacteria. The interaction between the steroid derivatives and the bacteria membrane disrupts the integrity of the cellular membrane and results in the death of the bacteria cell. In addition, compounds of the present invention also act to sensitize bacteria to other antibiotics. At concentrations of the steroid derivatives below the corresponding minimum bacteriostatic concentration, the derivatives cause bacteria to become more susceptible to other antibiotics by increasing the permeability of the outer membrane of the bacteria. Measurements used to quantitate the effects of the steroid derivatives on bacteria include: measurement of minimum inhibitory concentrations (MICs), measurement of minimum bactericidal concentrations (MBCs) and the ability of the steroid derivatives to lower the MICs of other antibiotics, e.g., erythromycin and novobiocin.

A person of skill will recognize that the compounds described herein preserve certain stereochemical and electronic characteristics found in steroids. The term "same configuration" as used herein refers to substituents on the fused steroid having the same stereochemical orientation. For example substituents $R_3$, $R_7$ and $R_{12}$ are all $\beta$-substituted or $\alpha$-substituted. The configuration of the moities $R_3$, $R_7$, and $R_{12}$ substituted on C3, C7, and C12 may be important for interaction with the cellular membrane.

In another aspect, the invention features several applications of using the above-described compounds. For example, an effective amount of an anti-microbial composition comprising such a compound can be administered to a host (including a human host) to treat a microbial infection. The compound by itself may provide the anti-microbial effect, in which case the amount of the compound administered is sufficient to be anti-microbial. Alternatively, an additional anti-microbial substance to be delivered to the microbial cells (e.g., an antibiotic) is included in the anti-microbial composition. By facilitating delivery to the target cells, the compounds can enhance the effectiveness of the additional antimicrobial substance. In some cases the enhancement may be substantial. Particularly important target microbes are bacteria (e.g., gram negative bacteria generally or bacteria which have a substantial (>40%) amount of a lipid A or lipid A-like substance in the outer membrane). Other microbes including fungi, viruses, and yeast may also be the target organisms.

The compounds can also be administered in other contexts to enhance cell permeability to introduce any of a large number of different kinds of substances into a cell, particularly the bacterial cells discussed above. In addition to introducing anti-microbial substances, the invention may be used to introduce other substances such as macromolecules (e.g., vector-less DNA). In addition, the compounds according to the invention can be used to permeabilize a sperm cell.

The invention can also be used to make anti-microbial compositions (e.g., disinfectants, antiseptics, antibiotics etc.) which comprise one of the above compounds. These compositions are not limited to pharmaceuticals, and they may be used topically or in non-therapeutic contexts to control microbial (particularly bacterial) growth. For example,

they may be used in applications that kill or control microbes on contact.

In yet another aspect, the invention generally features methods of identifying compounds that are effective against a microbe by administering a candidate compound and a compound according to the invention the microbe and determining whether the candidate compound has a static or toxic effect (e.g, an antiseptic, germicidal, disinfectant, or antibiotic effect) on the microbe. Again, bacteria such as those discussed above are preferred. This aspect of the invention permits useful testing of an extremely broad range of candidate anti-microbials which are known to have anti-microbial effect in some contexts, but which have not yet been shown to have any effect against certain classes of microbes such as the bacteria discussed above. As described in greater detail below, this aspect of the invention permits testing of a broad range of antibiotics currently thought to be ineffective against gram negative or lipid A-like containing bacteria.

In yet another aspect the invention features compositions which include one of the above compounds in combination with a substance to be introduced into a cell such as an antimicrobial substance as described in greater detail above. The compound and the additional substance may be mixed with a pharmaceutically acceptable carrier.

Other features or advantages of the present invention will be apparent from the following detailed description of several embodiments, and also from the appending claims.

The invention encompasses steroid derivatives that can be made by the synthetic routes described herein, and methods of treating a subject having a condition mediated by a bacterial infection by administering an effective amount of a pharmaceutical composition containing a compound disclosed herein to the subject.

## DESCRIPTION OF THE PREFERRED EMBODIMENTS

In general, the present invention provides the compounds of formula I described above. The preparation methods and the MIC and MBC of compounds of formula I are described. The cellular membrane permeability is also measured and described. Compounds that are useful in accordance with the invention, as described below, include novel steroid derivatives that exhibit bacteriostatic, bactericidal, and bacterial sensitizer properties. Those skilled in the art will appreciate that the invention extends to other compounds within the formulae given in the claims below, having the described characteristics. These characteristics can be determined for each test compound using the assays detailed below and elsewhere in the literature.

Known compounds that are used in accordance with the invention and precursors to novel compounds according to the invention can be purchased, e.g., from Sigma Chemical Co., St. Louis; Aldrich, Milwaukee; Steroloids and Research Plus. Other compounds according to the invention can be synthesized according to known methods and the methods described below using publicly available precursors.

The compounds of the present invention include but are not limited by amine or guanidine groups covalently tethered to a steroid backbone, e.g., cholic acid. The backbone can be used to orient the amine or guanidine groups on one face of the steroid. Other steroid backbones also can be used, e.g., 5 member fused rings.

The biological activity of the compounds can be determined by standard methods known to those of skill in the art, such as the "minimal inhibitory concentration (MIC)" assay described in the present examples, whereby the lowest concentration at which no change in optical density (OD) is observed for a given period of time is recorded as MIC. When the compound alone is tested against a control that lacks the compound, the antimicrobial effect of the compound alone is determined.

Alternatively, "fractional inhibitory concentration (FIC)" is also useful for determination of synergy between the compounds of the invention, or the compounds in combination with known antibiotics. FICs can be' performed by checkerboard titrations of compounds in one dimension of a microtiter plate, and of antibiotics in the other dimension, for example. The FIC is calculated by looking at the impact of one antibiotic on the MIC of the other and vice versa. An FIC of one indicates that the influence of the compounds is additive and an FIC of less than one indicates synergy. Preferably, an FIC of less than 0.5 is obtained for synergism. As used herein, FIC can be determined as follows:

$$\text{FIC} = \frac{\text{MIC (compound in combination)}}{\text{MIC (compound alone)}} + \frac{\text{MIC (antibiotic in combination)}}{\text{MIC (antibiotic alone)}}$$

This procedure permits determination of synergystic effects of the compound with other compounds. For example, substances that generally may not be sufficiently effective against certain bacteria at safe dosages can be made more effective with the compound of the invention, thus enabling use of the substances against new categories of infections. Specifically, many existing antibiotics are effective against some gram positive bacteria, but are not currently indicated to treat gram negative bacterial infection. In some cases, the antibiotic may be ineffective by itself against gram negative bacteria because it fails to enter the cell. Compounds of the invention may increase permeability so as to.render the antibiotics effective against gram negative bacteria.

In addition, fractional inhibitory concentration is also useful for determination of synergy between compounds

of the invention in combination with other compounds having unknown anti-bacterial activity or in combination with other compounds, e.g., compounds which have been tested and show anti-bacterial activity. For example, compounds of the invention may increase permeability so as to render compounds lacking anti-bacterial activity effective against bacteria. The FIC can also be used to test for other types of previously unappreciated activity of substances that will be introduced into the cell by means of permeability enhancing compounds according to the invention.

While we do not wish to be bound to any single specific theory, and such a theory is not necessary to practice the invention, one mechanism of action is the lipid A interaction of multiple (usually three) moieties, which under phisiological conditions are positively charged, e.g., guanidino or amino moieties. The moieties extend away from the general plane of the remainder of the molecule, thus mimicing certain aspects of the structure of polymixins. In this regard, compounds of the invention will generally be useful in the way that polymixins are useful. Moreover, in regard to systemic administration, those skilled in the art will recognize appropriate toxicity screens that permit selection of compounds that are not toxic at dosages that enhance microbial permeability.

As noted, the invention also involves topical as well as non-therapeutic (antiseptic, germicidal, or disinfecting) applications in which the compounds are contacted with surfaces to be treated. The term "contacting" preferably refers to exposing the bacteria to the compound so that the compound can effectively inhibit, kill, or lyse bacteria, bind endotoxin (LPS), or permeabilize gram- negative bacterial outer membranes. Contacting may be in vitro, for example by adding the compound to a bacterial culture to test for susceptibility of the bacteria to the compound. Contacting may be in vivo, for example administering the compound to a subject with a bacterial disorder, such as septic shock. "Inhibiting" or "inhibiting effective amount" refers to the amount of compound which is required to cause a bacteriostatic or bactericidal effect. Examples of bacteria which may be inhibited include *E. coli, P. aeruginosa, E. cloacae, S. typhimurium, M. Tuberculosis and S. aureus*.

The method of inhibiting the growth of bacteria may further include the addition of antibiotics for combination or synergistic therapy. The appropriate antibiotic administered will typically depend on the susceptibility of the bacteria such as whether the bacteria is gram negative or gram positive, and will be . easily discernable by one of skill in the art. Examples of particular classes of antibiotics to be tested for synergistic therapy with the compounds of the invention (as described above) include aminoglycosides (e.g., tobramycin), penicillins (e.g.; piperacillin), cephalosporins (e.g., ceftazidime), fluoroquinolones (e.g., ciprofloxacin), carbepenems (e.g., imipenem), tetracyclines and macrolides (e.g., erythromycin and clarithromycin). The method of inhibiting the growth of bacteria may further include the addition of antibiotics for combination or synergistic therapy. The appropriate antibiotic administered will typically depend on the susceptibility of the bacteria such as whether the bacteria is gram negative or gram positive, and will be easily discernable by one of skill in the art. Further to the antibiotics listed above, typical antibiotics include aminoglycosides (amikacin, gentamicin, kanamycin, netilmicin, tobramycin, streptomycin, azithromycin, clarithromycin, erythromycin, erythromycin estolate/ethylsuccinate/ gluceptate/lactobionate/stearate), beta-lactams such as penicillins (e.g., penicillin G, penicillin V, methicillin, nafcillin, oxacillin, cloxacillin, dicloxacillin, ampicillin, amoxicillin, ticarcillin, carbenicillin, mezlocillin, azlocillin and piperacillin), or cephalosporins (e.g., cephalothin, cefazolin, cefaclor, cefamandole, cefoxitin, cefuroxime, cefonicid, cefmetazole, cefotetan, cefprozil, loracarbef, cefetamet, cefoperazone, cefotaxime, ceftizoxime, ceftriaxone, ceftazidime, cefepime, cefixime, cefpodoxime, and cefsulodin). Other classes of antibiotics include carbapenems (e. g., imipenem), monobactams (e.g., aztreonam), quinolones (e.g., fleroxacin, nalidixic acid, norfloxacin, ciprofloxacin, ofloxacin, enoxacin, lomefloxacin and cinoxacin), tetracyclines (e.g., doxycycline, minocycline, tetracycline), and glycopeptides (e.g., vancomycin, teicoplanin), for example. Other antibiotics include chloramphenicol, clindamycin, trimethoprim, sulfamethoxazole, nitrofurantoin, rifampin and mupirocin.

Administration

The compounds may be administered to any host, including a human or non-human animal, in an amount effective to inhibit not only growth of a bacterium, but also a virus or fungus. These compounds are useful as antimicrobial agents, antiviral agents, spermicidal agents, and antifungal agents. The compounds may be administered to any host, including a human or non-human animal, in an amount effective to inhibit not only growth of a bacterium, but also a virus or fungus. These compounds are useful as antimicrobial agents, antiviral agents, and antifungal agents.

The compounds of the invention can be administered parenterally by injection or by gradual infusion over time. The compounds can be administered topically, intravenously, intraperitoneally, intramuscularly, subcutaneously, intracavity, or transdermally. Preferred methods for delivery of the compound include orally, by encapsulation in microspheres or proteinoids, by aerosol delivery to the lungs, or transdermally by iontophoresis or transdermal electroporation. Other methods of administration will be known to those skilled in the art.

Preparations for parenteral administration of a compound of the invention include sterile aqueous or nonaqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles

include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

The invention provides a method of treating or ameliorating an endotoxemia or septic shock (sepsis) associated disorder, or one or more of the symptoms of sepsis comprising administering to a subject displaying symptoms of sepsis or at risk for developing sepsis, a therapeutically effective amount of a compound of the invention. The term "ameliorate" refers to a decrease or lessening of the symptoms of the disorder being treated. Such symptoms which may be ameliorated include those associated with a transient increase in the blood level of TNF, such as fever, hypotension, neutropenia, leukopenia, thrombocytopenia, disseminated intravascular coagulation, adult respiratory distress syndrome, shock and multiple organ failure. Patients who require such treatment include those at risk for or those suffering from toxemia, such as endotoxemia resulting from a gram-negative bacterial infection, venom poisoning, or hepatic failure, for example. In addition, patients having a gram-positive bacterial, viral or fungal infection may display symptoms of sepsis and may benefit from such a therapeutic method as described herein. Those patients who are more particularly able to benefit from the method of the invention are those suffering from infection by *E. coli, Haemophilus influenza B, Neisseria meningitidis, staphylococci,* or *pneumococci*. Patients at risk for sepsis include those suffering from gunshot wounds, renal or hepatic failure, trauma, burns, immunocompromised (HIV), hematopoietic neoplasias, multiple myeloma, Castleman's disease or cardiac myxoma.

In addition, the compounds may be incorporated into biodegradable polymers allowing for sustained release, the polymers being implanted in the vicinity of where delivery is desired, for example, at the site of an bacterial infection. The biodegradable polymers and their use are described in detail in Brem et al., J. Neurosurg, 74:441-446 (1991).

As mentioned above, the present invention provides a pharmaceutical formulation having an effective amount of a compound of formula I for treating a patient having a bacterial infection. As used herein, an effective amount of the compound is defined as the amount of the compound which, upon administration to a patient, inhibits growth of bacteria, kills bacteria cells, sensitizes bacteria to other antibiotics, or eliminates the bacterial infection entirely in the treated patient. The dosage of the composition will depend on the condition being treated, the particular derivative used, and other clinical factors such as weight and condition of the patient and the route of administration of the compound. However, for oral administration to humans, a dosage of 0.01 to 100 mg/kg/day, preferably 0.01-1 mg/kg/day, is generally sufficient. Effective doses will also vary, as recognized by those skilled in the art, dependent on route of administration, excipient usage, and the possibility of co-usage with other therapeutic treatments including other antibiotic agents.

For example, the term "therapeutically effective amount" as used herein for treatment of endotoxemia refers to the amount of compound used is of sufficient quantity to decrease the subject's response to LPS and decrease the symptoms of sepsis. The term "therapeutically effective" therefore includes that the amount of compound sufficient to prevent, and preferably reduce by at least 50%, and more preferably sufficient to reduce by 90%, a clinically significant increase in the plasma level of TNF. The dosage ranges for the administration of compound are those large enough to produce the desired effect. Generally, the dosage will vary with the age, condition, sex, end extent of the infection with bacteria or other agent as described above, in the patient and can be determined by one skilled in the art. The dosage can be adjusted by the individual physician in the event of any contraindications. In any event, the effectiveness of treatment can be determined by monitoring the level of LPS and TNF in a patient. A decrease in serum LPS and TNF levels should correlate with recovery of the patient.

In addition, patients at risk for or exhibiting the symptoms of sepsis can be treated by the method as described above, further comprising administering, substantially simultaneously with the therapeutic administration of compound, an inhibitor of TNF, an antibiotic, or both. For example, intervention in the role of TNF in sepsis, either directly or indirectly, such as by use of an anti-TNF antibody and/or a TNF antagonist, can prevent or ameliorate the symptoms of sepsis. Particularly preferred is the use of an anti-TNF antibody as an active ingredient, such as a monoclonal antibody with TNF specificity as described by Tracey, et al. (*Nature,* 330:662, 1987).

A patient who exhibits the symptoms of sepsis may be treated with an antibiotic in addition to the treatment with compound. Typical antibiotics include an aminoglycoside, such as gentamicin or a beta-lactam such as penicillin, or cephalosporin or any of the antibiotics as previously listed above. Therefore, a preferred therapeutic method of the invention includes administering a therapeutically effective amount of cationic compound substantially simultaneously with administration of a bactericidal amount of an antibiotic. Preferably, administration of compound occurs within about 48 hours and preferably within about 2-8 hours, and most preferably, substantially concurrently with administration of the antibiotic.

The term "bactericidal amount" as used herein refers to an amount sufficient to achieve a bacteria-killing blood concentration in the patient receiving the treatment. The bactericidal amount of antibiotic generally recognized as safe for administration to a human is well known in the art, and as is known in the art, varies with the specific antibiotic and the type of bacterial infection being treated.

Because of the antibiotic, antimicrobial, and antiviral properties of the compounds, they may also be used as preservatives or sterillants of materials susceptible to microbial or viral contamination. The compounds of the invention can be utilized as broad spectrum antimicrobial agents directed toward various specific applications. Such applications include use of the compounds as preservatives in processed foods when verified as effective against organisms including *Salmonella, Yersinia, Shigella*, either alone or in combination with antibacterial food additives such as lysozymes; as a topical agent (*Pseudomonas*, *Streptococcus*) and to kill odor producing microbes (*Micrococci*). The relative effectiveness of the compounds of the invention for the applications described can be readily determined by one of skill in the art by determining the sensitivity of any organism to one of the compounds.

While primarily targeted at classical gram-negative-staining bacteria whose outer capsule contains a substantial amount of lipid A, it may also be effective against other organisms with a hydrophobic outer capsule. For example, *mycobacterium spp*. have a waxy protective outer coating, and compounds of the invention in combination with antibiotics may provide enhanced effectiveness against Mycobaterial infection, including tuberculosis. In that case, the compounds could be administered nasally (aspiration), by any of several known techniques.

Apart from anti-microbial action, the permeability provided by the compounds may enchance introduction of a great variety of substances into microbes. For example, the compounds may be used to enhance introduction of macromolecules such as DNA or RNA into microbes, particularly gram negative bacteria. In that case, there may be no need for the traditional vectors (e.g., phages) used to package nucleic acids when transfecting the microbes. Conditions and techniques for introducing such macromolecules into microbes, using the compounds of the invention will in most cases be routine.

The formulations include those suitable for oral, rectal, nasal, topical (including buccal and sublingual), vaginal or parenteral (including subcutaneous, intramuscular, intravenous, intradermal, intraocular, intratracheal, and epidural) administration. The formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into associate the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion and as a bolus, etc.

A tablet may be made by compression or molding, optionally with one or more accessory ingredients. Compressed tablets may be prepared by compressing, in a suitable machine, the active ingredient in a free-flowing form such as a powder or granules, optionally mixed with a binder, lubricant, inert diluent, preservative, surface-active or dispersing agent. Molded tables may be made by molding, in a suitable machine, a mixture of the powdered compound moistened with an inert liquid diluent. The tablets may optionally coated or scored and may be formulated so as to provide a slow or controlled release of the active ingredient therein.

Formulations suitable for topical administration in the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutical acceptable carrier. A preferred topical delivery system is a transdermal patch containing the ingredient to be administered.

Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is taken, i.e., by rapid inhalation through the nasal passage from a container of the powder held close up to the nose. Suitable formulations, wherein the carrier is a liquid, for administration, as for example, a nasal spray, aerosol, or as nasal drops, include aqueous or oily solutions of the active ingredient.

Formulations suitable for vaginal administration may be presented as pessaries, tampons, creams, gels, pastes, foams or spray formulations containing in addition to the active ingredient such as carriers as are known in the art to be appropriate.

Formulations suitable for parenteral administration include aqueous and non-aqueous sterile injection solutions which may contain anti-oxidants, buffers, other bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) conditions requiring only the

addition of the sterile liquid carrier, for example, water for injections, immediately prior to use. Extemporaneous injection solutions and suspensions may be prepared from sterile powders, granules and tables of the kind previously described.

Preferred unit dosage formulations are those containing a daily dose or unit, daily sub-dose, as herein above recited, or an appropriate fraction thereof, of the administered ingredient.

It should be understood that in addition to the ingredients, particularly mentioned above, the formulations of this invention may include other agents conventional in the art having regard to the type of formulation in question, for example, those suitable for oral administration may include flavoring agents.

The carrier in the pharmaceutical composition must be "acceptable" in the sense of being compatible with the active ingredient of the formulation (and preferably, capable of stabilizing it) and not deleterious to the subject to be treated.

Without further elaboration, it is believed that the above description has adequately enabled the present invention. The following specific embodiments are, therefore, to be construed as merely illustrative, and not limitative of the remainder of the disclosure in any way whatsoever.

Examples 1-6 represent typical syntheses of compounds 1 through 48 as exemplified in Scheme 1 through 7. Example 7 represents MIC and MCB testing. Example 8 represents the ability of the compounds of formula I to lower the MIC's of other antibiotics. Example 9 represents other compounds of formula I which can be synthesized using known starting materials and reaction schemes that are similar to those described herein. For example, the hydroxyl groups on cholic acid can be converted into amine by the method found in Hsieh et al., Synthesis and DNA Binding Properties of C3-, C12-, and C24- Substituted Amino-Steroids Derived from Bile Acids, *Biorganic and Medicinal Chemistry*, 1995, vol. 6, 823-838.

**Scheme 1.** Preparation of compounds 1, 2, 4 and 5.

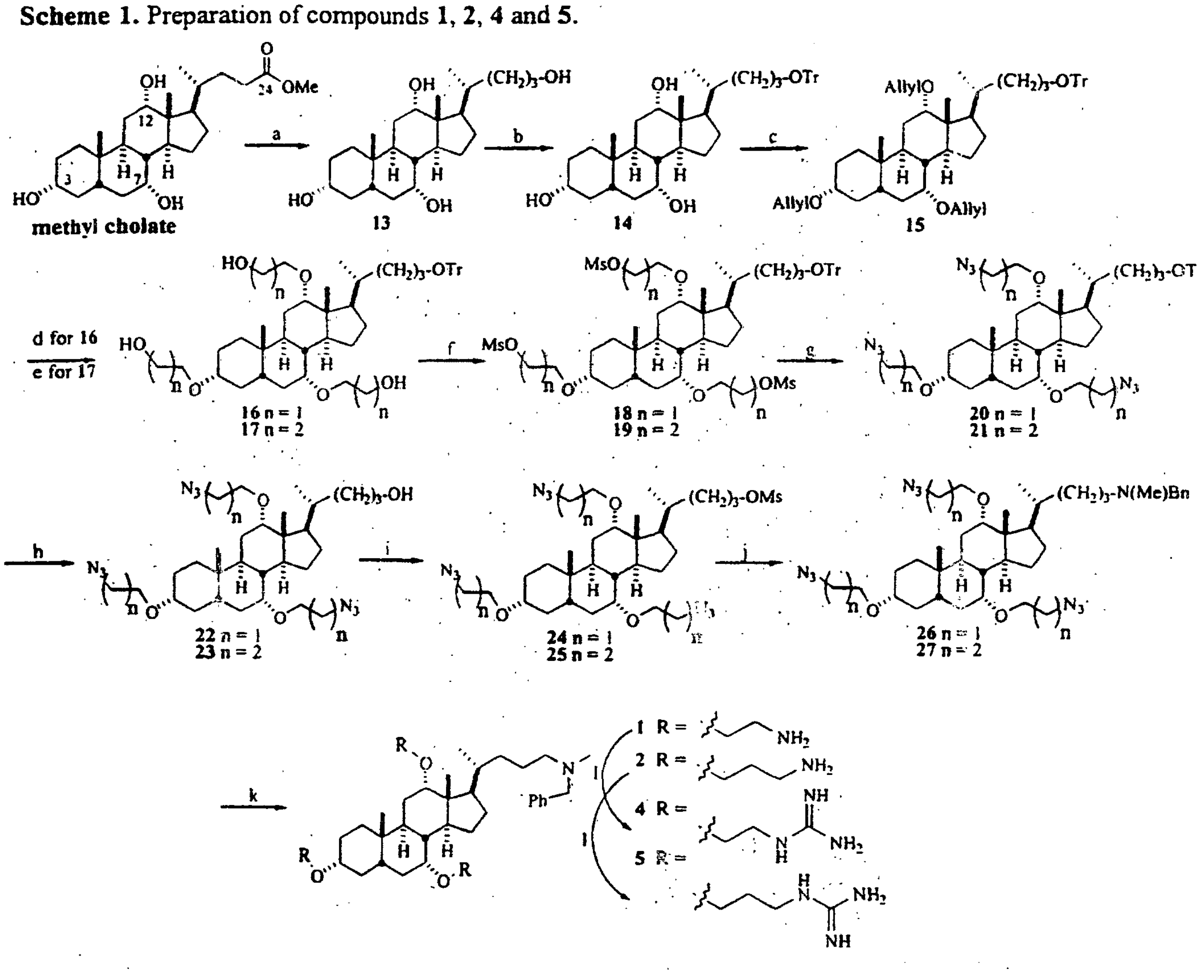

methyl cholate
13
14
15
16 n = 1
17 n = 2
18 n = 1
19 n = 2
20 n = 1
21 n = 2
22 n = 1
23 n = 2
24 n = 1
25 n = 2
26 n = 1
27 n = 2
1
2
4
5

Reagents (reaction yields in parentheses): a) $LiAlH_4$, THF (98%). b) tritylchloride, $Et_3N$, DMF (70%). c) allylbromide, NaH, THF (96%). d) $O_3$, $CH_2Cl_2$, MeOH; $Me_2S$; $NaBH_4$ (95%). e) 9-BBN, THF; $H_2O_2$, NaOH (80%). f) MsCl, $CH_2Cl_2$, $Et_3N$ (78%, 82%). g) $NaN_3$, DMSO (66% for 20, 19 carried directly on to 23). h) TsOH, MeOH (94%, 94% overall from 19). i) MsCl, $CH_2Cl_2$, $Et_3N$ (99%, 97%). j) N-benzylmethylamine (95%, 96%). k) $LiAlH_4$, THF (95%, 99%). l) $NH_2C(NH)SO_3H$, MeOH (91%, 89%).

**Scheme 2.** Preparation of compound **3**.

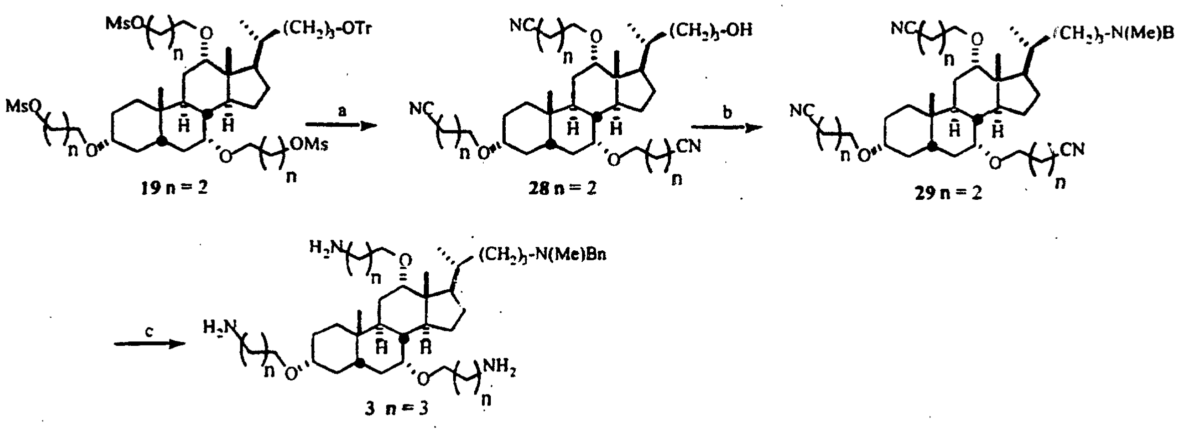


19 n = 2

28 n = 2

29 n = 2

3 n = 3

Reagents (reaction yields in parentheses): a) KCN. DMSO; MeOH, TsOH (92%). (b) MsCl, $Et_3N$, $CH_2Cl_2$; BnMeNH (88%). c) $LiAlH_4$, $AlCl_3$, THF (50%).

**Scheme 3.** Preparation of **6** and **7**.

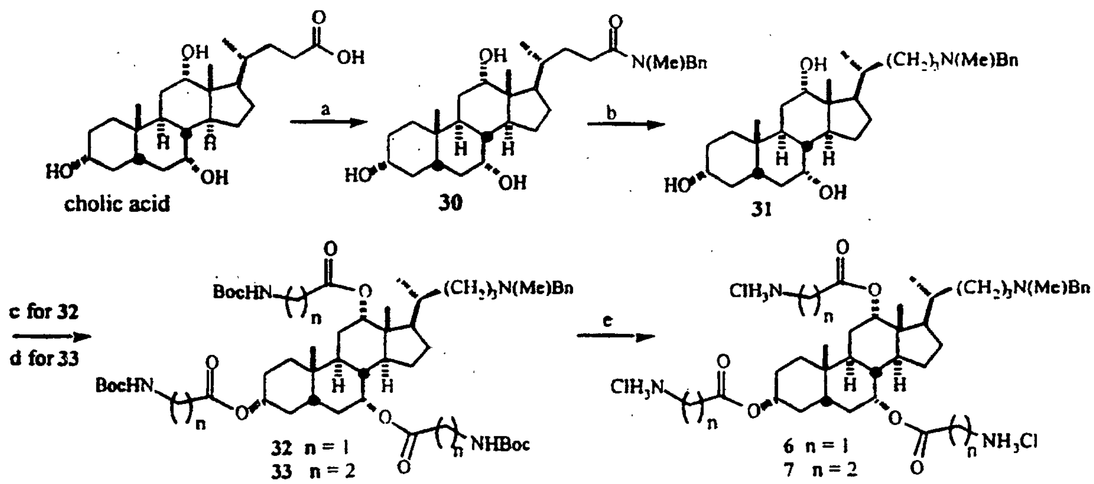


cholic acid

**30**

**31**

32 n = 1
33 n = 2

6 n = 1
7 n = 2

**Reagents** (reaction yields in parentheses): a) dicycohexylcarbodiimide. N-hydroxysuccinimide. methylphenylamine, $CH_2Cl_2$, MeOH (85%). b) $LiAlH_4$. THF (82%). c) dicyclohexylcarbodiimide. dimethylaminopyridine. Boc-glycine. $CH_2Cl_2$ (68%). d) dicyclohexylcarbodiimide. dimethylaminopyridine. Boc-β-alanine. $CH_2Cl_2$ (72%). e) HCl. dioxane (~100%, ~100%).

**Scheme 4.** Synthesis of compound 8.

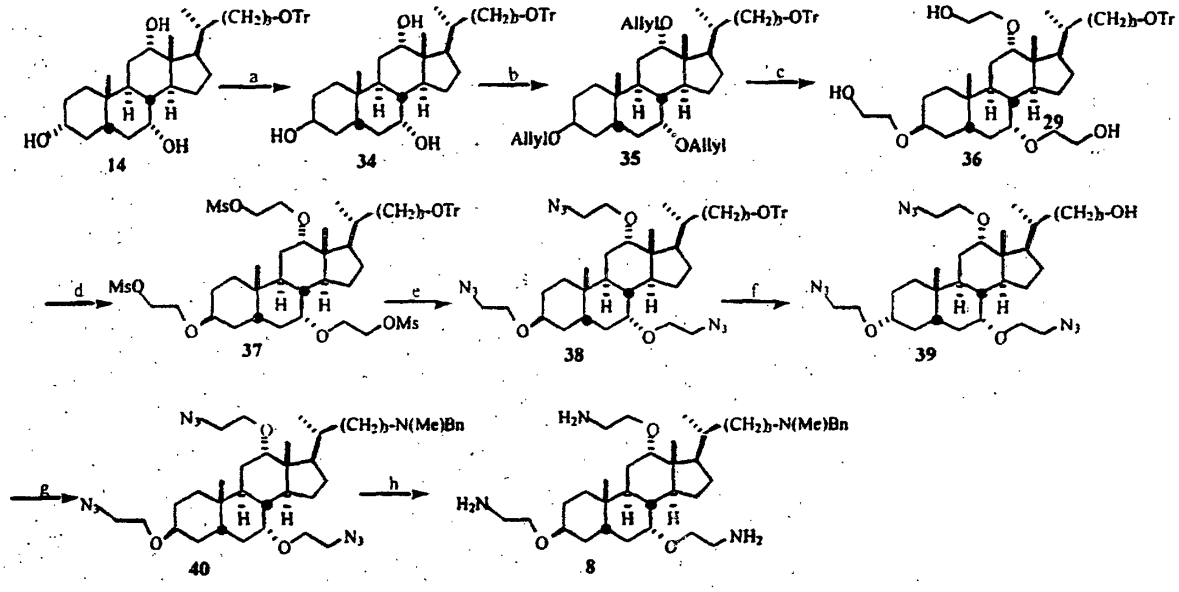

Reagents (reaction yields in parentheses): a) DIAD, $Ph_3P$, *p*-nitrobenzoic acid, THF (85%); NaOH, MeOH (85%). b) allylbromide, NaH, THF (79%). c) $O_3$, $CH_2Cl_2$, MeOH; $Me_2S$; $NaBH_4$ (65%). d) MsCl, $CH_2Cl_2$, $Et_3N$ (86%). e) $NaN_3$, DMSO (80%). f) TsOH, MeOH (94%). g) MsCl, $CH_2Cl_2$, $Et_3N$; N-benzylmethylamine (93%). g) $LiAlH_4$, THF (94%).

**Scheme 5.** Synthesis of compounds 9 and 10.

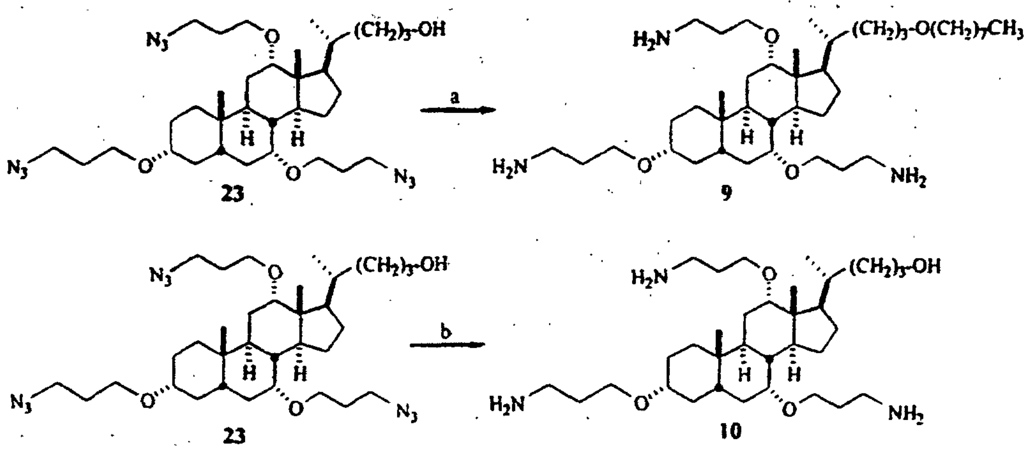

Reagents (reaction yields in parentheses): a) NaH, octyl bromide, DMF (80%); $LiAlH_4$ THF (60%). b) $LiAlH_4$, THF (60%).

**Scheme 6.** Synthesis of compound **11.**

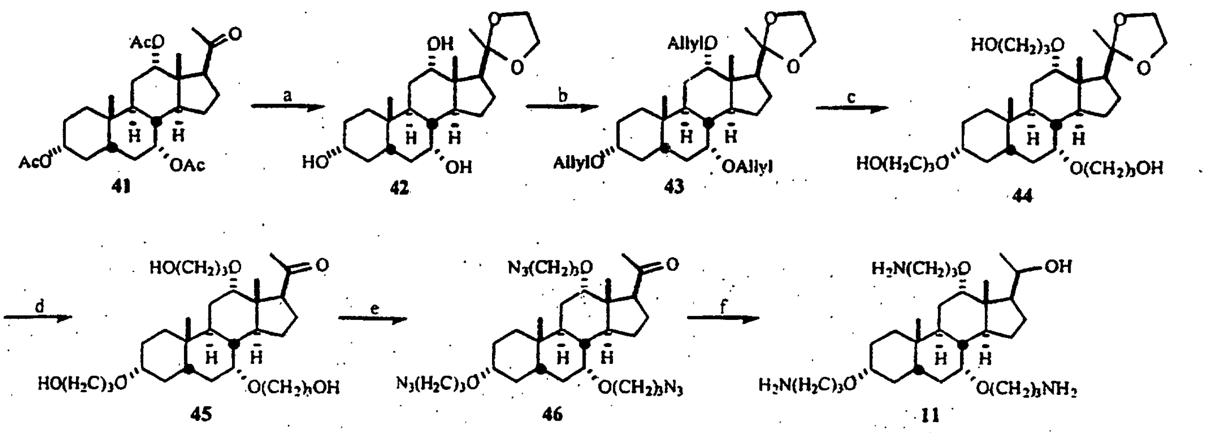


41 42 43 44 45 46 11

Reagents (reaction yields in parentheses): a) ethylene glycol, *p*-toluenesulfonic acid, benzene; NaOH, MeOH (96%). b) allylbromide, NaH, THF (90%). c) 9-BBN, THF; NaOH, $H_2O_2$, $H_2O$ (54%). d) pyridinium *p*-toluenesulfonate, MeOH (98%). e) methanesulfonyl chloride, $Et_3N$, $CH_2Cl_2$ ; $NaN_3$, DMSO (88%). f) $LiAlH_4$, THF (69%).

**Scheme 7.** Synthesis of compound **12.**

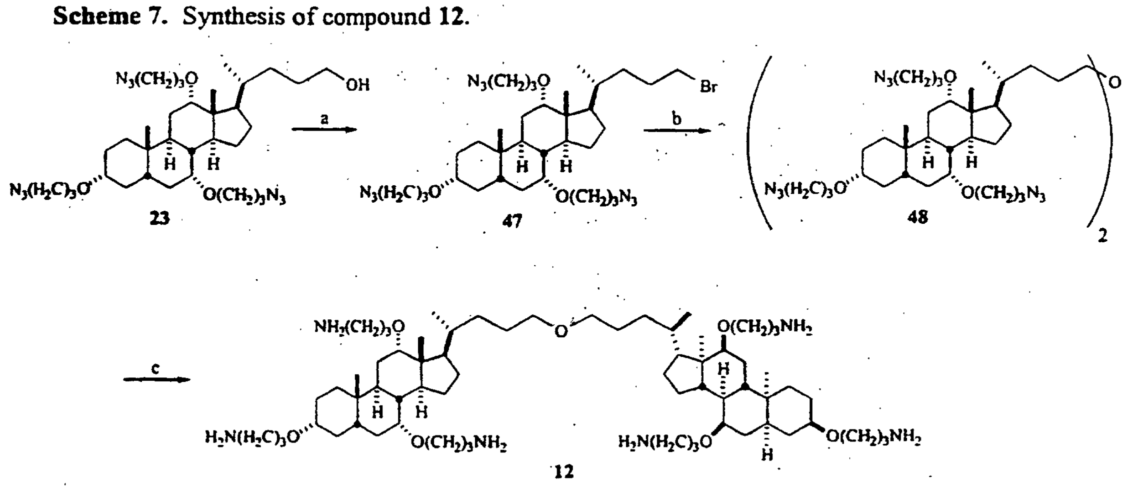


23 47 48 12

Reagents (reaction yields in parentheses): a) methanesulfonyl chloride, $Et_3N$, $CH_2Cl_2$; NaBr, DMF (97%). b) 23, NaH, DMF (52%). c) $LiAlH_4$, THF (76%).

Synthesis of Compounds 1-48

General:

$^1H$ and $^{13}C$ NMR spectra were recorded on a Varian Gemini 2000 (200 MHZ) or Varian Unity 300 (300 MHZ) spectrometer and are referenced to $CHCl_3$($^1H$) or $CDCl_3$($^{13}C$). IR spectra were recorded on a Perkin Elmer 1600 FTIR instrument. Mass spectrometric data were obtained on a JOEL SX 102A spectrometer. THF was dried over Na°/benzophenone and $CH_2Cl_2$ was dried over $CaH_2$ prior to use. Other reagents and solvents were obtained commercially and were used as received.

**Example 1**

Compound 13:

To a 1 L round-bottom flask were added methyl cholate (30.67 g, 72.7 mmol) in dry THF (600 mL) and $LiAlH_4$ (4.13 g, 109 mmol). After reflux for 48 hours, saturated aqueous $Na_2SO_4$ (100 mL) was introduced slowly, and the resulted precipitate was filtered out and washed with hot THF and MeOH. Recrystallization from MeOH gave colorless

crystals of 13 (28.0 g, 98% yield). m.p. 236.5-238°C; IR (KBr) 3375, 2934, 1373, 1081 $cm^{-1}$; $^1H$ NMR ($CDCl_3$/MeOH-d4, 200 MHZ) δ 3.98 (bs, 1 H), 3.83 (bs, 1 H), 3.60-3.46 (m, 2 H), 3.38 (bs, 5 H), 2.30-2.10 (m, 2 H), 2.05-1.05 (series of multiplets, 22 H), 1.03 (bs, 3 H), 0.92 (s, 3 H), 0.71 (s, 3 H); $^{13}C$ NMR ($CDCl_3$/MeOH-d4, 50 MHZ) δ 73.89, 72.44, 68.99, 63.51, 48.05, 47.12, 42.49, 40.37, 39.99, 36.62, 36.12, 35.58, 35.40, 32.77, 30.69, 30.04, 29.02, 28.43, 27.27, 23.96, 23.08, 18.00, 13.02; HRFAB-MS (thioglycerol + $Na^+$ matrix) *m*/*e*: ($[M+Na]^+$) 417.2992 (55.3%); cacld. 417.2981.

Compound 14:

To a round-bottom flask were added 13 (28.2 g, 71.7 mmol) in DMF (300 mL), $Et_3N$ (20 mL, 143.4 mmol), trityl chloride (25.98g, 93.2 mmol) and DMAP (0.13 g, 1.07 mmol). The mixture was stirred at 50°C under $N_2$ for 30 hours followed by the introduction of water (1000 mL) and extraction with EtOAc (5 x 200 mL). The combined extracts were washed with water and brine and then dried over $MgSO_4$. After removal of solvent in vacuo, the residue was purified using $SiO_2$ chromatography ($CH_2Cl_2$, $Et_2O$ and MeOH as eluents) to give 14 as a pale yellow solid (31.9 g, 70% yield). m.p. 187°C (decomposition); IR (KBr) 3405, 2935, 1448, 1075 $cm^{-1}$; $^1H$ NMR ($CDCl_3$, 200 MHZ) δ 7.46-7.42 (m, 6 H), 7.32-7.17 (m, 9 H), 3.97 (bs, 1 H), 3.83 (bs, 1 H), 3.50-3.38 (m, 1 H), 3.01 (bs, 1 H), 2.94 (dd, *J* = 14.2, 12.2 Hz, 2 H), 2.64 (bs, 1 H), 2.51 (bs, 1 H), 2.36-2.10 (m, 2 H), 2.00-1.05 (series of multiplets, 22 H), 0.96 (d, *J* = 5.8 Hz, 3 H), 0.87 (s, 3 H), 0.64 (s, 3 H); $^{13}C$ NMR ($CDCl_3$, 50 MHZ) δ 144.77, 128.93, 127.91, 127.01, 86.43, 73.35, 72.06, 68.66, 64.28, 47.47, 46.53, 41.74, 41.62, 39.64, 35.57, 35.46, 34.91, 34.82, 32.40, 30.55, 28.21, 27.69, 26.80, 26.45, 23.36, 22.59, 17.83, 12.61; HRFAB-MS (thioglycerol + $Na^+$ matrix) *m*/*e*: ($[M+Na]^+$) 659.4069 (100%); cacld. 659.4076.

Compound 15:

To a round-bottom flask were added 14 (20.0 g, 31.4 mmol) in dry THF (600 mL) and NaH (60% in mineral oil, 6.3 g, 157.2 mmol). The mixture was refluxed for 30 minutes under $N_2$ followed by addition of allyl bromide (27 mL, 314 mmol). After 60 hours of reflux, additional NaH (3 eq.) and allyl bromide (4 eq.) were added. Following'another 50 hours of reflux, water (20 mL) was introduced slowly followed by addition of 1% HCl until the aqueous layer became neutral. The mixture was then extracted with ether (3 x 100 mL) and the combined extracts were washed with water (100 mL) and brine (2 x 100 mL). The ether solution was dried over anhydrous $Na_2SO_4$, and after removal of solvent, the residue was purified using $SiO_2$ chromatography (hexanes and EtOAc/hexanes 1:8 as eluents) to give 15 (22.76 g, 96% yield) as a pale yellow glass. IR (neat) 2930, 1448, 1087 $cm^{-1}$; $^1H$ NMR ($CDCl_3$, 200 M Hz) δ 7.48-7.30 (m, 6 H) , 7.32-7.14 (m; 9 H), 6.04-5.80 (m, 3 H), 5.36-5.04 (series of multiplets, 6 H), 4.14-3.94 (m, 4 H), 3.74 (td, *J* = 13.8, 5.8 Hz, 2 H), 3.53 (bs, 1 H) , 3.20-2.94 (m, 3 H) , 3.31 (bs, 1 H), 2.38-1.90 (m, 4 H), 1.90-0.96 (series of multiplets, 20 H), 0.90 (d, *J* = 5.4 Hz, 3 H), 0.89 (s, 3 H), 0.64 (s, 3 H); $^{13}C$ NMR ($CDCl_3$, 50 MHZ) δ 144.83, 136.27, 136.08, 128.94, 127.90, 126.98, 116.46, 115.70, 86.42, 80.94, 79.29, 74.98, 69.52, 69.39, 68.86, 64.39, 46.51, 46.42, 42.67, 42.14, 39.92, 35.63, 35.51, 35.13, 32.45, 28.98, 28.09, 27.66, 27.57, 26.72, 23.32, 23.11, 17.92, 12.69; HRFAB-MS (thioglycerol+$Na^+$ matrix) *m*/*e*: ($[M+Na]^+$) 779.5013 (86.1%); cacld. 779.5015.

Compound 16:

To a three-necked round bottom flask was added 15 (3.34 g, 4.4 mmol) in $CH_2Cl_2$ (200 mL) and methanol (100 mL). Through the cold solution (-78°C) ozone was bubbled through until a blue color persisted. Excess ozone was removed with oxygen flow. The mixture was left in a dry ice-acetone bath for an hour. Methyl sulfide (2.4 mL) was added and 15 minutes later, the mixture was treated with $NaBH_4$ (1.21 g, 32 mmol) in 5% aqueous NaOH solution (10 mL) /methanol (10 mL) and allowed to warm to room temperature. The mixture was washed with brine (3 x 50 mL), and the combined brine wash was extracted with $CH_2Cl_2$ (2 x 50 mL). The organic solution was dried over $MgSO_4$. After $SiO_2$ chromatography (MeOH (5%) in $CH_2Cl_2$), 3.30 g (95% yield) of 16 was isolated as an oil. IR (neat) 3358, 2934, 1448, 1070 $cm^{-1}$; $^1H$ NMR ($CDCl_3$, 200 MHZ) δ 7.50-7.42 (m, 6 H), 7.32-7.17 (m, 9 H), 3.80-2.96 (series of multiplets, 20 H), 2.25-0.96 (series of multiplets, 24 H), 0.89 (bs, 6 H), 0.65 (s, 3 H); $^{13}C$ NMR ($CDCl_3$, 50 MHZ) δ 144.73, 128.88, 127.87, 126.96, 86.38, 81.05, 79.75, 76.59, 70.33, 69.66, 69.30, 64.20, 62.25, 62.16, 62.03, 46.77, 46.36, 42.63, 41.77, 39.60, 35.43, 35.23, 35.05, 34.89, 32.42, 28.91, 27.93, 27.56, 27.15, 26.68, 23.'35, 22.98, 22.85, 18.15, 12.60; HRFAB-MS (thioglycerol+$Na^+$ matrix) *m*/*e*: ($[M+Na]^+$) 791.4860 (100%), cacld. 791.4863.

Compound 17:

To a round-bottom flask was added 16 (1.17 g, 1.55 mmol) in dry THF (30 mL) under $N_2$ in ice-bath followed by 9-BBN/THF solution (0.5 M, 10.2 mL, 5.51 mmol). The mixture was stirred at room temperature for 12 hours. Aqueous NaOH (20%) (2 mL) and hydrogen peroxide (30%) (2 mL) were added in sequence. The mixture was refluxed for 1 hour followed by the addition of brine (60 mL) and extraction with EtOAc (4 x 30 mL). The combined extracts were

dried over anhydrous $Na_2SO_4$. The product (1.01 g, 80% yield) was obtained as a colorless oil after $SiO_2$ chromatograhy (5% MeOH in $CH_2Cl_2$). IR (neat) 3396, 2936, 1448, 1365, 1089 $cm^{-1}$; $^1H$ NMR($CDCl_3$, 200 MHZ) δ 7.50-7.42 (m, 6 H), 7.34-7.16 (m, 9 H), 3.90-3.56 (m, 13 H), 3.50 (bs, 1 H), 3.40-2.96 (series of multiplets, 6 H), 2.30-0.94 (series of multiplets, 30 H), 0.90 (s, 3 H), 0.88 (d, *J* = 5.4 Hz, 3 H), 0.64 (s, 3 H); $^{13}C$ NMR($CDCl_3$, 50 MHZ) δ 144.73, 128.88, 127.85, 126.94, 86.36, 80.52, 78.90, 76.36, 66.82, 66.18, 65.77, 64.22, 61.53, 61.41, 61.34, 46.89, 46.04, 42.60, 41.59, 39.60, 35.37, 35.27, 34.88, 32.75, 32.44, 32.31, 28.82, 27.65, 27.48, 27.13, 26.77, 23.35, 22.74, 22.38, 18.08, 12.48; HRFAB-MS (thioglycerol+$Na^+$ matrix) *m/e*: ($[M+Na]^+$) 833.5331 (100%), cacld. 833.5332.

Compound 18:

To a round-bottom flask were added 16 (3.30 g, 4.29 mmol) in $CH_2Cl_2$ (150 mL) and $NEt_3$(2.09 mL, 15.01 mmol). The mixture was put in ice-bath under $N_2$ followed by addition of mesyl chloride (1.10 mL, 14.16 mmol). After 30 minutes, water (30 mL) and brine (200 mL) were added. The CH2Cl2 layer was washed with brine (2 x 50 mL) and dried over anhydrous $Na_2SO_4$. The combined aqueous mixture was extracted with EtOAc (3 x 100 mL). The combined extracts were washed with brine and dried over anhydrous $Na_2SO_4$. The desired product (3.35 g, 78% yield) was isolated as a pale yellow oil after $SiO_2$ chromatography (EtOAc/hexanes 1:1). IR (neat) 2937, 1448, 1352, 1174, 1120, 924 $cm^{-1}$; $^1H$ NMR ($CDCl_3$, 200 MHZ) δ 7.52-7.40 (m, 6 H), 7.34-7.20, (m, 9 H), 4.42-4.24 (m, 6 H), 3.90-3.64 (m, 4 H), 3.60-3.30 (m, 4 H), 3.24-3.00 (m, 3 H), 3.10 (s, 6 H), 3.05 (s, 3 H), 2.20-1.96 (m, 3 H) 1.96-1.60 (m, 8 H), 1.60-0.94 (series of multiplets, 13 H), 0.91 (bs, 6 H) 0.65 (s, 3 H); $^{13}C$ NMR ($CDCl_3$, 50 MHz) δ 114.68, 128.85, 127.85, 126.96, 86.37, 81.37, 79.58, 76.58, 69.95, 69.43, 69.34, 66.52, 66.31, 65.59, 64.11, 46.80, 46.20, 42.65, 41.48, 39.35, 37.82, 37.48, 35.36, 34.92, 34.73, 32.37, 28.66, 28.01, 27.44, 27.03, 26.72, 23.17, 22.91, 22.72, 18.13, 12.50; HRFAB-MS (thioglycerol+$Na^+$ matrix) *m/e*: ($[M+Na]^+$) 1205.4176 (81.5%), cacld. 1205.4189.

Compound 19:

To a round-bottom flask were added 17 (1.01 g, 1.25 mmol) in $CH_2Cl_2$ (50 mL) and $NEt_3$ (0.608 mL, 4.36 mmol). The mixture was put in ice-bath under $N_2$ followed by addition of mesyl chloride (0.318 mL, 4.11 mmol). After 30 minutes, water (10 mL) and then brine (80 mL) were added. The $CH_2Cl_2$ layer was washed with brine (2 x 20 mL) and dried over anhydrous $Na_2SO_4$. The combined aqueous mixture was extracted with EtOAc (3 x 40 mL). The combined extracts were washed with brine and dried over anhydrous $Na_2SO_4$. The desired product (1.07 g, 82%) was isolated as a pale yellowish oil after $SiO_2$ chromatography (EtOAc/hexanes 1:1). IR (neat) 2938, 1356, 1176, 1112 $cm^{-1}$; $^1H$ NMR ($CDCl_3$, 300 MHZ) δ 7.46-7.43, (m, 6 H), 7.32-7.22 (m, 9 H), 4.40-4.31 (m, 6 H), 3.72-3.64 (m, 2 H), 3.55 (dd, *J* = 6.3, 5.8 Hz, 2 H), 3.51 (bs, 1 H), 3.32-3.14 (m, 3 H), 3.14-2.92 (m, 3 H), 3.01 (s, 3 H), 3.01 (s, 3 H), 3.00 (s, 3 H), 2.10-1.92 (m, 10 H), 1.92-1.58 (m, 8 H), 1.56-0.92 (series of multiplets, 12 H) , 0.90 (s, 3 H), 0.89 (d, *J* = 5.4 Hz, 3 H), 0.64 (s, 3 H); $^{13}C$ NMR($CDCl_3$, 75 MHZ) δ 144.67, 128.85, 127.85, 126.96, 86.42, 81.06, 79.83, 76.81, 68.12, 68.06, 68.02, 64.26, 64.06, 63.42, 46.76, 46.38, 42.73, 41.87, 39.73, 37.44, 37.32, 37.29, 35.52, 35.48, 35.32, 35.06, 32.53, 30.55, 30.28, 30.02, 29.15, 27.96, 27.69, 27.61, 26.75, 23.52, 23.02, 18.17, 12.64; HRFAB-MS (thioglycerol+$Na^+$ matrix) *m/e*: ($[M+Na]^+$) 1067.4672 (100%), cacld. 1067.4659.

Compound 20:

To a round-bottom flask were added 18 (1.50 g, 1.50 mmol) in dry DMSO (20 mL) and $NaN_3$ (0.976 g, 15 mmol). The mixture was heated to 80°C and stirred under $N_2$ overnight then diluted with water (100 mL). The resulted aqueous mixture was extracted with EtOAc (3 x 50 mL), and the combined extracts washed with brine and dried over anhydrous $Na_2SO_4$. The desired product (0.83 g, 66% yield) was isolated as a clear glass after $SiO_2$ chromatography (EtOAc/hexanes 1:5). IR (neat) 2935, 2106, 1448, 130.2, 1114 $cm^{-1}$; $^1H$ NMR ($CDCl_3$, 200 MHZ) δ 7.50-7.42 (m, 6 H), 7.36-7.20 (m, 9 H), 3.84-3.70 (m, 2 H), 3.65 (t, *J* = 4.9 Hz, 2 H), 3.55 (bs, 1 H), 3.44-3.08 (m, 10 H), 3.02 (t, *J* = 6.4 Hz, 2 H), 2.38-0.96 (series of multiplets, 24 H), 0.92 (d, *J* = 5.6 Hz, 3 H), 0.91 (s, 3 H), 0.65 (s, 3 H); $^{13}C$ NMR ($CDCl_3$, 50 MHZ) δ 114.84, 128.97, 127.92, 126.99, 86.42, 81.24, 80.12, 76.59, 67.84, 67.29, 66.66, 64.36, 51.67, 51.44, 51.18, 46.53, 46.23, 42.21, 41.93, 39.73, 35.66, 35.36, 35.06, 34.78, 32.40, 28.95, 27.76, 27.39, 26.87, 23.45, 22.98, 22.92, 17.98, 12.53; HRFAB-MS (thioglycerol+$Na^+$ matrix) *m/e*: ($[M+Na]^+$) 866.5040 (100%), cacld. 866.5057.

Compound 22:

To a round-bottom flask were added 20 (830 mg, 0.984 mmol) in MeOH (30 mL) and $CH_2Cl_2$ (30 mL) and *p*-toluenesulfonic acid (9.35 mg, 0.0492 mmol). The solution was stirred at room temperature for 2.5 hours then saturated aqueous $NaHCO_3$ (10 mL) was introduced. Brine (30 mL) was added, and the mixture was extracted with EtOAc (4 x 20 mL). The combined extracts were dried over anhydrous $Na_2SO_4$. The desired product (0.564 g, 95% yield) was

isolated as a pale yellowish oil after $SiO_2$ chromatography (EtOAc/hexanes 1:2). IR (neat) 3410, 2934, 2106, 1301, 1112 $cm^{-1}$; $^1H$ NMR ($CDCl_3$, 200 MHZ) δ 3.80-3.54 (m, 7 H), 3.44-3.20 (m, 10 H), 2.35-0.96 (series of multiplets, 24 H), 0.95 (d, *J* = 6.4 Hz, 3H), 0.92 (s, 3 H), 0.68 (s, 3 H); $^{13}C$ NMR ($CDCl_3$, 50 MHZ) δ 81.10, 80.01, 76.60, 67.75, 67.16, 66.56, 63.63, 51.57, 51.34, 51.06, 46.29, 46.12, 42.12, 41.81, 39.60, 35.55, 35.23, 34.94, 34.66, 31.75, 29.48, 28.81, 27.72, 27.66, 27.29, 23.32, 22.86, 22.80, 17.85, 12.39; HRFAB-MS (thioglycerol+$Na^+$ matrix) *m*/*e*: ($[M+Na]^+$) 624.3965 (100%), cacld. 624.3962.

Compound 23:

To a round-bottom flask were added 19 (1.07 g, 1.025 mmol) and $NaN_3$ (0.666 g, 10.25 mmol) followed the introduction of dry DMSO (15 mL). The mixture was heated up to 80°C under $N_2$ overnight. After the addition of $H_2O$ (100 mL), the mixture was extracted with EtOAc (4 x 40 mL) and the combined extracts were washed with brine (2 x 50 mL) and dried over anhydrous $Na_2SO_4$. After removal of solvent, the residue was dissolved in MeOH (15 mL) and $CH_2Cl_2$ (15 mL) followed by the addition of catalytic amount of *p*-toluenesulfonic acid (9.75 mg, 0.051 mmol). The solution was stirred at room temperature for 2.5 hours before the addition of saturated $NaHCO_3$ solution (15 mL). After the addition of brine (60 mL), the mixture was extracted with EtOAc (5 x 30 mL). The combined extracts were washed with brine (50 mL) and dried over anhydrous $Na_2SO_4$. The desired product (0.617 g, 94% yield for two steps) was obtained as a yellowish oil after $SiO_2$ chromatography (EtOAc/hexanes 1:2). IR (neat) 3426, 2928, 2094, 1456, 1263, 1107 $cm^{-1}$; $^1H$ NMR ($CDCl_3$, 300 MHZ) δ 3.68-3.56 (m, 3 H), 3.56-3.34 (series of multiplets, 10 H), 3.28-3.00 (series of multiplets, 4 H), 2.20-2.00 (m, 3 H), 1.98-1.55 (series of multiplets, 15 H), 1.55-0.96 (series of multiplets, 13 H), 0.92 (d, *J* = 6.6 Hz, 3 H), 0.89 (s, 3 H), 0.66 (s, 3 H); $^{13}C$ NMR ($CDCl_3$, 75 MHZ) δ 80.63, 79.79, 76.04, 64.99, 64.45, 64.30, 63.72, 49.01, 48.94, 48.74, 46.49, 46.39, 42.70, 41.98, 39.80, 35.65, 35.42, 35.28, 35.08, 31.99, 29.78, 29.75, 29.70, 29.49, 29.06, 27.87, 27.79, 27.65, 23.53, 23.04, 22.85, 18.05, 12.59; HRFAB-MS (thioglycerol+Na matrix) *m*/*e*: ($[M+Na]^+$) 666.4415 (100%), cacld. 666.4431.

Compound 24:

To a round-bottom flask were added 22 (0.564 g, 0.938 mmol) in $CH_2Cl_2$ (30 mL) and $NEt_3$ (0.20 mL, 1.40 mmol). The mixture was put in ice-bath under $N_2$ followed by addition of mesyl chloride (0.087 mL, 1.13 mmol). After 30 minutes, water (20 mL) and brine (100 mL) were added. The $CH_2CL_2$ layer was washed with brine (2 x 20 mL) and dried over anhydrous $Na_2SO_4$. The combined aqueous mixture was extracted with EtOAc (3 x 30 mL). The combined extracts were washed with brine and dried over anhydrous $Na_2SO_4$. The desired product (0.634 g, 99% yield) was isolated as a pale yellowish oil after $SiO_2$ chromatography (EtOAc/hexanes 1:2). IR (neat) 2935, 2106, 1356, 1175, 1113 $cm^{-1}$; $^1H$ NMR ($CDCl_3$, 300 MHZ) δ 4.20 (t, *J* = 6.8 Hz, 2 H), 3.80-3.75 (m, 1 H), 3.70-3.64 (m, 3 H), 3.55 (bs, 1 H), 3.44-3.01 (m, 10 H), 3.00 (s, 3 H) , 2.32-2.17 (m, 3 H) , 2.06-2.03 (m, 1 H), 1.90-0.88 (series of multiplets, 20 H), 0.95 (d, *J* = 6.6 Hz, 3 H), 0.91 (s, 3 H), 0.68 (s, 3 H); $^{13}C$ NMR ($CDCl_3$, 75 MHZ) δ 80.90, 79.86, 76.43, 70.78, 67.64, 66.99, 66.48, 51.50, 51.26, 50.97, 46.05, 45.96, 42.08, 41.71, 39.51, 37.33, 35.15, 34.86, 34.60, 31.34, 28.73, 27.62, 27.59, 27.51, 25.68, 23.22, 22.80, 22.70, 17.62, 12.33; HRFAB-MS (thioglycerol+$Na^+$ matrix) *m*/*e*: ($[M+Na]^+$) 702.3741 (100%), cacld. 702.3737.

Compound 25:

To a round-bottom flask were added 23 (0.617 g, 0.96 mmol) in $CH_2Cl_2$ (30 mL) and $NEt_3$ (0.20 mL, 1.44 mmol). The mixture was put in ice-bath under $N_2$ followed by addition of mesyl chloride (0.089 mL, 1.15 mmol). After 30 minutes, water (20 mL) and brine (120 mL) were added. The $CH_2Cl_2$ layer was washed with brine (2 x 20 mL) and dried over anhydrous $Na_2SO_4$. The combined aqueous mixture was extracted with EtOAc (3 x 30 mL). The combined extracts were washed with brine and dried over anhydrous $Na_2SO_4$. The desired product (0.676 g, 97% yield) was isolated as a pale yellowish oil after removal of solvent. IR (neat) 2934, 2094, 1454, 1360, 1174, 1112 $cm^{-1}$; $^1H$ NMR ($CDCl_3$, 300 MHZ) δ 4.17 (t, *J* = 6.6 Hz, 2 H), 3.65-3.28 (series of multiplets, 11 H), 3.64-3.00 (series of multiplets, 4 H), 2.97 (s, 3 H), 2.18-1.96 (series of multiplets, 16 H), 1.54-0.94 (series of multiplets, 11 H), 0.89 (d, *J* = 6.6 Hz, 3 H), 0.86 (s, 3 H), 0.63 (s, 3 H); $^{13}C$ NMR ($CDCl_3$, 75 MHZ) δ 80.47, 79.67, 75.92, 70.84, 64.90, 64.37, 64.17, 48.90, 48.86, 48.66, 46.32, 46.26, 42.63, 41.87, 39.70, 37.39, 35.34, 35.28, 35.20, 34.99, 31.61, 29.68, 29.60, 28.96, 27.78, 27.68, 27.57, 25.79, 23.41, 22.95, 22.74, 17.82, 12.50; HRFAB-MS (thioglycerol matrix) *m*/*e*: ($[M+H]^+$) 722.4385 (22.1%), cacld. 722.4387.

Compound 26:

To a 50 mL round-bottom flask was added 24 (0.634 g, 0.936 mmol) and N-benzylmethylamine (2 mL). The

mixture was heated under $N_2$ at 80°C overnight. Excess N-benzylmethylamine was removed under vacuum, and the residue was subjected to $SiO_2$ chromatography (EtOAc/hexanes 1:2). The desired product (0.6236 g, 95% yield) was isolated as a pale yellow oil. IR (neat) 2935, 2106, 1452, 1302, 1116 $cm^{-1}$; $^1H$ NMR ($CDCl_3$, 200 MHZ) δ 7.32-7.24 (m, 5 H), 3.80-3.76 (m, 1 H), 3.70-3.60 (m, 3 H), 3.54 (bs, 1 H), 3.47 (s, 2 H), 3.42-3.10 (m, 10 H), 2.38-2.05 (m, 5 H), 2.17 (s, 3 H), 2.02-0.88 (series of multiplets, 21 H), 0.93 (d, *J* = 7.0 Hz, 3 H), 0.91 (s, 3 H), 0.66 (s, 3 H); $^{13}C$ NMR ($CDCl_3$, 50 MHz) δ 139.60, 129.34, 128.38, 127.02, 81.22, 80.10, 76.71, 67.85, 67.29, 66.65, 62.45, 58.38, 51.65, 51.44, 51.16, 46.50, 46.21, 42.40, 42.20, 41.93, 39.72, 35.80, 35.34, 35.05, 34.76, 33.65, 28.93, 27082, 27.75, 27.38, 24.10, 23.45, 22.98, 22.91, 18.05, 12.50; HRFAB-MS (thioglycerol+$Na^+$ matrix) *m*/*e*: ($[M-H]^+$) 703.4748 (90.2%), cacld. 703.4772; ($[M+H]^+$) 705.4911 (100%), cacld 705.4928; ($[M+Na]^+$) 727.4767 (1.5%), cacld. 727.4748.

Compound 27:

To a 50 mL round-bottom flask was added 25 (0.676 g, 0.937 mmol) and *N*-benzylmethylamine (2 mL). The mixture was heated under $N_2$ at 80°C overnight. Excess *N*-benzylmethylamine was removed under vacuum and the residue was subjected to $SiO_2$ chromatography (EtOAc/hexanes 1:2). The desired product (0.672 g, 96% yield) was isolated as a pale yellow oil. IR (neat) 2934, 2096, 1452, 1283, 1107 $cm^{-1}$; $^1H$ NMR ($CDCl_3$, 300 MHz) δ 7.34-7.20 (m, 5 H), 3.68-3.37 (series of multiplets, 13 H), 3.28-3.04 (m, 4 H), 2.33 (t, *J* = 7.0 Hz, 2 H), 2.18 (s, 3 H), 2.20-2.00 (m, 3 H), 1.96-1.56 (series of multiplets, 14 H), 1.54-1.12 (m, 10 H), 1.10-0.96 (m, 3 H) , 0.91 (d, *J* = 8.7 Hz, 3 H) , 0.89 (s, 3 H) , 0.65 (s, 3 H); $^{13}C$ NMR ($CDCl_3$, 75 MHz) δ 139.48, 129.23, 128.30, 126.96, 80.66, 79.81, 76.08, 65.00, 64.46, 64.34, 62.50, 58.37, 49.02, 48.95, 48.75, 46.65, 46.40, 42.69, 42.43, 42.00, 39.83, 35.86, 35.45, 35.30, 35.10, 33.83, 29.81, 29.78, 29.72, 29.09, 27.88, 27.81, 27.66, 24.19, 23.57, 23.06, 22.87, 18.15, 12.62; HRFAB-MS (thioglycerol matrix) *m*/*e*: ($[M+H]^+$) 747.5406 (77.2%), cacld. 747.5398.

Compound 1:

To a round-bottom flask were added 26 (0.684 g, 0.971 mmol) in dry THF (30 mL) and $LiAlH_4$ (113.7 mg, 3.0 mmol) under $N_2$. The mixture was stirred at room temperature for 12 hours, and then $Na_2SO_4 \cdot 10\ H_2O$ powder (10 g) was added slowly. After the grey color disappeared, the mixture was filtered through Celite and washed with dry THF. The product (0.581 g, 95% yield) was obtained as a colorless glass. IR (neat) 3372, 2937, 1558, 1455, 1362, 1102 $cm^{-1}$; $^1H$ NMR ($CDCl_3$, 300 MHz) δ 7.34-7.20 (m, 5 H), 3.68-3.48 (m, 5 H), 3.47 (s, 2 H), 3.29 (bs, 1 H), 3.22-3.00 (m, 3 H), 2.96-2.80 (m, 6 H), 2.32 (t, *J* = 6.8, 5.4 Hz, 2 H), 2.17 (s, 3 H), 2.20-2.00 (m, 3 H), 1.96-0.96 (series of multiplets, 27 H), 0.93 (d, *J* = 6. 8 Hz, 3 H), 0.90, (s, 3 H), 0.67 (s, 3 H); $^{13}C$ NMR ($CDCl_3$, 75 MHz) δ 139.50, 129.22, 128.31, 126.96, 80.76, 79.85, 76.10, 70.90, 70.33, 70.24, 62.48, 58.27, 46.55, 46.45, 42.72, 42.58, 42.33, 41.99, 39.77, 35.78, 35.37, 35.01, 33.73, 29.07, 27.95, 27.71, 24.06, 23.46, 22.99, 18.14, 12.55; HRFAB-MS (thioglycerol matrix) *m*/*e*: ($[M+H]^+$) 627.5211 (100%), cacld. 627.5213.

HCl salt of compound 1:

Compound 1 was dissolved in a minimum amount of $CH_2Cl_2$ and excess HCl in ether was added. Solvent and excess HCl were removed *in vacuo* and a noncrystalline white powder was obtained. $^1H$ NMR (methanol-d4/ 15% $CDCl_3$, 300 MHz) δ 7.61-7.57 (m, 2 H), 7.50-7.48 (m, 3 H), 4.84 (bs, 10 H), 4.45 (bs, 1 H), 4.30 (bs, 1 H), 3.96-3.82 (m, 2 H), 3.78-3.69 (m, 2 H), 3.66 (bs, 1 H), 3.59-3.32 (series of multiplets, 4 H), 3.28-3.02 (m, 8 H), 2.81 (s, 3 H), 2.36-2.15 (m, 4 H), 2.02-1.68 (m, 8 H), 1.64-0.90 (series of multiplets, 12 H), 1.01 (d, *J* = 6.35 Hz, 3 H), 0.96 (s, 3 H), 0.73 (s, 3 H); $^{13}C$ NMR (methanol-d4/15% $CDCl_3$, 75 MHz) δ 132.31, 131.20, 130.92, 130.40, 83.13, 81.09, 78.48, 65.54, 64.98, 64.11, 60.87, 57.66, 47.51, 46.91, 43.52, 43.00, 41.38, 41.19, 41.16, 40.75, 40.30, 36.37, 36.08, 36.00, 35.96, 33.77, 29.68, 29.34, 28.65, 28.37, 24.42, 24.25, 23.33, 21.51, 18.80, 13.04.

Compound 2:

To a round-bottom flask were added 27 (0.82 g, 1.10 mmol) in dry THF (150 mL) and $LiAlH_4$ (125 mg, 3.30 mmol) under $N_2$. The mixture was stirred at room temperature for 12 hours and $Na_2SO_4$ 10 $H_2O$ powder (10 g) was added slowly. After the grey color disappeared, the mixture was filtered through a cotton plug and washed with dry THF. THF was removed *in vacuo* and the residue dissolved in $CH_2Cl_2$ (50 mL). After filtration, the desired product was obtained as a colorless glass (0.73 g, 99% yield). IR (neat) 3362, 2936, 2862, 2786, 1576, 1466, 1363, 1103 $cm^{-1}$; $^1H$ NMR ($CDCl_3$, 300 MHz) δ 7.32-7.23 (m, 5 H), 3.67-3.63 (m, 1 H), 3.60-3.57 (m, 1 H), 3.53 (t, J = 6.4 Hz, 2 H), 3.47 (s, 2 H), 3.46 (bs, 1 H), 3.24-3.17(m, 2 H), 3.12-2.99 (m, 2 H), 2.83-2.74 (series of multiplets, 6 H), 2.30 (t, *J* = 7.3 Hz, 2 H), 2.15 (s, 3 H), 2.20-2.00 (m, 3 H), 1.95-1.51 (series of multiplets, 20 H), 1.51-1.08, (series of multiplets, 10 H), 1.06-0.80 (m, 3 H), 0.87 (d, *J* = 8.1 Hz, 3 H), 0.86 (s, 3 H), 0.61 (s, 3 H); $^{13}C$ NMR ($CDCl_3$, 75 MHz) δ 139.35, 129.16,

128.22, 126.88, 80.44, 79.29, 75.96, 66.70, 66.52, 66.12, 62.45, 58.26, 46.76, 46.27, 42.69, 42.41, 42.02, 40.68, 40.10, 40.02, 39.82, 35.84, 35.47, 35.30, 35.06, 34.15, 34.09, 34.03, 33.80, 28.96, 27.93, 27.75, 27.71, 24.32, 23.53, 23.03, 22.75, 18.17, 12.58; HRFAB-MS (thioglycerol+$Na^+$ matrix) *m/e*: ($[M+Na]^+$) 691.5504 (38.5%), cacld. 691.5502.

HCl salt of compound 2:

Compound 2 was dissolved in a minimum amount of $CH_2Cl_2$ and excess HCl in ether was added. Removal of the solvent and excess HCl gave a noncrystalline white powder. $^1H$ NMR (methanol-d4/15% $CDCl_3$, 300 MHz) δ 7.60-7.59 (m, 2 H), 7.50-7.47 (m, 3 H), 4.82 (bs, 10 H), 4.43 (bs, 1 H), 4.32 (bs, 1 H), 3.85-3.79 (m, 1 H), 3.75-3.68 (m, 1 H), 3.64 (t, *J* = 5.74 Hz, 2 H), 3.57 (bs, 1 H), 3.36-3.28 (m, 2 H), 3.25-3.00 (series of multiplets, 10 H), 2.82 (s, 3 H), 2.14-1.68 (series of multiplets, 19 H), 1.65-1.15 (series of multiplets, 11 H), 0.98 (d, *J* = 6.6 Hz, 3 H), 0.95 (s, 3 H), 0.72 (s, 3 H); $^{13}C$ NMR (methanol-d4/15% $CDCl_3$, 75 MHz) δ 132.21, 131.10, 130.58, 130.28, 81.96, 80.72, 77.60, 66.84, 66.58, 66.12, 61.03, 57.60, 44.16, 42.77, 40.62, 39.57, 39.43, 36.28, 36.03, 35.96, 35.78, 33.65, 29.48, 29.27, 29.11, 29.01, 28.61, 28.56, 28.35, 24.25, 23.56, 23.30, 21.17, 18.64, 12.90.

Compound 4:

A suspension of 1 (79.1 mg, 0.126 mmol) and aminoiminomethanesulfonic acid (50.15 mg, 0.404 mmol) in methanol and chlorform was stirred at room temperature for 24 hours, and the suspension became clear. An ether solution of HCl (1 *M*; 1 mL) was added followed by the removal of solvent with $N_2$ flow. The residue was dissolved in $H_2O$ (5 mL) followed by the addition of 20% aqueous NaOH (0.5 mL). The resulting cloudy mixture was extracted with $CH_2Cl_2$ (4 x 5 mL). The combined extracts were dried over anhydrous $Na_2SO_4$. Removal of solvent gave the desired product (90 mg, 95%) as white powder. m.p. 111-112°C. IR (neat) 3316, 2937, 1667, 1650, 1556, 1454, 1348, 1102 $cm^{-1}$; $^1H$ NMR (5% methanol-d4/ $CDCl_3$, 300 MHz) δ 7.26-7.22 (m, 5 H), 4.37 (bs, 3 H), 3.71-3.51(series of multiplets, 5 H), 3.44 (s, 2 H), 3.39-3.10 (series of multiplets, 10 H), 2.27 (t, *J* = 6.83 Hz, 2 H), 2.13 (s, 3 H), 2.02-0.94 (series of multiplets, 33 H), 0.85 (d, *J* = 5.62 Hz, 3 H), 0.84 (s, 3 H), 0.61 (s, 3 H); $^{13}C$ NMR (5% methanol-d4/ $CDCl_3$, 75 MHz) δ 158.54, 158.48, 158.43, 138.27, 129.47, 128.32, 127.19, 81.89, 80.30, 77.34, 69.02, 68.46, 67.21, 62.36, 58.00, 47.36, 46.18, 43.26, 43.00, 42.73, 42.18, 41.48, 39.32,35.55, 34.97, 34.89, 34.67, 33.63, 28.93, 28.28, 27.53, 27.16, 23.96, 23.28, 23.16, 22.77, 18.36, 12.58; HRFAB-MS (thioglycerol+$Na^+$ matrix) *m/e*: ($[M+H]^+$) 753.5858 (100%), cacld. 753.5867.

HCl salt of compound 4:

Compound 4 was dissolved in minimum amount of $CH_2Cl_2$ and MeOH followed by addition of excess HCl in ether. The solvent was removed by $N_2$ flow, and the residue was subjected to high vacuum overnight. The desired product was obtained as noncrystalline white powder. $^1H$ NMR (methanol-d4/20% $CDCl_3$, 300 MHz) δ 7.58 (bs, 2 H), 7.50-7.48 (m, 3 H), 4.76 (bs, 13 H), 4.45 (d, *J* = 12.9 Hz, 1 H), 4.27 (dd, 1 H, *J* = 12.9, 5.4 Hz), 3.82-3.00 (series of multiplets, 17 H), 2.81-2.80 (m, 3 H), 2.20-1.02 (series of multiplets, 27 H), 0.98 (d, *J* = 6.59 Hz, 3 H), 0.95 (s, 3 H), 0.72 (s, 3 H); $^{13}C$ NMR (methanol-d4/ 20% $CDCl_3$, 75 MHz) δ 158.88, 158.72, 132.00, 131.96, 130.98, 130.15, 82.51, 81.07, 78.05, 68.50, 68.02, 67.94, 67.10, 60.87, 60.53, 57.38, 47.16, 46.91, 43.91, 43.11, 43.01, 42.91, 42.55, 40.28, 39.88, 39.95, 35.90, 35.73, 35.64, 33.53, 29.18, 28.35, 27.99, 24.02, 23.30, 21.35, 18.52, 18.44, 13.06.

Compound 5:

A suspension of 2 (113 mg, 0.169 mmol) and aminoiminomethanesulfonic acid (67.1 mg, 0.541 mmol) in methanol and chlorform was stirred at room temperature for 24 hours. HCl in ether (1 M, 1 mL) was added followed by the removal of solvent with $N_2$ flow. The residue was subject to high vacuum overnight and dissolved in $H_2O$ (5 mL) followed by the addition of 20% NaOH solution (1.0 mL). The resulting mixture was extracted with $CH_2Cl_2$ (5 x 5 mL). The combined extracts were dried over anhydrous $Na_2SO_4$. Removal of solvent gave desired the product (90 mg, 95% yield) as a white solid. m.p. 102-104°C. IR (neat) 3332, 3155, 2939, 2863, 1667, 1651, 1558, 1456, 1350, 1100 $cm^{-1}$; $^1H$ NMR (5% methanol-d4/$CDCl_3$, 300 MHz) δ 7.35-7.24 (m, 5 H), 3.75-3.64 (m, 1 H), 3.57 (bs, 5 H), 3.50 (s, 2 H), 3.53-3.46 (m, 1 H), 3.40-3.10 (series of multiplets, 14 H), 2.34 (t, *J* = 7.31 Hz, 2 H), 2.19 (s, 3 H), 2.13-0.96 (series of multiplets, 36 H), 0.91 (bs, 6 H), 0.66 (s, 3 H); $^{13}C$ NMR (5% methanol-d4/$CDCl_3$, 75 MHz) δ 157.49, 157.31, 157.23, 138.20, 129.52, 128.34, 127.23, 81.17, 79.19, 76.42, 65.63, 65.03, 64.70, 62.36, 58.02, 47.23, 46.24, 42.89, 42.18, 41.45, 39.45, 39.40, 39.30, 38.71, 35.61, 35.55, 35.02, 34.82, 33.69, 29.87, 29.59, 29.42, 28.84, 27.96, 27.56, 23.95, 23.40, 22.82, 22.64, 18.28, 12.54; HRFAB-MS (thioglycerol+$Na^+$ matrix) *m/e*: ($[M+H]^+$) 795.6356 (84.3%), cacld. 795.6337.

Hcl salt of compound 5:

Compound 5 was dissolved in minimum amount of $CH_2Cl_2$ and MeOH followed by the addition of excess HCl in ether. The solvent and excess HCl were removed by $N_2$ flow and the residue was subject to high vacuum overnight. The desired product was obtained as noncrystalline white powder. $^1H$ NMR (methanol-d4/10% $CDCl_3$, 300 MHz) δ 7.62-7.54 (m, 2 H), 7.48-7.44 (m, 3 H), 4.84 (bs, 16 H), 4.46 (d, *J* = 12.7 Hz, 1 H), 4.26 (dd, *J* = 12.7, 3.42 Hz, 1 H), 3.78-3.56 (series of multiplets, 5 H), 3.38-3.05 (series of multiplets, 13 H), 2.80 (d, 3 H), 2.19-2.04 (m, 3 H), 2.02-1.04 (series of multiplets, 30 H), 0.98 (d, *J* = 6.35 Hz, 3 H), 0.95 (s, 3 H), 0.72 (s, 3 H); $^{13}C$ NMR (methanol-d4/10% $CDCl_3$, 75 MHz) δ 158.75, 158.67, 132.32, 131.24, 130.83, 130.43, 82.49, 81.02, 77.60, 66.47, 65.93, 61.19, 60.85, 57.69, 47.79, 47.60, 44.29, 43.07, 40.86, 40.42, 40.19, 40.09, 39.76, 36.68, 36.50, 36.15, 35.94, 33.91, 30.75, 30.46, 29.74, 29.33, 28.71, 24.41, 24.03, 23.38, 22.21, 22.16, 18.59, 18.52, 13.09.

**Example 2**

Compound 28:

A suspension of 19 (0.641 g, 0.614 mmol) and KC (0.40 g, 6.14 mmol) in anhydrous DMSO (5 mL) was stirred under $N_2$ at 80°C overnight followed by the addition of $H_2O$ (50 mL). The aqueous mixture was extracted with EtOAc (4 x 20 mL). The combined extracts were washed with brine once, dried over anhydrous $Na_2SO_4$ and concentrated *in vacuo*. The residue was dissolved in $CH_2Cl_2$ (3 mL) and MeOH (3 mL) and catalytic amount of *p*-toluenesulfonic acid (5.84 mg, 0.03 mmol) was added. The solution was stirred at room temperature for 3 hours before the introduction of saturated $NaHCO_3$ solution (10 mL). After the addition of brine (60 mL), the mixture was extracted with EtOAc (4 x 30 mL). The combined extracts were washed with brine once and dried over anhydrous $Na_2SO_4$ and concentrated. The residue afforded the desired product (0.342 g, 92% yield) as pale yellowish oil after column chromatography (silica gel, EtOAc/ hexanes 2:1). IR (neat) 3479, 2936, 2864, 2249, 1456, 1445, 1366, 1348, 1108 $cm^{-1}$; $^1H$ NMR ($CDCl_3$, 300 MHz) δ 3.76-3.53 (m, 7 H), 3.32-3.06 (series of multiplets, 4 H), 2.57-2.46 (m, 6 H), 2.13-1.00 (series of multiplets, 31 H), 0.93 (d, *J* = 6.35 Hz, 3 H), 0.90 (s, 3 H), 0.67 (s, 3 H); $^{13}C$ NMR ($CDCl_3$, 75 MHz) δ 119.91, 119.89, 80.75, 79.65, 76.29, 65.83, 65.37, 65.19, 63.63, 46.57, 46.44, 42.77, 41.79, 39.71, 35.63, 35.26, 35.02, 32.00, 29.46, 29.03, 27.96, 27.74, 26.64, 26.42, 26.12, 23.56, 22.98, 22.95, 18.24, 14.65, 14.54, 14.30, 12.60; HRFAB-MS (thioglycerol+$Na^+$ matrix) *m*/*e*: ($[M+Na]^+$) 618.4247 (67.8%), cacld. 618.4247.

Compound 29:

To a solution of 28 (0.34 g, 0.57 mmol) in dry $CH_2Cl_2$ (15 mL) under $N_2$ at 0°C was added $NEt_3$ (119.5 μL, 0.857 mmol) followed by the addition of mesyl chloride (53.1 μL, 0.686 mmol). The mixture was allowed to stir at 0°C for 30 minutes before the addition of $H_2O$ (6 mL). After the introduction of brine (60 mL), the aqueous mixture was extracted with EtOAc (4 x 20 mL). The combined extracts were washed with brine once, dried over anhydrous $Na_2SO_4$ and concentrated. To the residue was added *N*-benzylmethylamine (0.5 mL) and the mixture was stirred under $N_2$ at 80°C overnight. Excess *N*-benzylmethylamine was removed *in vacuo* and the residue was subject to column chromatography (silica gel, EtOAc/hexanes 2:1 followed by EtOAc) to afford product (0.35 g, 88% yield) as a pale yellow oil. IR (neat) 2940, 2863, 2785, 2249, 1469, 1453, 1366, 1348, 1108 $cm^{-1}$; $^1H$ NMR ($CDCl_3$, 300 MHz) δ 7.34-7.21 (m, 5 H), 3.76-3.69 (m, 1 H), 3.64-3.50 (m, 4 H), 3.48 (s, 2 H), 3.31-3.05 (series of multiplets, 4 H), 2.52-2.46 (m, 6 H), 2.33 (t, *J* = 7.32 H, 2 Hz), 2.18 (s, 3 H), 2.13-0.95 (series of multiplets, 30 H), 0.91 (d, *J* = 6.80 H, 3 Hz), 0.90 (s, 3 H), 0.66 (s, 3 H); $^{13}C$ NMR ($CDCl_3$, 75 MHz) δ 139.37, 129.17, 128.26, 126.93, 119.96, 119.91, 80.73, 79.59, 76.26, 65.79, 65.35, 65.13, 62.47, 58.25, 46.74, 46.40, 42.72, 42.38, 41.76, 39.68, 35.78, 35.22, 34.98, 33.79, 28.99, 27.92, 27.71, 26.63, 26.38, 26.09, 24.21, 23.54, 22.96, 22.90, 18.28, 14.62, 14.51, 14.26, 12.58; HRFAB-MS (thioglycerol+$Na^+$ matrix) *m*/*e*: ($[M+H]^+$) 699.5226 (100%), cacld. 699.5213.

Compound 3:

A solution of 29 (0.074 g, 0.106 mmol) in anhydrous THF (10 mL) was added dropwise to a mixture of $AlCl_3$ (0.1414 g, 1.06 mmol) and $LiAlH_4$ (0.041 g, 1.06 mmol) in dry THF (10 mL). The suspension was stirred for 24 hours followed by the addition of 20% NaOH aqueous solution (2 mL) at ice-bath temperature. Anhydrous $Na_2SO_4$ was added to the aqueous slurry. The solution was filtered and the precipitate washed twice with THF. After removal of solvent, the residue was subject to column chromatography (silica gel, MeOH/ $CH_2Cl_2$ 1:1 followed by MeOH/$CH_2Cl_2$/$NH_3 \cdot H_2O$ 4:4:1) to afford the desired product (0.038 g, 50% yield) as a clear oil. IR (neat) 3362, 2935, 2863, 2782, 1651, 1574, 1568, 1557, 1471, 1455, 1103 $cm^{-1}$; $^1H$ NMR ($CDCl_3$, 300 MHz) δ 7.32-7.22 (m, 5 H), 3.60-3.02 (series of broad multiplets, 18 H), 2.90-2.70 (m, 5 H), 2.33 (t, *J* = 7.20 Hz, 2 H), 2.24-2.04 (m, 3 H), 2.18 (s, 3 H), 1.96-0.96 (series of

multiplets, 30 H), 0.90 (d, *J* = 7.57 Hz, 3 H), 0.89 (s, 3 H), 0.64 (s, 3 H); $^{13}C$ NMR ($CDCl_3$, 75 MHz) δ 139.44, 129.24, 128.31, 126.97, 80.63, 79.65, 75.97, 68.44, 68.00, 67.96, 62.54, 58.40, 46.77, 46.30, 42.73, 42.43, 42.07, 41.92, 41.74, 41.72, 39.81, 35.82, 35.48, 35.07, 33.84, 31.04, 30.30, 30.10, 29.03, 28.11, 27.82, 27.81, 27.74, 27.67, 27.64, 24.31, 23.50, 23.04, 22.93, 18.22, 12.63; HRFAB-MS (thioglycerol+$Na^+$ matrix) *m*/*e*: ($[M+H]^+$) 711.6139 (100%), cacld. 711.6152; ($[M+Na]^+$) 733.5974 (46.1%), cacld. 733.5972.

## Example 3

Compound 30:

Cholic acid (3.0 g, 7.3 mmol) was dissolved in $CH_2Cl_2$ (50 mL) and methanol (5 mL). Dicyclohexylcarbodiimide (DCC) (1.8 g, 8.8 mmol) was added followed by *N*-hydroxysuccinimide (~100 mg) and benzylmethylamine (1.1 g, 8.8 mmol). The mixture was stirred for 2 hours, then filtered. The filtrate was concentrated and chromatographed ($SiO_2$, 3% MeOH in $CH_2Cl_2$) to give 3.0 g of a white solid (81% yield). m.p. 184-186°C; IR (neat) 3325, 2984, 1678 $cm^{-1}$; $^1H$ NMR ($CDCl_3$, 200 MHz) δ 7.21 (m, 5 H), 4.51 (m, 2 H), 3.87 (m, 1 H), 3.74 (m, 2 H), 3.36 (m, 2 H), 2.84 (s, 3 H), 2.48-0.92 (series of multiplets, 28 H), 0.80 (s, 3 H), 0.58 (d, *J* = 6.5 Hz, 3 H); $^{13}C$ NMR ($CDCl_3$, 50 MHz) δ 174.30, 173.94, 137.36, 136.63, 128.81, 128.46, 127.85, 127.50, 127.18, 126.28, 72.96, 71.76, 68.35, 53.39, 50.65, 48.77, 46.91, 46.33, 41.44, 39.36, 39.18, 35.76, 35.27, 34.76, 33.87, 31.54, 34.19, 31.07, 30.45, 28.11, 27.63, 26.14, 25.59, 24.92, 23.26, 17.51, 12.41; FAB-MS (thioglycerol+$Na^+$ matrix) *m*/*e*: ($[M+H]^+$) 512 (100%), cacld. 512.

Compound 31:

Compound 30 (2.4 g, 4.7 mmol) was added to a suspension of $LiAlH_4$ (0.18 g, 4.7 mmol) in THF (50 mL). The mixture was refluxed for 24 hours, then cooled to 0°C. An aqueous solution of $Na_2SO_4$ was carefully added until the grey color of the mixture dissipated. The salts were filtered out, and the filtrate was concentrated *in vacuo* to yield 2.1 g of a white solid (88%). The product proved to be of sufficient purity for further reactions. m.p. 70-73°C; IR (neat) 3380, 2983, 1502 $cm^{-1}$ ; $^1H$ NMR ($CDCl_3$, 300 MHz) δ 7.23 (m, 5 H), 3.98 (bs, 2 H), 3.81 (m, 3 H), 3.43 (m, 3 H), 2.74 (m, 2 H), 2.33 (m, 3 H), 2.25 (s, 3 H), 2.10-0.90 (series of multiplets, 24 H), 0.98 (s, 3 H), 0.78 (s, 3 H); $^{13}C$ NMR ($CDCl_3$, 75 MHz) δ 135.72, 129,63, 128.21, 128.13, 125.28, 72.91, 71.63, 62.05, 60.80, 56.79, 47.00, 46.23, 41.44, 40.81, 39.41, 35.42, 35.24, 34.63, 34.02, 33.22, 31.73, 30.17, 29.33, 29.16, 28.02, 27.49, 26.17, 25.55, 23.10, 22.48, 22.33, 17.54, 12.65; FAB-MS (thioglycerol matrix) *m*/*e*: ($[M+H]^+$) 498 (100%), cacld. 498.

Compound 32:

Compound 31 (0.36 g, 0.72 mmol) was dissolved in $CH_2Cl_2$ (15 mL) and Bocglycine (0.51 g, 2.89 mmol), DCC (0.67 g, 3.24 mmol) and dimethylaminopyridine (DMAP) (-100 mg) were added. The mixture was stirred under $N_2$ for 4 hours then filtered. After concentration and chromatography ($SiO_2$, 5% MeOH in $CH_2Cl_2$), the product was obtained as a 0.47 g of a clear glass (68%). $^1H$ NMR ($CDCl_3$, 300 MHz) δ 7.30 (m, 5 H), 5.19 (bs, 1 H), 5.09 (bs, 3 H), 5.01 (bs, 1 H), 4.75 (m, 1 H), 4.06-3.89 (m, 6 H), 2.33 (m, 2 H), 2.19 (s, 3 H) 2.05-1.01 (series of multiplets, 26 H), 1.47 (s, 9 H), 1.45 (s, 18 H), 0.92 (s, 3 H), 0.80 (d, *J* = 6.4 Hz, 3 H), 0.72 (s, 3 H). $^{13}C$ NMR ($CDCl_3$, 75 MHz) δ 170.01, 169.86, 169.69, 155.72, 155.55, 139.90, 129.05, 128.17, 126.88, 79.86, 76.53, 75.09, 72.09, 62, 35, 57.88, 47.78, 45.23, 43.12, 42.79, 42.16, 40.81, 37.94, 35.51, 34.69, 34.57, 34.36, 33.30, 31.31, 29.66, 28.80, 28.34, 27.22, 26.76, 25.61, 24.02, 22.83, 22.47, 17.'93, 12.19; FAB-MS (thioglycerol matrix) *m*/*e*: ($[M+H]^+$) 970 (100%), cacld. 970.

Compound 33:

Compound 31 (0.39 g, 0.79 mmol) was dissolved in $CH_2Cl_2$ (15 mL) and Boc-β-alanine (0.60 g, 3.17 mmol), DCC (0.73 g, 3.56 mmol) and dimethylaminopyridine (DMAP) (~100 mg) were added. The mixture was stirred under $N_2$ for 6 hours then filtered. After concentration and chromatography ($SiO_2$, 5% MeOH in $CH_2Cl_2$), the product was obtained as a 0.58 g of a clear glass (72%). IR (neat) 3400, 2980, 1705, 1510 $cm^{-1}$; $^1H$ NMR ($CDCl_3$, 300 MHz) δ 7.27 (m, 5 H), 5.12 (bs, 4 H), 4.93 (bs, 1 H), 4.71 (m, 1 H), 3.40 (m, 12 H), 2.59-2.48 (m, 6 H), 2.28 (m, 2 H), 2.17 (s, 3 H) 2.05-1.01 (series of multiplets, 26 H), 1.40 (s, 27 H), 0.90 (s, 3 H), 0.77 (d, *J* = 6.1 Hz, 3 H), 0.70 (s, 3 H). $^{13}C$ NMR ($CDCl_3$, 75 MHz) δ 171.85, 171.50, 171.44, 155.73, 138.62, 129.02, 128.09, 126.87, 79.18, 75.53, 74.00, 70.91, 62.20, 57.67, 47.84, 44.99, 43.28, 41.98, 40.73, 37.67, 36.12, 34.94, 34.65, 34.47, 34.20, 33.29, 31.23, 29.57, 28.74, 28.31, 28.02, 27.86, 27.12, 26.73, 25.46, 24.86, 23.95, 22.77, 22.39, 17.91, 12.14; HRFAB-MS (thioglycerol+$Na^+$ matrix) *m*/*e*: ($[M+H]^+$) 1011.6619 (100%), cacld. 1011.6634.

Compound 6:

Compound 32 (0.15 g, 0.15 mmol) was stirred with excess 4 *N* HCl in dioxane for 40 minutes. The dioxane and HCl were removed in vacuo leaving 0.12 g of a clear glass (-100%). $^1$H NMR ($CD_3OD$, 300 MHz) δ 7.62 (bs, 2 H), 7.48 (bs, 3 H), 5.30 (bs, 1 H), 5.11 (bs, 1 H), 4.72 (bs, 1 H), 4.46 (m, 1 H), 4.32 (m, 1H) 4.05-3.91 (m, 4 H), 3.10 (m, 2 H), 2.81 (s, 3 H), 2.15-1.13 (series of multiplets, 25 H), 1.00 (s, 3 H), 0.91 (bs, 3 H), 0.82 (s, 3 H). $^{13}$C NMR ($CD_3OD$, 125 MHz) δ 166.86, 166.50, 131.09, 130.18, 129.17, 128.55, 76.60, 75.43, 72.61, 72.04, 70.40, 66.22, 60.07, 58.00, 57.90, 54.89, 54.76, 46.44, 44.64, 43.39, 42.22, 38.56, 36.78, 34.14, 33.92, 33.84, 31.82, 30.54, 29.67, 28.79, 27.96, 26.79, 26.00, 24.99, 23.14, 22.05, 21.82, 19.91, 17.27, 11.60; HRFAB-MS (thioglycerol+$Na^+$ matrix) *m*/*e*: ([M - 4 Cl - 3 H]$^+$) 669.4576 (100%), cacld. 669.4591.

Compound 7:

Compound 33 (0.20 g, 0.20 mmol) was stirred with excess 4 N HCl in dioxane for 40 minutes. The dioxane and HCl were removed *in vacuo* leaving 0.12 g of a clear glass (~100%). $^1$H NMR ($CD_3OD$, 500 MHz) δ 7.58 (bs, 2 H), 7.49 (bs, 3 H), 5.21 (bs, 1 H), 5.02 (bs, 1 H), 4.64 (m, 1 H), 4.44 (m, 1 H), 4.28 (m, 1 H), 3.30-2.84 (m, 14 H), 2.80 (s, 3 H), 2.11-1.09 (series of multiplets, 25 H), 0.99 (s, 3 H), 0.89 (d, *J* = 4.1 Hz, 3 H), 0.80 (s, 3 H); $^{13}$C NMR ($CD_3OD$, 125 MHz) δ 171.92, 171.56, 171.49, 132.44, 131.32, 131.02, 130.51, 78.13, 76.61, 61.45, 57.94, 46.67, 44.80, 42.36, 40.85, 39.33, 37.03, 36.89, 36.12, 36.09, 35.79, 35.63, 33.81, 33.10, 32.92, 32.43, 30.28, 28.43, 28.04, 26.65, 24.02, 22.86, 21.98, 18.70, 12.68; HRFAB-MS (thioglycerol+$Na^+$ matrix) *m*/*e*: ([M - 4 Cl - 3 H]$^+$) 711.5069 (43%), cacld. 711.5061.

**Example 4**

Compound 34:

Diisopropyl azodicarboxylate (DIAD) (1.20 mL, 6.08 mmol) was added to triphenylphosphine (1.60 g, 6.08 mmol) in THF (100 mL) at 0°C and was stirred for half an hour during which time the yellow solution became a paste. Compound 14 (2.58 g, 4.06 mmol) and p-nitrobenzoic acid (0.81 g, 4.87 mmol) were dissolved in THF (50 mL) and added to the paste. The resulted mixture was stirred at ambient temperature overnight. Water (100 mL) was added and the mixture was made slightly basic by adding $NaHCO_3$ solution followed by extraction with EtOAc (3 x 50 mL). The combined extracts were washed with brine once and dried over anhydrous $Na_2SO_4$. The desired product (2.72 g, 85% yield) was obtained as white powder after $SiO_2$ chromatography ($Et_2O$/hexanes 1:2). m.p. 207-209°C; IR (KBr) 3434, 3056, 2940, 2868, 1722, 1608, 1529,1489, 1448, 1345 cm$^{-1}$; $^1$H NMR ($CDCl_3$, 300 MHz) δ 8.30-8.26 (m, 2 H), 8.21-8.16 (m, 2 H), 7.46-7.42 (m, 6 H), 7.31-7.18 (m, 9 H)5.33 (bs, 1 H), 4.02 (bs, 1 H), 3.90 (bs, 1 H), 3.09-2.97 (m, 2 H), 2.68 (td, *J* = 14.95, 2.56 Hz, 1 H), 2.29-2.19 (m, 1 H), 2.07-1.06 (series of multiplets, 24 H), 1.01 (s, 3 H), 0.98 (d, *J* = 6.6 Hz, 3 H), 0.70 (s, 3 H); $^{13}$C NMR ($CDCl_3$, 75 MHz) δ 164.21, 150.56, 144.70, 136.79, 130.77, 128.88, 127.86, 126.98, 123.70, 86.47, 73.24, 73.00, 68.70, 64.22, 47.79, 46.79, 42.15, 39.76, 37.47, 35.52, 35.34, 34.23, 33.79, 32.46, 31.12, 28.74 27.71, 26.85, 26.30, 25.16. 23.41, 17.98, 12.77; HRFAB-MS (thioglycerol+$Na^+$ matrix) *m*/*e*: ([M+Na]$^+$) 808.4203 (53.8%), cacld. 808.4189. The nitrobenzoate (2.75 g, 3.5 mmol) was dissolved in $CH_2Cl_2$ (40 mL) and MeOH (20 mL) and 20% aqueous NaOH (5 mL) were added. The mixture was heated up to 60°C for 24 hours. Water (100 mL) was introduced and extracted with EtOAc. The combined extracts were washed with brine and dried over anhydrous $Na_2SO_4$. The desired product (1.89 g, 85% yield) was obtained as white solid after $SiO_2$ chromatography (3% MeOH in $CH_2Cl_2$ as eluent). m.p. 105-106°C; IR (KBr) 3429, 3057, 2936, 1596, 1489, 1447, 1376, 1265, 1034, 704 cm$^{-1}$; $^1$H NMR ($CDCl_3$, 300 MHz) δ 7.46-7.42 (m, 6 H), 7.32-7.19 (m, 9 H), 4.06 (bs, 1 H), 3.99 (bs, 1 H), 3.86 (bd, *J* = 2.44 Hz, 1 H), 3.09-2.97 (m, 2 H), 2.47 (td, *J* = 14.03, 2.44 Hz, 1 H), 2.20-2.11 (m, 1 H), 2.04-1.04 (series of multiplets, 25 H), 0.97 (d, *J* = 6.59 Hz, 3 H), 0.94 (s, 3 H), 0.68 (s, 3 H); $^{13}$C NMR ($CDCl_3$, 75 MHz) δ 144.70, 128.88, 127.86, 126.97, 86.45, 73.31, 68.84, 67.10, 64.23, 47.71, 46.74, 42.10, 39.70, 36.73, 36.73, 36.15, 35.53, 35.45, 34.45, 32.46, 29.93, 28.67, 27.86, 27.71, 26.87, 26.04, 23.43, 23.16, 17.94, 12.75; HRFAB-MS (thioglycerol+$Na^+$ matrix) *m*/*e*: ([M+Na]$^+$) 659.4064 (100%), cacld. 659.4076.

Compound 35:

To a round-bottom flask were added 34 (2.0 g, 3.14 mmol), NaH (60% in mineral oil, 3.8 g, 31.4 mmol) and THF (150 mL). The suspension was refluxed for 2 hours followed by the addition of allyl bromide (2.72 mL, 31.4 mL). After refluxing for 28 hours, another 10 eq. of NaH and allyl bromide were added. After 72 hours, another 10 eq. of NaH and allyl bromide were added. After 115 hours, TLC showed almost no starting material or intermediates. Water (100 mL) was added to the suspension carefully, followed by extraction with EtOAc (5 x 50 mL). The combined extracts

were washed with brine and dried over anhydrous $Na_2SO_4$. The desired product (1.81 g, 79% yield) was obtained as a yellowish glass after $SiO_2$ chromatography (5% EtOAc/hexanes). IR (neat) 3060, 3020, 2938, 2865, 1645, 1596, 1490, 1448, 1376, 1076, 705 $cm^{-1}$; $^1H$ NMR ($CDCl_3$, 300 MHz) $\delta$ 7.46-7.42 (m, 6 H), 7.31-7.18 (m, 9 H), 6.06-5.85 (m, 3 H), 5.35-5.20 (m, 3 H), 5.15-5.06 (m, 3 H), 4.10-4.00 (m, 2 H), 3.93-3.90 (m, 2 H), 3.85-3.79 (ddt, *J* = 13.01, 4.88, 1.59 Hz, 1 H), 3.73-3.66 (ddt, *J* = 13.01, 5.38, 1.46 Hz, 1 H), 3.58 (bs, 1 H), 3.54 (bs, 1 H), 3.32 (d, *J* = 2.93 Hz, 1 H), 3.07-2.96 (m, 2 H), 2.36 (td, *J* = 13.67, 2.68 Hz, 1 H), 2.24-2.10 (m, 2 H), 2.03-1.94 (m, 1 H), 1.87-0.86 (series of multiplets, 20 H), 0.91 (s, 3 H), 0.90 (d, *J* = 6.83 Hz, 3 H), 0.64 (s, 3 H); $^{13}C$ NMR ($CDCl_3$, 75 MHz) $\delta$ 144.77, 136.29, 136.21, 136.13, 128.90, 127.86, 126.94, 116.13, 115.51, 115.42, 86.44, 81.11, 75.65, 73.92, 69.40, 68.81, 64.43, 46.68, 46.54, 42.93, 39.93, 36.98, 35.66, 35.14, 35.14, 32.83, 32.54, 30.48, 28.51, 27.72, 27.64, 26.82, 24.79, 23.65, 23.43, 23.40, 18.07, 12.80; HRFAB-MS (thioglycerol+$Na^+$ matrix) *m*/*e*: ($[M+H]^+$) 757.5185 (12.9%), cacld. 757.5196.

Compound 36:

Ozone was bubbled through a solution of 35 (0.551 g, 0.729 mmol) in $CH_2Cl_2$ (40 mL) and MeOH (20 mL) at -78°C until the solution turned a deep blue. Excess ozone was blown off with oxygen. Methylsulfide (1 mL) was added followed by the addition of $NaBH_4$ (0.22 g, 5.80 mmol) in 5% NaOH solution and methanol. The resulted mixture was stirred overnight at room temperature and washed with brine. The brine was then extracted with EtOAc (3 x 20 mL). The combined extracts were dried over $Na_2SO_4$. The desired product (0.36 g, 65% yield) was obtained as a colorless glass after $SiO_2$ chromatography (5% MeOH/$CH_2Cl_2$). IR (neat) 3396, 3056, 2927, 1596, 1492, 1462, 1448, 1379, 1347, 1264, 1071 $cm^{-1}$; $^1H$ NMR ($CDCl_3$, 300 MHz) $\delta$ 7.46-7.42 (m, 6 H), 7.32-7.18 (m, 9 H), 3.77-3.57 (series of multiplets, 10 H), 3.48-3.44 (m, 2 H), 3.36-3.30 (m, 2 H), 3.26-3.20 (m, 1 H), 3.04-2.99 (m, 2 H), 2.37-0.95 (series of multiplets, 27 H), 0.92 (s, 3 H), 0. 91 (d, *J* = 6. 59 Hz, 3 H), 0.67 (s, 3 H); $^{13}C$ NMR ($CDCl_3$, 75 MHz) $\delta$ 144.69, 128.87, 127.84, 126.94, 86.44, 81.05, 76.86, 74.65, 69.91, 69.22, 68.77, 64.24, 62.44, 62.42, 62.26, 46.92, 46.54, 42.87, 39.73, 36.86, 35.52, 35.13, 32.82, 32.54, 30.36, 28.71, 27.61, 27.44, 26.79, 24.82, 23.51, 23.38, 23.31, 18.28, 12.74; HRFAB-MS (thioglycerol+$Na^+$ matrix) *m*/*e*: ($[M+Na]^+$) 791.4844 (96.4%), cacld. 791.4863.

Compound 37:

$NEt_3$ (0.23 mL, 1.66 mmol) was added to a solution of 36 (0.364 g, 0.47 mmol) in dry $CH_2Cl_2$ (30 mL) at 0°C under $N_2$ followed by the introduction of mesyl chloride (0.12 mL, 1.56 mmol). The mixture was stirred for 10 minutes and $H_2O$ (10 mL) added to quench the reaction, followed by extraction with EtOAc (3 x 30 mL). The combined extracts were washed with brine and dried over anhydrous $Na_2SO_4$. $SiO_2$ chromatography (EtOAc/hexanes 1:1) gave the desired product (0.411 g, 86% yield) as white glass. IR (neat) 3058, 3029, 2939, 2868, 1491, 1461, 1448, 1349, 1175, 1109, 1019 $cm^{-1}$; $^1H$ NMR ($CDCl_3$, 300 MHz) $\delta$ 7.46-7.42 (m, 6 H), 7.31-7.19 (m, 9 H), 4.35-4.26 (m, 6 H), 3.84-3.74 (m, 2 H), 3.64-3.56 (m, 4 H), 3.49-3.34 (m, 3 H), 3.06 (s, 3 H), 3.04 (s, 3 H), 3.02 (s, 3 H), 3.09-2.95 (m, 2 H), 2.28 (bt, *J* = 14.89 Hz, 1 H), 2.09-0.86 (series of multiplets, 21 H), 0.92 (s, 3 H), 0.90 (d, *J* = 6.78 Hz, 3 H) , 0.66 (s, 3 H); $^{13}C$ NMR ($CDCl_3$, 75 MHz) $\delta$ 144.66, 128.86, 127.86, 126.97, 86.46, 81.28, 77.18, 75.00, 70.14, 69.89, 69.13, 66.49, 65.85, 65.72, 64.22, 47.06, 46.35, 42.77, 39.58, 37.81, 37.64, 37.55, 36.75, 35.48, 35.02, 32.59, 32.52, 30.27, 28.43, 27.56, 27.52, 26.92, 24.62, 23.34, 23.25, 23.10, 18.24, 12.64; HRFAB-MS (thioglycerol+$Na^+$ matrix) *m*/*e*: ($[M+Na]^+$) 1025.4207 (100%), cacld. 1025.4189.

Compound 38:

The suspension of 37 (0.227 g, 0.227 mmol) and $NaN_3$ (0.147 g, 2.27 mmol) in dry DMSO (5 mL) was stirred at 80°C overnight, diluted with $H_2O$ (50 mL) and extracted with EtOAc (3 x 20 mL). The extracts were washed with brine once and dried over anhydrous $Na_2SO_4$. $SiO_2$ chromatography (EtOAc/hexanes 1:8) afforded the desired product (0.153 g, 80% yield) as a yellow oil. IR (neat) 2929, 2866, 2105, 1490, 1466, 1448, 1107, 705 $cm^{-1}$; $^1H$ NMR ($CDCl_3$, 300 MHz) $\delta$ 7.46-7.42 (m, 6 H), 7.32-7.19 (m, 9 H), 3.80-3.74 (m, 1 H), 3.70-3.55 (series of multiplets, 5 H), 3.41-3.19 (series of multiplets, 9 H), 3.04-2.98 (m, 2 H), 2.41 (td, *J* = 13.1, 2.44 Hz, 1 H), 2.29-2.14 (m, 2 H), 2.04-0.86 (series of multiplets, 20 H), 0.93 (s, 3 H), 0.91 (d, *J* = 6.60 Hz, 3 H), 0.66 (s, 3 H); $^{13}C$ NMR ($CDCl_3$, 75 MHz) $\delta$ 144.78, 128.93, 127.87, 126.96, 86.46, 81.30, 77.16, 75.21, 67.99, 67.44, 67.03, 64.41, 51.64, 51.57, 51,33, 46.71, 46.30, 42.35, 39.75, 36.72, 35.64, 35.20, 32.52, 32.42, 30.17, 28.63, 27.80, 27.22, 26.90, 24.80, 23.55, 23.30, 23.24, 18.23, 12.65; HRFAB-MS (thioglycerol+$Na^+$ matrix) *m*/*e*: ($[M+Na]^+$) 866.5049 (96.9%), cacld. 866.5057.

Compound 39:

*p*-Toluenesulfonic acid (1.72 mg) was added into the solution of 38 (0.153 g, 0.18 mmol) in $CH_2Cl_2$ (5 mL) and MeOH (5 mL), and the mixture was stirred for 2.5 hours. Saturated $NaHCO_3$ solution (5 mL) was introduced

followed by the introduction of brine (30 mL). The aqueous mixture was extracted with EtOAc and the combined extracts washed with brine and dried over $Na_2SO_4$. The desired product (0.10 g, 92% yield) was obtained as a pale yellowish oil after $SiO_2$ chromatography (EtOAc/hexanes 1:3). IR (neat) 3426, 2926, 2104, 1467, 144.1, 1347, 1107 $cm^{-1}$; $^1H$ NMR ($CDCl_3$, 300 MHz) δ 3.81-3.74 (m, 1 H), 3.71-3.54 (m, 7 H), 3.41-3.19 (m, 9 H), 2.41 (td, *J* = 13.61, 2.32 Hz, 1 H), 2.30-2.14 (m, 2 H), 2.07-1.98 (m, 1 H), 1.94-0.95 (series of multiplets, 21 H), 0.95 (d, *J* = 6.35 Hz, 3 H), 0.93 (s, 3 H), 0.69 (s, 3 H); $^{13}C$ NMR ($CDCl_3$, 75 MHz) δ 81.22, 77.08, 75.13, 67.94, 67.36, 66.97, 63.76, 51.59, 51.51, 51.26, 46.51, 46.24, 42.31, 39.68, 36.64, 35.58, 35.12, 32.34, 31.92, 30.11, 29.55, 28.54, 27.82, 27.16, 24.75, 23.47, 23.23, 23.18, 18.15, 12.56; HRFAB-MS (thioglycerol+$Na^+$ matrix) *m*/*e*: ($[M+Na]^+$) 624.3966 (54.9%), cacld. 624.3962.

Compound 40:

To a solution of 39 (0.10 g, 0.166 mmol) in $CH_2Cl_2$ (8 mL) at 0°C was added $NEt_3$ (34.8 μL, 0.25 mmol) under $N_2$ followed by the introduction of mesyl chloride (15.5 μL, 0.199 mmol). The mixture was stirred 15 minutes. Addition of $H_2O$ (3 mL) and brine (20 mL) was followed by extraction with EtOAc (4 x 10 mL). The combined extracts were washed with brine once and dried over $Na_2SO_4$. After removal of solvent, the residue was mixed with N-benzylmethylamine (0.5 mL) and heated to 80°C under $N_2$ overnight. Excess *N*-benzylmethylamine was removed *in vacuo* and the residue was subjected to $SiO_2$ chromatography (EtOAc/hexanes 1:4) to give the product (0.109 g, 93% yield) as a yellow oil. IR (neat) 2936, 2784, 2103, 1467, 1442, 1346, 1302, 1106, 1027 $cm^{-1}$; $^1H$ NMR ($CDCl_3$, 300 MHz) δ 7.32-7.23 (m, 5 H), 3.81-3.74 (m, 1 H), 3.71-3.55 (m, 5 H), 3.47 (s, 2 H), 3.41-3.19 (m, 9 H), 2.46-2.11 (m, 5 H), 2.18 (s, 3 H), 2.03-0.85 (series of multiplets, 20 H), 0.93 (s, 3 H), 0.93 (d, *J* = 6.35 Hz, 3 H,), 0.67 (s, 3 H); $^{13}C$ NMR ($CDCl_3$, 75 MHz) δ 139.54, 129.26, 128.32, 126.97, 81.26, 77.12, 75.17, 67.98, 67.42, 67.00, 62.50, 58.41, 51.61, 51.54, 51.29, 46.66, 46.28, 42.46, 42.32, 39.72, 36.68, 35.76, 35.16, 33.75, 32.38, 30.15, 28.59, 27.85, 27.19, 24.77, 24.15, 23.53, 23.28, 23.22, 18.28, 12.60; HRFAB-MS (thioglycerol+$Na^+$ matrix) *m*/*e*: ($[M+H]^+$) 705.4929 (100%), cacld. 705.4928.

Compound 8:

A suspension of 40 (0.109 g, 0.155 mmol) and $LiAlH_4$ (23.5 mg, 0.62 mmol) in THF (20 mL) was stirred under $N_2$ overnight. $Na_2SO_4 \cdot 10H_2O$ was carefully added and stirred until no grey color persisted. Anhydrous $Na_2SO_4$ was added and the white precipitate was filtered out and rinsed with dry THF. After removal of solvent, the residue was dissolved in minimum $CH_2Cl_2$ and filtered. The desired product (0.091 g, 94% yield) was obtained as a colorless oil after the solvent was removed. IR (neat) 3371, 3290, 3027, 2938, 2862, 2785, 1586, 1493, 1453, 1377, 1347, 1098 $cm^{-1}$; $^1H$ NMR ($CDCl_3$, 300 MHz)_δ 7.31-7.21 (m, 5 H), 3.65-3.53 (m, 4 H), 3.47 (s, 2 H), 3.42-3.34 .(m, 2 H), 3.30 (bs, 1 H), 3.26-3.20 (m, 1 H), 3.14-3.09 (m, 1 H), 2.89-2.81 (m, 6 H), 2.39-2.27 (m, 3 H), 2.17 (s, 3 H), 2.15-0.88 (series of multiplets, 29 H), 0.93 (d, *J* = 6.59 Hz, 3 H) , 0.92 (s, 3 H) , 0.67 (s, 3 H); $^{13}C$ NMR ($CDCl_3$, 75 MHz) δ 139.34, 129.16, 128.24, 126.90, 80.75, 76.44, 74.29, 70.58, 69.88, 69.75, 62.47, 58.27, 46.66, 46.47, 42.75, 42.63, 42.51, 42.35, 39.77, 36.87, 35.73, 35.04, 33.77, 32.90, 30.38, 28.71, 27.70, 27.32, 24.89, 24.09, 23.53, 23.36, 23.25, 18.24, 12.62; HRFAB-MS (thioglycerol+$Na^+$ matrix) *m*/*e*: ($[M+H]^+$) 627.5199 (23.3%), cacld. 627.5213.

Compound 9:

To a solution of 23 (0.18 g, 0.28 mmol) in dry DMF (4 mL) were added NaH (0.224 g, 60% in mineral oil, 5.60 mmol) and 1-bromo octane (0.48 mL, 2.80 mmol). The suspension was stirred under $N_2$ at 65°C overnight followed by the introduction of $H_2O$ (60 mL) and extraction with ether (4 x 20 mL). The combined extracts were washed with brine and dried over $Na_2SO_4$. $SiO_2$ chromatography (hexanes and 5% EtOAc in hexanes) afforded the desired product (0.169 g, 80% yield) as a pale yellowish oil. IR (neat) 2927, 2865, 2099, 1478, 1462, 1451, 1350, 1264, 1105 $cm^{-1}$; $^1H$ NMR ($CDCl_3$, 300 MHz) δ 3.69-3.35 (series of multiplets, 15 H), 3.26-3.02 (series of multiplets, 4 H), 2.19-2.02 (m, 3 H), 1.97-1.16 (series of multiplets, 37 H), 1.12-0.99 (m, 2 H), 0.92-0.86 (m, 9 H), 0.65 (s, 3 H) ; $^{13}C$ NMR ($CDCl_3$, 75 MHz) δ 80.69, 79.84, 76.13, 71.57, 71.15, 65.07, 64.49, 64.39, 49.08, 48.99, 48.80, 46.68, 46.45, 42.72, 42.05, 39.88, 35.74, 35.49, 35.36, 35.14, 32.42, 32.03, 30.01, 29.85, 29.81, 29.76, 29.67, 29.48, 29.14, 27.92, 27.80, 27.70, 26.58, 26.42, 23.59, 23.09, 22.92, 22.86, 18.11, 14.31, 12.65; HRFAB-MS (thioglycerol+$Na^+$ matrix) *m*/*e*: ($[M+Na]^+$) 778.5685 (22.1%), cacld. 778 5683. The triazide (0.169 g, 0.224 mmol) and $LiAlH_4$ (0.025 g, 0.67 mmol) were suspended in anhydrous THF (10 mL) and stirred under $N_2$ at room temperature overnight followed by careful introduction of $Na_2SO_4$ hydrate. After the grey color disappeared, anhydrous $Na_2SO_4$ was added and stirred. The white precipitate was removed by filtration and washed with THF. After removal of solvent, the residue was dissolved in 1 *M* hydrochloric acid and the aqueous solution was extracted with ether (5 mL) once. The aqueous solution was then made basic by adding 20% aqueous NaOH solution followed by extraction with $Et_2O$ (4 x 5 mL). The combined extracts were washed, dried and concentrated. The residue was then subject to $SiO_2$ chromatography ($MeOH/CH_2Cl_2$ (1:1) followed by MeOH/ $CH_2Cl_2/NH_3 \cdot H_2O$ (4:4:1)) to afford the desired product (0.091 g, 60% yield) as a colorless oil. IR (neat) 3361, 2927,

2855, 1576, 1465, 1351, 1105 $cm^{-1}$; $^1H$ NMR ($CD_3OD$, 300 MHz) δ 4.86 (bs, 6 H), 3.77-3.72 (m, 1 H), 3.70-3.61 (m, 1 H), 3.57-3.53 (m, 3 H), 3.43-3.38 (m, 4 H), 3.34-3.27 (m, 2 H), 3.18-3.10 (m, 2 H), 2.84-2.71 (m, 6 H), 2.22-2.07 (m, 3 H), 2.00-1.02 (series of multiplets, 39 H), 0.97-0.88 (m, 9 H) , 0.71 (s, 3 H) ; $^{13}C$ NMR ($CD_3OD$, 75 MHz) δ 82.20, 81.00, 77.62, 72.52, 72.06, 68.00, 67.92, 67.39, 48.20, 47.53, 44.26, 43.40, 41.42, 41.15, 40.84, 40.35, 36.88, 36.73, 36.42, 36.11, 34.24, 34.05, 33.94, 33.67, 33.17, 30.95, 30.72, 30.62, 29.81, 29.35, 28.87, 28.79, 27.51, 24.57, 23.90, 23.83, 23.44, 18.76, 14.62, 13.07; HRFAB-MS (thioglycerol matrix) *m/e*: ($[M+H]^+$) 678.6133 (100%), cacld. 678.6149.

Compound 10:

A suspension of 23 (0.126 g, 0.196 mmol) and $LiAlH_4$ (0.037 g, 0.98 mmol) in THF (40 mL) was stirred at room temperature under $N_2$ overnight followed by careful addition of $Na_2SO_4 \cdot 10H_2O$. After the grey color in the suspension disappeared, anhydrous $Na_2$ $SO_4$ was added and stirred until organic layer became clear. The white precipitate was removed by filtration and washed with twice THF. The THF was removed *in vacuo*, and the residue was subject to $SiO_2$ chromatography ($MeOH/CH_2Cl_2/NH_3 \cdot H_2O$ (4:4:1)) to afford the desired product (0.066 g, 60% yield) as a colorless oil. IR (neat) 3365, 2933, 2865, 1651, 1471, 1455, 1339, 1103 $cm^{-1}$; $^1H$ NMR ($CDCl_3$/30% CD $_3$ OD, 300 MHz) δ 4.43 (bs, 7 H), 3.74-3.68 (m, 1 H), 3.66-3.60 (m, 1 H), 3.57-3.50 (m, 5 H), 3.34-3.25 (M, 2 H), 3.17-3.06 (M, 2 H), 2.84-2.74 (M, 6 H), 2.19-2.01 (M, 3 H), 1.97-0.96 (series of multiplets, 27 H), 0.94 (d, *J* = 7.2 Hz, 3 H), 0.92 (s, 3 H), 0.69 (s, 3 H) ; $^{13}C$ NMR ($CDCl_3$, 75 MHz) δ 80.44, 79.27, 75.77, 66.59, 66.53, 65.86, 62.51, 46.21, 45.84, 42.55, 41.53, 40.09, 39.43, 39.31, 39.02, 35.16, 34.93, 34.86, 34.57, 32.93, 32.71, 31.57, 28.66, 28.33, 27.64, 27.22, 23.04, 22.40, 22.29, 17.60, 11.98; HRFAB-MS (thioglycerol+$Na^+$ matrix) *m/e*: ($[M+H]^+$) 566.4889 (8.9%), cacld. 566.4897.

## Example 5

Compound 42:

Compound 41 was prepared following the method reported by D. H. R. Barton, J. Wozniak, *S. Z. Zard, A Short And Efficient Degredation of The Bile Acid Side Chain. Some Novel Reactions of Sulphines And A-ketoesters*, Tetrahedron, 1989, vol. 45, 3741-3754. A mixture of 41 (1.00 g, 2.10 mmol), ethylene glycol (3.52 mL, 63 mmol) and *p*-TsOH (20 mg, 0.105 mmol) was refluxed in benzene under $N_2$ for 16 hours. Water formed during the reaction was removed by a Dean-Stark moisture trap. The cooled mixture was washed with $NaHCO_3$ solution (50 mL) and extracted with $Et_2O$ (50 mL, 2 x 30 mL). The combined extracts were washed with brine and dried over anhydrous $Na_2SO_4$. Removal of the solvent gave the product (1.09 g, 100%) as a white glass. IR (neat) 2939, 2876, 1735, 1447, 1377, 1247, 1074, 1057, 1039 $cm^{-1}$; $^1H$ NMR ($CDCl_3$, 300 MHz) δ 5.10 (t, *J* = 2.70 Hz, 1 H) , 4.92 (d, *J* = 2.69 Hz, 1 H), 4.63-4.52 (m, 1 H), 3.98-3.80 (m, 4 H), 2.32 (t, *J* = 9.51 Hz, 1 H), 2.13 (s, 3 H), 2.08 (s, 3 H), 2.05 (s, 3 H), 2.00-1.40 (series of multiplets, 15 H), 1.34-0.98 (m, 3 H), 1.20 (s, 3 H), 0.92 (s, 3 H), 0.82 (s, 3 H); $^{13}C$ NMR ($CDCl_3$, 75 MHz) δ 170.69, 170.63, 170.47, 111.38, 75.07, 74.23, 70.85, 64.95, 63.43, 49.85, 44.73, 43.39, 41.11, 37.37, 34.84, 34.80, 34.52, 31.42, 29.18, 27.02, 25.41, 24.16, 22.72, 22.57, 22.44, 21.73, 21.63, 13.40; HRFAB-MS (thioglycerol+$Na^+$ matrix) *m/e*: ($[M+H]^+$) 521.3106 (38.6%), cacld. 521.3114. The triacetate (1.09 g, 2.10 mmol) was dissolved in MeOH (50 mL). NaOH (0.84 g, 21 mmol) was added to the solution. The suspension was then refluxed under $N_2$ for 24 hours. MeOH was then removed *in vacuo* and the residue was dissolved in $Et_2O$ (100 mL) and washed with $H_2O$, brine, and then dried over anhydrous $Na_2SO_4$. The desired product (0.80 g, 96 % yield) was obtained as white solid after removal of solvent. m.p. 199-200°C. IR (neat) 3396, 2932, 1462, 1446, 1371, 1265, 1078, 1055 $cm^{-1}$; $^1H$ NMR (10 % $CD_3OD$ in $CDCl_3$, 300 MHz) δ 4.08-3.83 (series of multiplets, 9 H), 3.44-3.34 (m, 1 H), 2.41 (t, *J* = 9.28 Hz, 1 H), 2.22-2.10 (m, 2 H), 1.96-1.50 (series of multiplets, 12 H), 1.45-0.96 (series of multiplets, 4 H), 1.32 (s, 3 H), 0.89 (s, 3 H), 0.78 (s, 3 H); $^{13}C$ NMR (10% $CD_3OD$ in $CDCl_3$, 75 MHz) δ 112.11, 72.35, 71.57, 68.09, 64.54, 63.24, 49.36, 45.90, 41.48, 41.45, 39.18, 38.79, 35.29, 34.71, 34.45 29.90, 27.26, 26.60, 23.65, 22.54, 22.44, 22.35, 13.46; HRFAB-MS (thioglycerol+$Na^+$ matrix) *m/e*: ($[M+Na]^+$) 417.2622 (87.3%), cacld. 417.2617.

Compound 43:

To a round-bottom flask were added 42 (0.80 g, 2.03 mmol) and dry THF (100 mL) followed by the addition of NaH (60% in mineral oil, 0.81 g, 20.3 mmol). The suspension was refluxed under $N_2$ for 30 minutes before the addition of allyl bromide (1.75 mL, 20.3 mmol). After 48 hours of reflux, another 10 eq. of NaH and allyl bromide were added. After another 48 hours, TLC showed no intermediates left. Cold water (50 mL) was added to the cooled suspension. The resulted mixture was extracted with $Et_2O$ (60 mL, 2 x 30 mL). The combined extracts were washed with brine and dried over anhydrous $Na_2SO_4$. $SiO_2$ column chromatography (6% EtOAc in hexanes) gave the desired product (0.94 g, 90% yield) as a pale yellow oil. IR (neat) 3076, 2933, 2866, 1645, 1446, 1423, 1408, 1368, 1289, 1252, 1226, 1206, 1130, 1080, 1057 $cm^{-1}$; $^1H$ NMR ($CDCl_3$, 300 MHz) δ 6.02-5.84 (m, 3 H), 5.31-5.04 (m, 6 H), 4.12-4.05

(m, 2 H), 4.01-3.81 (m, 7 H), 3.70 (dd, *J* = 12.94, 5.62 Hz, 1 H), 3.55 (t, *J* = 2.56 Hz, 1 H), 3.33 (d, *J* = 2.93 Hz, 1 H), 3.18-3.08 (m, 1 H), 2.65 (t, *J* = 10.01 Hz, 1 H), 2.32-2.14 (m, 3 H), 1.84-1.45 (series of multiplets, 10 H), 1.41-1.22 (m, 3 H), 1.27 (s, 3 H), 1.14-0.92 (m, 2 H), 0.89 (s, 3 H), 0.75 (s, 3 H); $^{13}$C NMR ($CDCl_3$, 75 MHz) δ 136.38, 136.07, 136.00, 116.31, 115.54, 115.38, 112.34, 80.07, 79.22, 75.05, 69.83, 69.34, 68.82, 65.14, 63.24, 48.80, 45.96, 42.47, 42.15, 39.40, 35.55, 35.16, 35.15, 29.04, 28.22, 27.52, 24.21, 23.38, 23.11, 22.95, 22.58, 13.79; HRFAB-MS (thioglycerol+$Na^+$ matrix) *m*/*e*: ($[M+Na]^+$) 537.3549 (100%), cacld. 537.3556.

Compound 44:

To the solution of 43 (0.94 g, 1.83 mmol) in dry THF (50 mL) was added 9-BBN (0.5 M solution in THF, 14.7 mL, 7.34 mmol) and the mixture was stirred under $N_2$ at room temperature for 12 hours before the addition of 20% NaOH solution (4 mL) and 30% $H_2O_2$ solution (4 mL). The resulted mixture was then refluxed for an hour followed by the addition of brine (100 mL) and extracted with EtOAc (4 x 30 mL). The combined extracts were dried over anhydrous $Na_2SO_4$. After the removal of solvent, the residue was purified by $SiO_2$ column chromatography (EtOAc followed by 10% MeOH in $CH_2Cl_2$) to give the product (0.559 g, 54% yield) as a colorless oil. IR (neat) 3410, 2933, 2872, 1471, 1446, 1367, 1252, 1086 $cm^{-1}$; $^1$H NMR ($CDCl_3$, 300 MHz) δ 4.02-3.52 (series of multiplets, 17 H), 3.41-3.35 (m, 1 H), 3.29 (d, *J* = 2.44 Hz, 1 H), 3.22-3.15 (m, 3 H), 2.58 (t, *J* = 10.01 Hz, 1 H), 2.27-1.95 (m, 3 H), 1.83-1.48 (series of multiplets, 16 H), 1.40-0.93 (series of multiplets, 5 H), 1.27 (s, 3 H), 0.90 (s, 3 H), 0.75 (s, 3 H); $^{13}$C NMR ($CDCl_3$, 75 MHz) δ 112.41, 80.09, 79.09, 76.31, 66.70, 66.02, 65.93, 64.80, 63.26, 61.53, 61.25, 60.86, 48.59, 45.80, 42.51, 41.72, 39.10, 35.36, 35.02, 34.98, 32.87, 32.52, 32.40, 28.88, 27.94, 27.21, 24.33, 23.02, 22.84 (2 C's), 22.44, 13.69; HRFAB-MS (thioglycerol+$Na^+$ matrix) *m*/*e*: ($[M+Na]^+$) 591.3881 (100%), cacld. 591.3873.

Compound 45:

To a solution of 50 (0.559 g, 0.98 mmol) in acetone (40 mL) and water (4 mL) was added PPTS (0.124 g, 0.49 mmol) and the solution was refluxed under $N_2$ for 16 hours. The solvent was removed under reduced pressure. Water (40 mL) was then added to the residue and the mixture was extracted with EtOAc (40 mL, 2 x 20 mL). The combined extracts were washed with brine, dried and evaporated to dryness. $SiO_2$ column chromatography (8% MeOH in $CH_2Cl_2$) of the residue afforded the desired product (0.509 g, 98% yield) as clear oil. IR (neat) 3382, 2941, 2876, 1699, 1449, 1366, 1099 $cm^{-1}$; $^1$H NMR ($CDCl_3$, 300 MHz) δ 3.83-3.72 (m, 8 H), 3.66 (t, *J* = 5.62 Hz, 2 H), 3.54 (bs, 2 H), 3.43-3.28 (m, 4 H), 3.24-3.12 (m, 2 H), 2.26-2.00 (m, 4 H), 2.08 (s, 3 H), 1.98-1.50 (series of multiplets, 15 H), 1.42-0.96 (series of multiplets, 6 H), 0.90 (s, 3 H), 0.62 (s, 3 H); $^{13}$C NMR ($CDCl_3$, 75 MHz) δ 210.49, 78.87 (2 C's), 76.30, 66.86, 66.18, 65.69, 61.74, 61.43, 60.71, 55.31, 48.05, 43.02, 41.58, 39.53, 35.28, 35.09, 34.96, 32.77, 32.70, 32.31, 31.12, 28.72, 27.88, 27.14, 23.47, 22.75, 22.47, 22.34, 13.86; HRFAB-MS (thioglycerol+$Na^+$ matrix) *m*/*e*: ($[M+Na]^+$) 547.3624 (100%), cacld. 547.3611.

Compound 46:

To a solution of 45 (0.18 g, 0.344 mmol) in dry $CH_2Cl_2$ (10 mL) at 0°C was added $Et_3N$ (0.168 mL, 1.20 mmol) followed by the addition of mesyl chloride (0.088 mL, 1.13 mmol). After 10 minutes, $H_2O$ (3 mL) and brine (30 mL) were added. The mixture was extracted with EtOAc (30 mL, 2 x 10 mL) and the extracts were washed with brine and dried over anhydrous $Na_2SO_4$. After removal of solvent, the residue was dissolved in DMSO (5 mL) and $NaN_3$ (0.233 g, 3.44 mmol). The suspension was heated up to 50°C under $N_2$ for 12 hours. $H_2O$ (50 mL) was added to the cool suspension and the mixture was extracted with EtOAc (30 mL, 2 x 10 mL) and the extracts were washed with brine and dried over anhydrous $Na_2SO_4$. $SiO_2$ column chromatography (EtOAc/hexanes 1:5) afforded the product (0.191 g, 88% yield for two steps) as a pale yellow oil. IR (neat) 2933, 2872, 2096, 1702, 1451, 1363, 1263, 1102 $cm^{-1}$; $^1$H NMR ($CDCl_3$, 300 MHz) δ 3.72-3.64 (m, 2 H), 3.55-3.24 (series of multiplets, 11 H), 3.18-3.02 (m, 2 H), 2.22-2.02 (m, 4 H), 2.08 (s, 3 H), 1.95-1.46 (series of multiplets, 15 H), 1.38-0.96 (series of multiplets, 6 H), 0.89 (s, 3 H), 0.62 (s, 3 H); $^{13}$C NMR ($CDCl_3$, 75 MHz) δ 210.36, 79.69, 79.22, 75.98, 65.08, 64.80, 64.53, 55.31, 48.93, 48.86, 48.76, 48.06, 43.03, 41.91, 39.66, 35.44, 35.31, 35.12, 31.04, 29.77, 29.69, 29.67, 28.99, 28.10, 27.65, 23.60, 22.99, 22.95, 22.50, 14.00; HRFAB-MS (thioglycerol+$Na^+$ matrix) *m*/*e*: ($[M+Na]^+$) 622.3820 (100%), cacld. 622.3805.

Compound 11:

Compound 46 (0.191 g, 0.319 mmol) was dissolved in dry THF (20 mL) followed by the addition of $LiAlH_4$ (60.4 mg, 1.59 mmol). The grey suspension was stirred under $N_2$ at room temperature for 12 hours. $Na_2SO_4 \cdot 10H_2O$ powder was carefully added. After the grey color in the suspension disappeared, anhydrous $Na_2SO_4$ was added and the precipitate was filtered out. After the removal of solvent, the residue was purified by column chromatography (silica

gel, $MeOH/CH_2Cl_2/28\% NH_3 \cdot H_2O$ 3:3:1). After most of the solvent was rotavapped off from the fractions collected, 5% HCl solution (2 mL) was added to dissolve the milky residue. The resulted clear solution was then extracted with $Et_2O$ (2 x 10 mL). 20% NaOH solution was then added until the solution became strongly basic. $CH_2Cl_2$ (20 mL, 2 x 10 mL) was used to extract the basic solution. The combined extracts were dried over anhydrous $Na_2SO_4$ and removal of solvent gave the desired product (0.115 g, 69% yield) as a colorless oil. From $^1H$ NMR it appears that this compound was a mixture of two stereoisomers at $C_{20}$ with a ratio of approximately 9:1. The stereoisomers were not separated, but used as recovered. Spectra for the most abundant isomer: IR (neat) 3353, 2926, 2858, 1574, 1470, 1366, 1102 $cm^{-1}$; $^1H$ NMR (20% $CDCl_3$ in $CD_3OD$, 300 MHz) δ 4.69 (bs, 7 H), 3.76-3.69 (m, 1 H), 3.63-3.53 (m, 5 H), 3.50-3.40 (m, 1 H), 3.29 (bs, 1 H), 3.18-3.07 (m, 2 H), 2.94-2.83 (m, 1 H), 2.81-2.66 (m, 5 H), 2.23-2.06 (m, 4 H), 1.87-1.50 (series of multiplets, 15 H), 1.39-0.96 (series of multiplets, 6 H), 1.11 (d, *J* = 6.10 Hz, 3 H), 0.93 (s, 3 H) , 0.75 (s, 3 H); $^{13}C$ NMR (20% $CDCl_3$ in $CD_3OD$, 75 MHz) δ 81.46, 80.67, 77.32, 70.68, 67.90, 67.66, 67.18, 50.32, 47.17, 43.30, 43.06, 40.74, 40.64, 40.38, 40.26, 36.31, 36.28, 35.93, 34.30, 34.02, 33.29, 29.63, 29.31, 28.43, 26.10, 24.67, 24.09, 23.96, 23.50, 13.30 for the major isomer; HRFAB-MS (thioglycerol+$Na^+$ matrix) *m*/*e*: ($[M+H]^+$) 524.4431 (64.2%), cacld. 524.4427.

**Example 6**

Compound 47:

To a solution of 23 (0.15 g, 0.233 mmol) in dry $CH_2Cl_2$ (15 mL) at 0°C was added $Et_3N$ (48.8 μL, 0.35 mmol) followed by the addition of $CH_3SO_2Cl$ (21.7 μL, 0.28 mmol). The mixture was stirred for 15 minutes before $H_2O$ (3 mL) was added. Saturated NaCl solution (20 mL) was then added, and the mixture was extracted with EtOAc (40 mL, 2 x 20 mL). The combined extracts were washed with brine and dried over anhydrous $Na_2SO_4$. The solvent was rotovapped off and to the residue were added NaBr (0.12 g, 1.17 mmol) and DMF (10 mL). The suspension was heated up to 80°C under $N_2$ for 2 hours. DMF was removed under vacuum and the residue was chromatographed on silica (EtOAc/hexanes 1:10) to give the desired product (0.191 g, 97% yield) as a pale yellow oil. $^1H$ NMR ($CDCl_3$, 300 MHz) δ 3.69-3.35 (series of multiplets, 13 H), 3.28-3.02 (series of multiplets, 4 H), 2.18-2.04 (m, 3 H), 2.00-1.60 (series of multiplets, 16 H), 1.58-0.96 (series of multiplets, 11 H), 0.92 (d, *J* = 6.34 Hz, 3 H), 0.89 (s, 3 H), 0.66 (s, 3 H); $^{13}C$ NMR ($CDCl_3$, 75 MHz) δ 80.62, 79.81, 76.08, 65.07, 64.50, 64.34, 49.03, 48.98, 48.79, 46.49, 46.46, 42.73, 42.02, 39.85, 35.47, 35.34, 35.12, 34.79, 34.72, 29.82, 29.80, 29.74, 29.11, 27.91, 27.78, 27.69, 23.55, 23.07, 22.88, 18.10, 12.62; HRFAB-MS (thioglycerol+$Na^+$ matrix) *m*/*e*: ($[M-H]^+$) 706.3609 (63.1%), cacld. 706.3591; 704.3616 (52.8%), cacld. 704.3611.

Compound 48:

Compound 47 (0.191 g, 0.269 mmol) and 23 (0.295 g, 0.459 mmol) was dissolved in DMF (3 mL, distilled over BaO at 6 mm Hg before use) followed by the addition of NaH (0.054 g, 60% in mineral oil). The suspension was stirred under $N_2$ at room temperature for 24 hours. $H_2O$ (100 mL) was added to quench excess NaH and the mixture was then extracted with $Et_2O$ (40 mL, 3 x 20 mL) and the combined extracts were washed with brine and dried over anhydrous $Na_2SO_4$. The desired product (0.177 g, 52% yield based on compound 23) was obtained as a pale yellow oil after $SiO_2$ chromatography (EtOAc/hexanes 1:6, then 1:2). IR (neat) 2940, 2862, 2095, 1472, 1456, 1362, 1263, 1113 $cm^{-1}$; $^1H$ NMR($CDCl_3$, 300 MHz) δ 3.68-3.35 (series of multiplets, 26 H), 3.28-3.02 (series of multiplets, 8 H), 2.20-2.04 (m, 6 H), 1.96-1.60 (series of multiplets, 30 H), 1.52-0.98 (series of multiplets, 12 H), 0.91 (d, *J* = 6.59 Hz, 6 H), 0.89 (s, 6 H), 0.65 (s, 6 H); $^{13}C$ NMR($CDCl_3$, 75 MHz) δ 80.68, 79.83, 76.13, 71.71, 65.06, 64.48, 64.39, 49.08, 48.98, 48.80, 46.64, 46.44, 42.71, 42.04, 39.88, 35.73, 35.49, 35.36, 35.14, 32.41, 29.84, 29.81, 29.76, 29.14, 27.92, 27.78, 27.69, 26.58, 23.59, 23.08, 22.92, 18.12, 12.64.

Compound 12:

Compound 48 (0.219 g, 0.173 mmol) was dissolved in dry THF (10 mL) followed by the addition of $LiAlH_4$ (65 mg, 1.73 mmol). The grey suspension was stirred under $N_2$ at room temperature for 12 hours. $Na_2SO_4 \cdot 10H_2O$ powder was carefully added. After the grey color in the suspension disappeared, anhydrous $Na_2SO_4$ was added and the precipitate was filtered out. After the removal of solvent, the residue was purified by column chromatography (silica gel, $MeOH/CH_2Cl_2/28\% NH_3 \cdot H_2O$ 2.5:2.5:1). After most of the solvent was rotavapped off from the fractions collected, 5% HCl solution (2 mL) was added to dissolve the milky residue. The resulted clear solution was then extracted with $Et_2O$ (2 x 10 mL). 20% NaOH solution was then added until the solution became strongly basic. $CH_2Cl_2$ (20 mL, 2 x 10 mL) was used to extract the basic solution. The combined extracts were dried over anhydrous $Na_2SO_4$ and removal of solvent gave the desired product (0.147 g, 76% yield) as a white glass. IR (neat) 3364, 3287, 2934, 2861, 1596, 1464,

1363, 1105 $cm^{-1}$; $^1H$ NMR (20% $CDCl_3$ in $CD_3OD$, 500 MHz) δ 4.74 (bs, 12 H), 3.75-3.70 (m, 2 H), 3.65-3.61 (m, 2 H), 3.57-3.52 (m, 6 H), 3.40 (t, *J* = 3.60 Hz, 4 H), 3.30 (bs, 4 H), 3.16-3.10 (m, 4 H), 2.84-2.73 (m, 12 H), 2.18-2.07 (m, 6 H), 1.97-1.61 (series of multiplets, 30 H), 1.58-0.98 (series of multiplets, 24 H), 0.95 (d, *J* = 6.84 Hz, 6 H), 0.94 (s, 6 H), 0.70 (s, 6 H); $^{13}C$ NMR (20% $CDCl_3$ in $CD_3OD$, 125 MHz) δ 81.70, 80.52, 77.09, 72.34, 67.75 (2 C's), 67.07, 47.80, 47.13, 43.76, 42.87, 41.20, 40.65, 40.58, 40.14, 36.43, 36.25, 36.08, 35.77, 34.15, 33.87 (2 C's), 33.18, 29.55, 28.92, 28.47, 28.42, 27.25, 24.27, 23.54, 23.41, 18.70, 13.07; HRFAB-MS (thioglycerol+$Na^+$ matrix) *m*/*e*: ($[M+H]^+$) 1113.9625 (68.8%), cacld. 1113.9610.

## Example 7

Testing of Compounds with Gram-Negative Bacteria

MIC and MBC Measurements

General:

Tryptic soy broth (TSB) was made by dissolving 27.5 grams of tryptic soy broth without dextrose (DIFCO Laboratories) in 1 liter of deionized water and sterilizing at 121°C for 15 minutes. Solid agar (TSA) plates were made by dissolving 6.4 grams of tryptic soy broth and 12 grams of agar (purified grade, Fischer Scientific) in 800 mL of deionized water and sterilizing at 121°C for 20 minutes. Aliquots (20 mL) of the homogeneous solution were then poured in sterile plastic petri dishes (100 x 15 mm, Fisher Scientific). Solutions of compounds 1-12 were made by dissolving the HCl salt of the respective compound into an appropriate amount of deionized and sterilized water followed by microfiltration.

Representative procedure for measuring MIC and MBC values:

A suspension was prepared of *E. coli* (ATCC 10798) containing ~$10^6$ CFU (colony forming units)/mL from a culture incubated in TSB at 37°C for 24 hours. Aliquots of 1 mL of the suspension were added to test tubes containing 1 mL TSB and incrementally varied concentrations of 1-12 and/or erythromycin or novobiocin. In the sensitization experiments, erythromycin or novobiocin were added 15 minutes later than 1-12. The samples were subjected to stationary incubation at 37°C for 24 hours. Sample turbidity was determined by measuring absorption at 760 nm (HP 8453 UV-Visible Chemstation, Hewlett Packard). Additionally, an alliquot from each of the samples showing no measurable turbidity was subcultured on TSA plates (alliquots were diluted to provide fewer than 300 CFU). Colonies that grew on the subculture after overnight incubation were counted and the number of CFU/mL in the samples were calculated. The calculated values were compared to the number of CFU/mL in the original inoculum. MIC values were determined as the concentrations of the studied compounds at which the number of CFU/mL remained constant or decreased after incubation for 24 hours. The MBC values were determined as the lowest concentrations of the studied compounds that allowed less than 0.1% of the original bacterial suspension to survive.

Results

The stereochemistry of the steroid backbone results in different activity of the cholic acid derivatives (compare 2 and 8, Tables 1, 2, 6 and 7). Guanidine groups attached to the steroid provide lower MIC values than compounds containing amine groups (compare 1, 2, 4 and 5, compare Tables 1-8). The length of the tether between the amine or guanidine groups and the steroid backbone also influences activity (compare 1-3, Tables 1, 2, 6 and 7). Ester tethers between amine groups and the steroid backbone provide compounds with MIC values that are higher than the corresponding compounds containing ether tethers (compare 1, 2, 6 and 7, Tables 1 and 2). The group attached to the backbone at C-20 or C-24 also influences the activity of the cholic acid derivatives. A long carbon chain attached to the steroid via an ether linkage at C-24 lowers the MIC of the compound as compared to the compound with a hydroxyl group at C-24 (compare 2, 9 and 10, Tables 1, 2, 6 and 7). Short chains of carbon or oxygen attached at C-20 decrease the MIC values of the cholic acid derivatives (compare 10 and 11, Tables 1 and 2). Covalently linking the cholic acid derivatives increases the activity of the compounds (compare 10 and 12, Tables 1 and 2).

Table 1

| Measurement of MIC and MBC values of 1-12 with *E. coli* (ATCC 10798) | | |
|---|---|---|
| Compound | MIC (μg/mL) | MBC (μg/mL) |
| 1 | 20 | 34 |
| 2 | 7 | 16 |
| 3 | 6 | a |
| 4 | 5 | 10 |
| 5 | 2 | 4 |
| 6 | 65 | a |
| 7 | 28 | a |
| 8 | 46 | a |
| 9 | 3 | 10 |
| 10 | 36 | 60 |
| 11 | 140 | >160 |
| 12 | 4 | 4 |

[a] Value not measured.

Table 2

| Measurement of the concentrations of 1-12 required to lower the MIC of erythromycin from 70 μg/mL to 1 μg/mL with *E. coli* (ATCC 10798). | | |
|---|---|---|
| Compound | MIC (μg/mL) | MBC (μg/mL) |
| 1 | 2 | 20 |
| 2 | 1 | 10 |
| 3 | 1.5 | a |
| 4 | 1.5 | 10 |
| 5 | 1 | 3 |
| 6 | 22 | a |
| 7 | 2.5 | a |
| 8 | 10 | a |
| 9 | 3 | 3 |
| 10 | 2 | 50 |
| 11 | 40 | >160 |
| 12 | 1.5 | 2.5 |

[a]Value not measured.

Table 3

| Measurement of the concentrations of 1, 2, 4 and 5 required to lower the MIC of novobiocin from >500 μg/mL to 1 μg/mL with *E. coli* (ATCC 10798). | | |
|---|---|---|
| Compound | MIC (μg/mL) | MBC (μg/mL) |
| 1 | 20 | 34 |

Table 3 (continued)

| Measurement of the concentrations of 1, 2, 4 and 5 required to lower the MIC of novobiocin from >500 μg/mL to 1 μg/mL with *E. coli* (ATCC 10798). | | |
|---|---|---|
| Compound | MIC (μg/mL) | MBC (μg/mL) |
| 2 | 7 | 16 |
| 4 | 5 | 10 |
| 5 | 2 | 4 |
| 11 | 40 | 140 |
| 12 | 2.5 | a |

[a] Value not measured.

Table 4

| Measurement of MIC and MBC values of 1, 2, 4 and 5 with *E. coli* (ATCC 25922). | | |
|---|---|---|
| Compound | MIC (μg/mL) | MBC (μg/mL) |
| 1 | 25 | 40 |
| 2 | 10 | 20 |
| 4 | 6 | 9 |
| 5 | 2 | 4 |

Table 5

| Measurement of the concentrations of 1, 2, 4 and 5 required to lower the MIC of erythromycin from 60 μg/mL to 1 μg/mL with *E. coli* (ATCC 25922). | | |
|---|---|---|
| Compound | MIC (μg/mL) | MBC (μg/mL) |
| 1 | 2 | 14 |
| 2 | 1 | 5 |
| 4 | 1 | 5 |
| 5 | 1.5 | 1.5 |

Table 6

| Measurement of MIC and MBC values of 1-5, 8-12 with *P. aureginosa* (ATCC 27853). | | |
|---|---|---|
| Compound | MI C (μg/mL) | MBC (μg/mL) |
| 1 | 15 | >50 |
| 2 | 9 | 40 |
| 3 | 16 | a |
| 4 | 15 | 40 |
| 5 | 6 | 15 |
| 8 | 50 | a |
| 9 | 8 | a |
| 10 | 23 | a |

[a] Value not measured.

Table 7

| Measurement of the concentrations of 1- 5, 8-12 required to lower the MIC of erythromycin from 240 μg/mL to 5 μg/mL with *P. aureginosa* (ATCC 27853). | | |
|---|---|---|
| Compound | MIC (μg/mL) | MBC (μg/mL) |
| 1 | 8 | 45 |
| 2 | 4 | 25 |
| 3 | 6 | a |
| 4 | 5 | 40 |
| 5 | 3 | 10 |
| 8 | 40 | a |
| 9 | 5 | a |
| 10 | 7 | a |

[a] Value not measured.

Table 8

| Measurement of the concentrations of 1, 2, 4 and 5 required to lower the MIC of novobiocin from >500 μg/mL to 1 μg/mL with *P. aureginosa* (ATCC 27853). | |
|---|---|
| Compound | MIC (μg/mL) |
| 1 | 6 |
| 2 | 4 |
| 4 | 6 |
| 5 | 6 |

**Example 8**

Demonstration of Membrane Disrupting Properties of the Cholic Acid Derivatives

Using a technique described by J. M. Shupp, S. E. Travis, L. B. Price, R. F. Shand, P. Keim, *Rabid Bacterial Permeabilization Reagent Useful For Enzyme Assays*, Biotechniques, 1995, vol. 19, 18-20, we have shown that the cholic acid derivatives increase the permeability of the outer membrand of Gram-negative bacteria. The values for half maximum luminescence (indicating permeabilization of the outer membrane allowing luciferin to enter the cell) for 2 is 7 μg/mL and for 10 is 33 μg/mL. These values correspond to the measured MICs of 2 and 10.

## Example 9

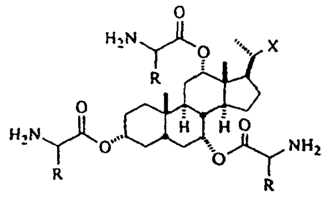

The R groups correspond to the side chain of any combination of amino acids (D or L)

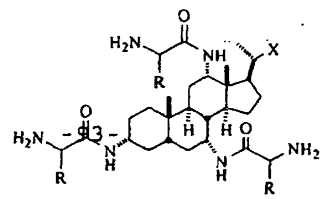

The R groups correspond to the side chain of any combination of amino acids (D or L)

Alterations in the stereochemistry within the steroid (AB ring juncture in this case)

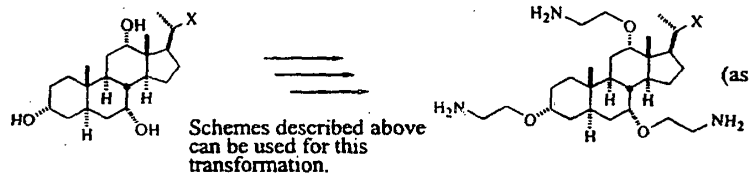


(as an example)

Alterations in the saturation within the steroid (AB ring juncture in this case)

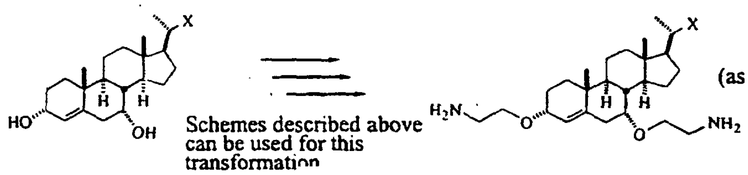


(as an example)

Alterations in the number of hydroxyl groups on the steroid (OH-12 in this case).

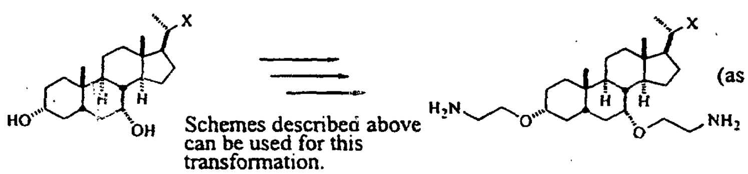


(as an example)

Alterations in other groups on the steroid (in the A ring in this case)

(as an example)

(or suitable protecting group)

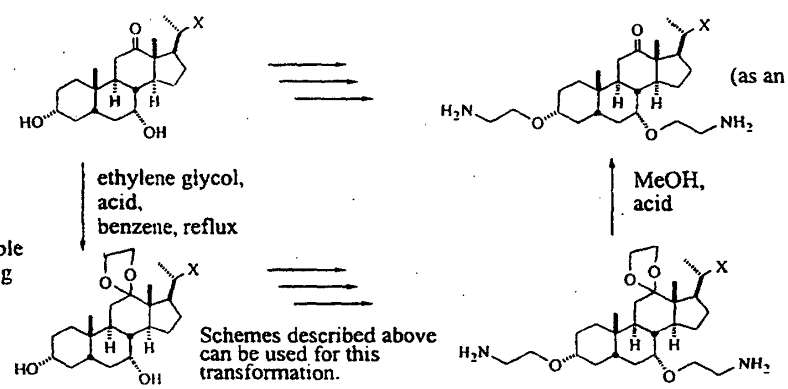


Descriptions of the steroid starting materials shown above can be found in *Dictionary of Steroids*, Hill, R. A.; Kirk, D. N.; Makin, H. L. J.; Murphy, G. M., eds., Chapman and Hall: New York, 1991.

## Claims

**1.** A compound according to formula I

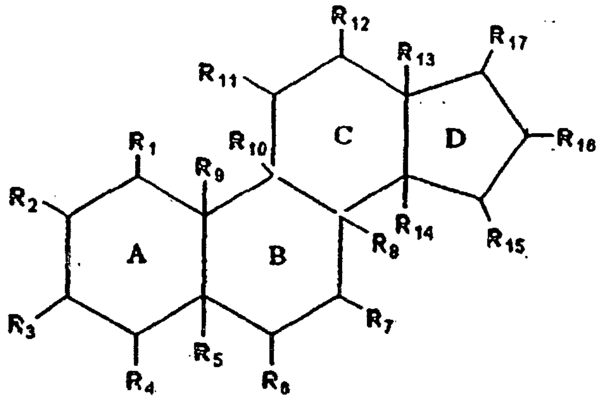

I

wherein:

fused rings A, B, C, and D are independently saturated or fully or partially unsaturated; and

$R_1$ through $R_4$, $R_6$, $R_7$, $R_{11}$, $R_{12}$, $R_{15}$, $R_{16}$, and $R_{17}$ is each independently selected from the group consisting of hydrogen, hydroxyl, (C1-C10) alkyl, (C1-C10) hydroxyalkyl, (C1-C10) alkyloxy-(C1-C10) alkyl, a substituted or unsubstituted (C1-C10) aminoalkyl wherein the substituents are selected from the group consisting of hydroxyl, protected hydroxyl, amino, protected amino, carboxy, protected carboxy, cyano, methyl sulfonylamino, alkoxy, alkyl, aryl, N-benzyl, acyloxy, nitro, C1-C10 haloalkyl, C2-C6 alkenyl, C2-C6 alkynyl, oxo, a (C1-C10) alkyloxy-(C1-C10)alkyl linking group attached to a second steroid of formula I, (C1-C10) aminoalkyloxy, (C1-C10) aminoalkylcarboxy, (C1-C10) aminoalkylaminocarbonyl, H2N-HC(Q5)-C(O)-O-, H2N-HC(Q5)-C(O)-N(H)-, (C1-C10) azidoalkyloxy, (C1-C10) cyanoalkyloxy, P.G.-HN-CH(Q5)-C(O)-O-, (C1-C10) guanidinoalkyl oxy, and (C1-C10) guanidinoalkyl carboxy, where Q5 is H, P.G. is an amino protecting group, and

$R_5$, $R_8$, $R_9$, $R_{10}$, $R_{13}$, and $R_{14}$ is each independently:

deleted when one of fused rings A, B, C, or D is unsaturated so as to complete the valency of the carbon atom at that site, or

selected from the group consisting of hydrogen, hydroxyl, (C1-C10) alkyl, (C1-C10) hydroxyalkyl, (C1-C10) alkyloxy-(C1-C10) alkyl, a substituted or unsubstituted (C1-C10) aminoalkyl wherein the substituents are selected from the group consisting of hydroxyl, protected hydroxyl, amino, protected amino, carboxy, protected carboxy, cyano, methyl sulfonylamino, alkoxy, alkyl, aryl, N-benzyl, acyloxy, nitro, C1-C10 haloalkyl, C2-C6 alkenyl, C2-C6 alkynyl, oxo, a (C1-C10) alkyloxy-(C1-C10) alkyl, linking group attached to a second steroid of formula I, (C1-C10) aminoalkyloxy, (C1-C10) aminoalkylcarboxy, (C1-C10) aminoalkylaminocarbonyl, H2N-HC(Q5)-C(O)-O-, H2N-HC(Q5)-C(O)-N(H)-, (C1-C10) azidoalkyloxy, (C1-C10) cyanoalkyloxy, P.G.-HN-CH(Q5)-C(O)-O-, (C1-C10) guanidinoalkyloxy, and (C1-C10) guanidinoalkylcarboxy, where Q5 is H, P.G. is an amino protecting group, and

provided that at least two of $R_1$ through $R_{14}$ are independently selected from the group consisting of (C1-C10) aminoalkyloxy, (C1-C10) aminoalkylcarboxy, (C1-C10) aminoalkylaminocarbonyl, H2N-HC(Q5) -C(O)-O-, H2N-HC(Q5)-C(O)-N(H)-, (C1-C10) azidoalkyloxy, (C1-C10) cyanoalkyloxy, P.G.-HN-CH(Q5) -C(O)-O-, (C1-C10) guanidinoalkyloxy, and (C1-C10) guanidinoalkylcarboxy;

further provided that the compound has at least one of the following features:

(a) at least one of the following pairs is deleted and the valency of the ring carbon atoms at these deleted positions is completed with a double bond: $R_5$ and $R_9$; $R_8$ and $R_{10}$; and $R_{13}$ and $R_{14}$; or (C1-C10)alkyloxy-(C1-C10)alkyl or a pharmaceutically acceptable salt thereof.

(b) each of $R_3$, $R_7$, and $R_{12}$ is independently selected from the group consisting of -O-C(O)-HC(Q5)-NH2, where Q5 is H; or (c) $R_{17}$ is a (C1-C10)alkyloxy-(C1-C10)alkyl linking group attached to a second steroid and the second steroid is a compound of formula I.

**2.** A compound having the formula:

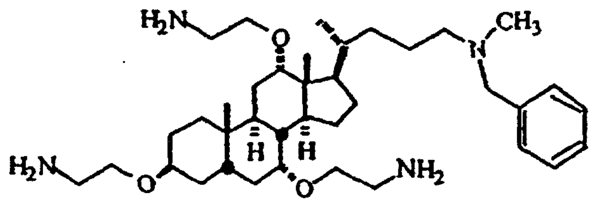

3. A compound having the formula:

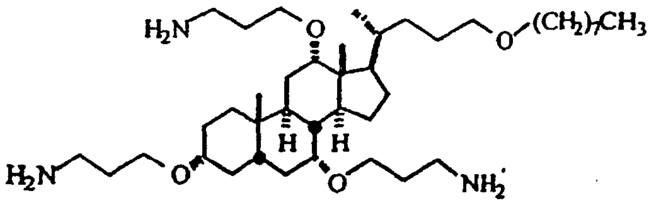

4. A compound having the formula:

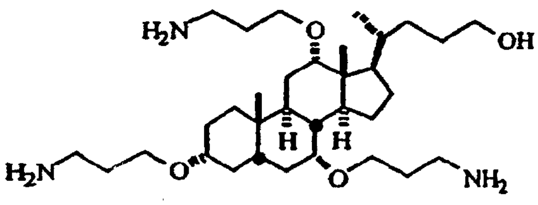

5. A compound having the formula:

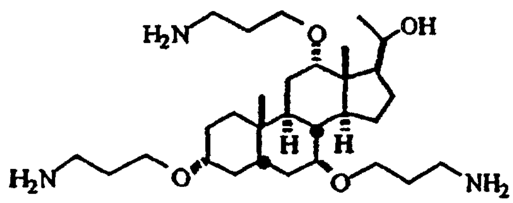

11

6. A compound having the formula:

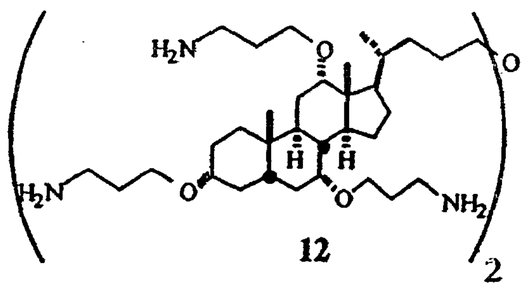

12

7. A method of preparing a compound of formula I as defined in Claim 1 the method comprising:

contacting a compound of formula IV,

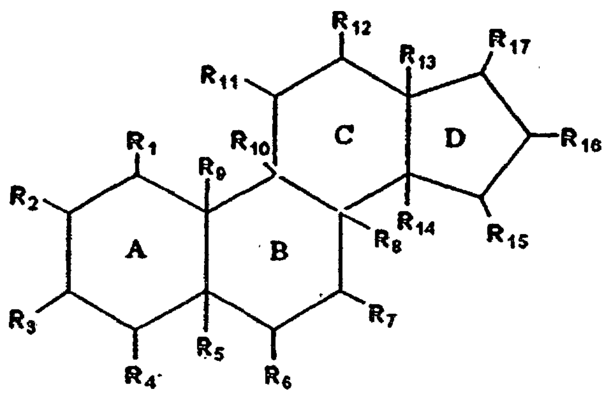

IV

where at least two of $R_1$ through $R_{14}$ are hydroxyl, and the remaining moieties on the fused rings A, B, C, and D are defined for formula I, with a protected amino acid to produce a protected amino acid compound of formula IV where at least two of $R_1$ through $R_{14}$ are (P.G.-HN-HC(Q5)-C(O)-O-), where Q5 is H and P.G. is an amino protecting group; and removing the protecting group of the protected amino acid compound to form a compound of formula I.

8. A pharmaceutical composition comprising an effective amount of a compound of any one of claims 1 to 6.

9. The pharmaceutical composition of claim 8, wherein the composition includes additional antibiotics.

10. Use of a compound of any one of claims 1 to 6 in the manufacture of a medicament for treating a microbial infection of a host.

11. The use of claim 10 in which the host is a human.

12. Use as claimed in claim 10 or claim 11 wherein the compound is in the form of an anti-microbial composition which further comprises a second anti-microbial substance to be delivered into a microbial cell.

13. Use as claimed in claim 12 in which the second anti-microbial substance is an anti-biotic.

14. Use as claimed in any one of claims 10 to 13 in which the infection is a bacterial infection.

15. Use as claimed in claim 14 in which the infection is a gram-negative bacterial infection.

16. Use as claimed in claim 14 in which the bacterial infection is an infection with a bacterium **characterized by** an outer membrane comprising a substantial percentage of lipid A.

17. Use of a compound of any one of claims 1 to 6 in the manufacture of a medicament for enhancing cell permeability,

**18.** Use as claimed in claim 17, wherein the compound is used together with a substance to be introduced into the cell.

**19.** Use as claimed in claim 17 or claim 18, in which the cell is a bacterium.

**20.** Use as claimed in claim 19 in which the bacterium is a gram-negative bacterium.

**21.** Use as claimed in claim 19 in which the bacterium is **characterized by** an outer membrane comprising a substantial percentage of lipid A.

**22.** Use as claimed in claim 17 in which the cell is a sperm cell and the compound is part of a spermicidal composition.

**23.** A method of identifying compounds effective against a microbe comprising administering a candidate compound and a compound according to any one of claims 1 to 6 to the microbe and determining whether the candidate compound has a static or toxic effect on the microbe.

**24.** The method of claim 23 in which the microbe is a bacterium.

**25.** The method of claim 24 in which the bacterium is a gram-negative bacterium.

**26.** The method of claim 24 in which the bacterium is **characterized by** an outer membrane comprising greater than 40% of lipid A.

**27.** A method of microbial growth control comprising contacting a microbe with an effective amount of anti-microbial composition comprising a compound according to any one of claims 1 to 6.

**28.** The method of claim 27 in which the anti-microbial composition further comprises an anti-microbial substance.

**29.** The method of claim 28 in which the antimicrobial substance is a disinfectant, an antibiotic, a disinfectant.

**30.** A composition of matter comprising the compound of any one of claims 1 to 6 in combination with a substance to be introduced into a cell.

**31.** The composition of claim 30 in which the substance to be introduced into a cell is an anti-microbial substance.

**32.** The composition of claim 30 or claim 31 in which the compound and the substance are mixed with a pharmaceutically acceptable carrier.

**33.** The composition of any one of claims 30 to 32 in which the anti-microbial substance is an antibiotic.

**34.** Compound of any one of claims 1 to 6 for use as a medicament.

**Patentansprüche**

**1.** Verbindung gemäß der Formel I

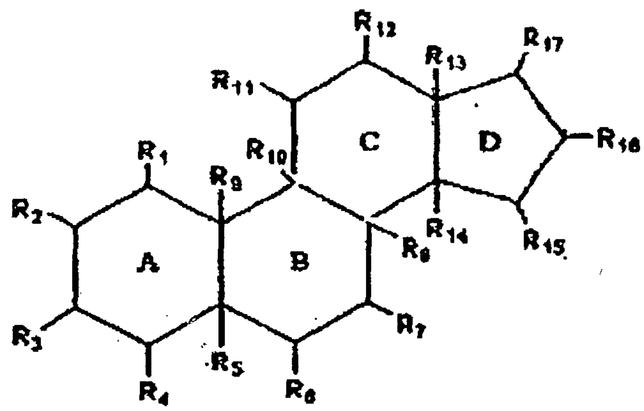

I

worin:

die kondensierten Ringe A, B, C und D unabhängig voneinander gesättigt oder vollständig oder partiell ungesättigt sind; und

$R_1$ bis $R_4$, $R_6$, $R_7$, $R_{11}$, $R_{12}$, $R_{15}$, $R_{16}$ und $R_{17}$ jeweils unabhängig voneinander ausgewählt sind aus der Gruppe von Wasserstoff, Hydroxyl, (C1-C10)-Alkyl, (C1-C10)-Hydroxyalkyl, (C1-C10)-Alkyloxy-(C1-C10)-Alkyl, einem substituierten oder unsubstituierten (C1-C10)-Aminoalkyl, wobei die Substituenten aus der aus Hydroxyl, geschütztem Hydroxyl, Amino, geschütztem Amino, Carboxy, geschütztem Carboxy, Cyano, Methylsulfonylamino, Alkoxy, Alkyl, Aryl, N-Benzyl, Acyloxy, Nitro bestehenden Gruppe ausgewählt sind, (C1-C10)-Halogenalkyl, (C2-C6)-Alkenyl, (C2-C6)-Alkinyl, Oxo, einer verbindenden Gruppe (C1-C10)-Alkyloxy-(C1-C10)Alkyl, die an ein zweites Steroid der Formel I gebunden ist, (C1-C10)-Aminoalkyloxy, (C1-C10)-Aminoalkylcarboxy, (C1-C10)-Aminoalkylaminocarbonyl, $H_2N$-HC(Q5)-C(O)-O-, $H_2N$-HC(Q5)-C(O)-N(H)-, (C1-C10)-Azidoalkyloxy, (C1-C10)-Cyanoalkyloxy, P.G.-HN-CH(Q5)-C(O)-O-, (C1-C10)-Guanidinoalkyloxy und (C1-C10)-Guanidinoalkylcarboxy, wobei Q5 H bedeutet, P.G. eine Aminoschutzgruppe bedeutet, und

$R_5$, $R_8$, $R_9$, $R_{10}$, $R_{13}$ und $R_{14}$ jeweils unabhängig voneinander:

eliminiert sind, wenn einer der kondensierten Ringe A, B, C oder D ungesättigt ist, so dass die Wertigkeit des Kohlenstoffatoms an dieser Stelle erfüllt wird, oder ausgewählt sind aus der Gruppe von Wasserstoff, Hydroxyl, (C1-C10)-Alkyl, (C1-C10)-Hydroxyalkyl, (C1-C10)-Alkyloxy-(C1-C10)-Alkyl, einem substituierten oder unsubstituierten (C1-C10)-Aminoalkyl, wobei die Substituenten aus der aus Hydroxyl, geschütztem Hydroxyl, Amino, geschütztem Amino, Carboxy, geschütztem Carboxy, Cyano, Methylsulfonylamino, Alkoxy, Alkyl, Aryl, N-Benzyl, Acyloxy, Nitro bestehenden Gruppe ausgewählt sind, (C1-C10)-Halogenalkyl, (C2-C6)-Alkenyl, (C2-C6)-Alkinyl, Oxo, einer verbindenden Gruppe (C1-C10)-Alkyloxy-(C1-C10)alkyl, die an ein zweites Steroid der Formel I gebunden ist, (C1-C10)-Aminoalkyloxy, (C1-C10)-Aminoalkylcarboxy, (C1-C10)-Aminoalkylaminocarbonyl, $H_2N$-HC(Q5)-C(O)-O-, $H_2N$-HC(Q5)-C(O)-N(H)-, (C1-C10) -Azidoalkyloxy, (C1-C10)-Cyanoalkyloxy, P.G.-HN-CH(Q5)-C(O)-O-, (C1-C10)-Guanidinoalkyloxy und (C1-C10)-Guanidinoalkylcarboxy,

wobei Q5 H bedeutet, P.G. eine Aminoschutzgruppe bedeutet, und
wobei mindestens zwei Reste von $R_1$ bis $R_{14}$ unabhängig voneinander aus der aus (C1-C10)-Aminoalkyloxy, (C1-C10)-Aminoalkylcarboxy, (C1-C10)-Aminoalkylaminocarbonyl, $H_2N$-HC(Q5)-C(O)-O-, $H_2N$-HC(Q5)-C(O)-N(H)-, (C1-C10)-Azidoalkyloxy, (C1-C10)-Cyanoalkyloxy, P.G.-HN-CH(Q5)-C(O)-O-, (C1-C10)-Guanidinoalkyloxy und (C1-C10)-Guanidinoalkylcarboxy bestehenden Gruppe ausgewählt sind;
wobei ferner die Verbindung mindestens eines der folgenden Merkmale aufweist:

(a) mindestens eines der im Folgenden angegebenen Paare ist eliminiert und die Wertigkeit der Ringkohlenstoffatome an diesen eliminierten Positionen wird durch eine Doppelbindung erfüllt: $R_5$ und $R_9$; $R_8$ und $R_{10}$; und $R_{13}$ und $R_{14}$; oder
(b) jeder der Reste $R_3$, $R_7$ und $R_{12}$ ist unabhängig voneinander aus der aus -Q-C(O)-HC(Q5)-NH2, wobei Q5

H ist, bestehenden Gruppe ausgewählt; oder

(c) $R_{17}$ ist eine verbindende Gruppe (C1-C10)Alkyloxy-(C1-C10)alkyl, die an ein zweites Steroid gebunden ist, und das zweite Steroid ist eine Verbindung der Formel I;

oder ein pharmazeutisch akzeptables Salz derselben.

**2.** Verbindung der Formel:

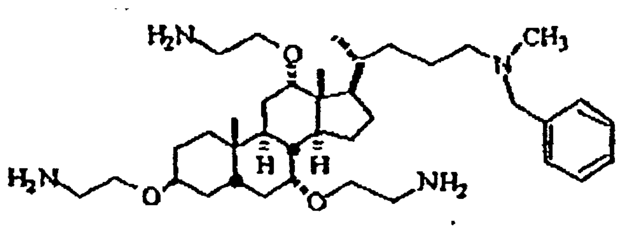

**3.** Verbindung der Formel:

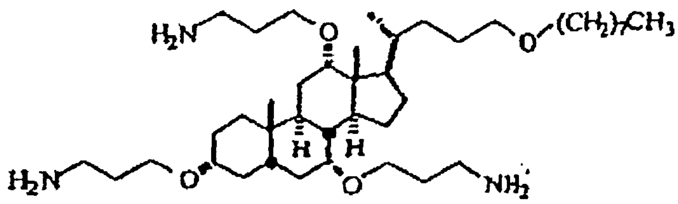

**4.** Verbindung der Formel:

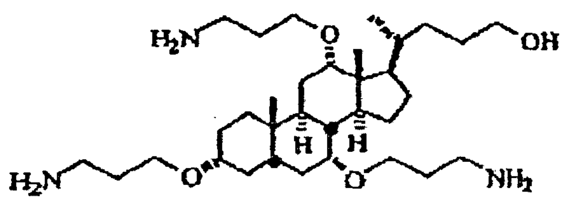

**5.** Verbindung der Formel:

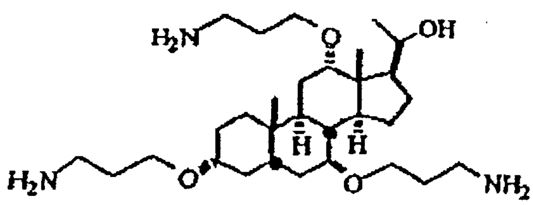

11

**6.** Verbindung der Formel:

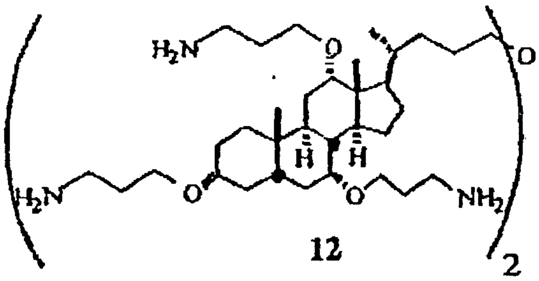

12

7. Verfahren zur Herstellung einer Verbindung der Formel I gemäß der Definition in Anspruch 1, wobei das Verfahren umfasst:

Kontaktieren einer Verbindung der Formel IV

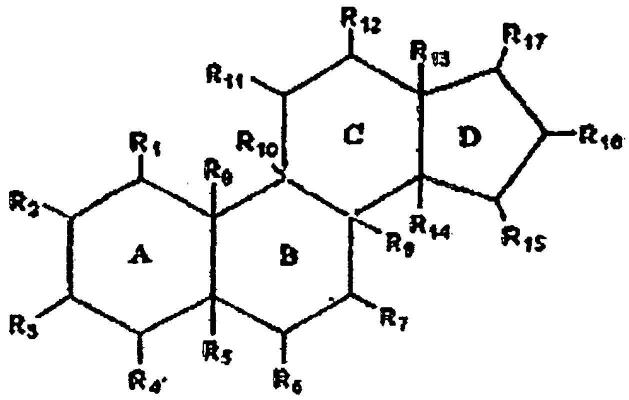

IV

worin mindestens zwei Reste von $R_1$ bis $R_{14}$ Hydroxyl sind, und die übrigen Einheiten an den kondensierten Ringen A, B, C und D wie für Formel I definiert sind, mit einer geschützten Aminosäure unter Bildung einer geschützten Aminosäureverbindung der Formel IV, worin mindestens zwei Reste von $R_1$ bis $R_{14}$ (P.G.-HN-HC(Q5)-C(O)-O-), wobei Q5 H ist und P.G. eine Aminoschutzgruppe ist, bedeuten; und
Entfernen der Schutzgruppe der geschützten Aminosäureverbindung unter Bildung einer Verbindung der Formel I.

8. Pharmazeutische Zusammensetzung, die eine wirksame Menge einer Verbindung nach einem der Ansprüche 1 bis 6 umfasst.

9. Pharmazeutische Zusammensetzung nach Anspruch 8, wobei die Zusammensetzung zusätzliche Antibiotika umfasst.

10. Verwendung einer Verbindung nach einem der Ansprüche 1 bis 6 zur Herstellung eines Medikaments zur Behandlung einer mikrobiellen Infektion eines Wirts.

11. Verwendung nach Anspruch 10, wobei der Wirt ein Mensch ist.

12. Verwendung nach Anspruch 10 oder Anspruch 11, wobei die Verbindung in der Form einer antimikrobiellen Zusammensetzung vorliegt, die ferner eine zweite in eine Mikrobenzelle abzugebende antimikrobielle Substanz umfasst.

13. Verwendung nach Anspruch 12, wobei die zweite antimikrobielle Substanz ein Antibiotikum ist.

14. Verwendung nach einem der Ansprüche 10 bis 13, wobei die Infektion eine Bakterieninfektion ist.

**15.** Verwendung nach Anspruch 14, wobei die Infektion eine Infektion gramnegativer Bakterien ist.

**16.** Verwendung nach Anspruch 14, wobei die Bakterieninfektion eine Infektion mit einem Bakterium ist, das durch eine äußere Membran, die einen wesentlichen Prozentsatz an Lipid A umfasst, gekennzeichnet ist,

**17.** Verwendung einer Verbindung nach einem der Ansprüche 1 bis 6 zur Herstellung eines Medikaments zur Verstärkung der Zellpermeabilität.

**18.** Verwendung nach Anspruch 17, wobei die Verbindung zusammen mit einer in die Zelle einzuführenden Substanz verwendet wird.

**19.** Verwendung nach Anspruch 17 oder 18, wobei die Zelle ein Bakterium ist.

**20.** Verwendung nach Anspruch 19, wobei das Bakterium ein gramnegatives Bakterium ist.

**21.** Verwendung nach Anspruch 19, wobei das Bakterium durch eine äußere Membran, die einen wesentlichen Prozentsatz an Lipid A umfasst, gekennzeichnet ist.

**22.** Verwendung nach Anspruch 17, wobei die Zelle ein Spermium ist und die Verbindung Teil einer spermiziden Zusammensetzung ist.

**23.** Verfahren der Identifizierung von gegenüber einer Mikrobe wirksamen Verbindungen, das das Verabreichen einer Kandidatenverbindung und einer Verbindung nach einem der Ansprüche 1 bis 6 an die Mikrobe und das Bestimmen, ob der Verbindungskandidat statische oder toxische Wirkung auf die Mikrobe hat, umfasst.

**24.** Verfahren nach Anspruch 23, wobei die Mikrobe ein Bakterium ist.

**25.** Verfahren nach Anspruch 24, wobei das Bakterium ein gramnegatives Bakterium ist.

**26.** Verfahren nach Anspruch 24, wobei das Bakterium durch eine äußere Membran, die zu mehr als 40 % Lipid A umfasst, gekennzeichnet ist.

**27.** Verfahren der Bekämpfung von Mikrobenwachstum, das das Kontaktieren einer Mikrobe mit einer wirksamen Menge einer antimikrobiellen Zusammensetzung, die eine Verbindung nach einem der Ansprüche 1 bis 6 enthält, umfasst.

**28.** Verfahren nach Anspruch 27, wobei die antimikrobielle Zusammensetzung ferner eine antimikrobielle Substanz umfasst.

**29.** Verfahren nach Anspruch 28, wobei die antimikrobielle Substanz ein Desinfektionsmittel, ein Antibiotikum, ein keimtötendes Mittel ist.

**30.** Substanzzusammensetzung, die die Verbindung nach einem der Ansprüche 1 bis 6 in Kombination mit einer in eine Zelle einzuführenden Substanz umfasst.

**31.** Zusammensetzung nach Anspruch 30, wobei die in eine Zelle einzuführende Substanz eine antimikrobielle Substanz ist.

**32.** Zusammensetzung nach Anspruch 30 oder Anspruch 31, wobei die Verbindung und die Substanz mit einem pharmazeutisch akzeptablen Träger gemischt sind.

**33.** Zusammensetzung nach einem der Ansprüche 30 bis 32, wobei die antimikrobielle Substanz ein Antibiotikum ist.

**34.** Verbindung nach einem der Ansprüche 1 bis 6 zur Verwendung als Medikament.

## Revendications

**1.** Composé selon la formule I :

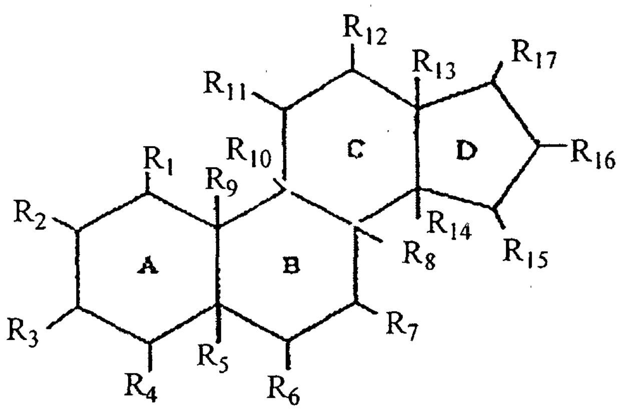

I

dans laquelle :

les cycles fusionnés A, B, C et D sont indépendamment saturés ou bien totalement ou partiellement insaturés ; et

$R_1$ à $R_4$, $R_6$, $R_7$, $R_{11}$, $R_{12}$, $R_{15}$, $R_{16}$ et $R_{17}$ sont chacun choisis indépendamment dans le groupe constitué par un atome d'hydrogène, un groupe hydroxyle, alkyle en $C_1$ à $C_{10}$, hydroxyalkyle en $C_1$ à $C_{10}$, alkyl($C_1$ à $C_{10}$) oxy-alkyle en $C_1$ à $C_{10}$,, un groupe substitué ou non substitué aminoalkyle en $C_1$ à $C_{10}$, dans lequel les substituants sont choisis dans le groupe constitué par un groupe hydroxyle, hydroxyle protégé, amino, amino protégé, carboxy, carboxy protégé, cyano, sulfonylaminométhyl, alcoxy, alkyle, aryle, N-benzyle, acyloxy, nitro, halogéno-alkyle en $C_1$ à $C_{10}$, alcényle en $C_2$ à $C_6$, alcynyle en $C_2$ à $C_6$, oxo, un groupe de liaison alkyl($C_1$ à $C_{10}$)oxy-alkyle en $C_1$ à $C_{10}$ fixé à un second stéroïde de formule I, aminoalkyl($C_1$ à $C_{10}$)oxy, aminoalkyl($C_1$ à $C_{10}$) carboxy, aminoalkyl($C_1$ à $C_{10}$) aminocarbonyle, H2N-HC(Q5)-C(O)-O-, H2N-HC(Q5)-C(O)-N(H)-, azidoalkyl($C_1$ à $C_{10}$)oxy, cyanoalkyl($C_1$ à $C_{10}$)oxy, P.G.-HN-CH(Q5)-C(O)-O-, guanidino-alkyl($C_1$ à $C_{10}$)oxy et guanidino-alkyl($C_1$ à $C_{10}$)carboxy, où Q5 est H, P. G. est un groupe protecteur d'amino et

$R_5$, $R_9$, $R_{10}$, $R_{10}$, $R_{13}$ et $R_{14}$ sont chacun indépendamment :

supprimés lorsque l'un des cycles fusionnés A, B, C ou D est insaturé de manière à compléter la valence de l'atome de carbone au niveau de ce site ou

choisis dans le groupe constitué par un atome d'hydrogène, un groupe hydroxyle, alkyle en $C_1$ à $C_{10}$, hydroxyalkyle en $C_1$ à $C_{10}$, alkyl($C_1$ à $C_{10}$)oxy-alkyle en $C_1$ à $C_{10}$, un groupe substitué ou non substitué aminoalkyle en $C_1$ à $C_{10}$, dans lequel les substituants sont choisis dans le groupe constitué par un groupe hydroxyle, hydroxyle protégé, amino, amino protégé, carboxy, carboxy protégé, cyano, méthylsulfonylamino, alcoxy, alkyle, aryle, N-benzyle, acyloxy, nitro, halogéno-alkyle en $C_1$ à $C_{10}$, alcényle en $C_2$ à $C_6$, alcynyle en $C_2$ à $C_6$, oxo, un groupe de liaison alkyl($C_1$ à $C_{10}$)oxy-alkyle en $C_1$ à $C_{10}$ fixé à un second stéroïde de formule I, aminoalkyl($C_1$ à $C_{10}$)oxy, aminoalkyl ($C_1$ à $C_{10}$)carboxy, aminoalkyl($C_1$ à $C_{10}$)aminocarbonyle, H2N-HC(Q5)-C(O)-O-, H2N-HC(Q5)-C(O)-N(H)-, azidoalkyl($C_1$ à $C_{10}$)oxy, cyanoalkyl($C_1$ à $C_{10}$)oxy, P.G.-HN-CH(Q5)-C(O)-O-, guanidino-alkyl($C_1$ à $C_{10}$)oxy et guanidino-alkyl($C_1$ à $C_{10}$)carboxy, où Q5 est H, P. G. est un groupe protecteur d'amino et

à condition qu'au moins deux de $R_1$ à $R_{14}$ soient choisis indépendamment dans le groupe constitué par un groupe aminoalkyl($C_1$ à $C_{10}$)oxy, aminoalkyl($C_1$ à $C_{10}$)carboxy, aminoalkyl($C_1$ à $C_{10}$) aminocarbonyle, H2N-HC(Q5)-C(O)-O-, H2N-HC(Q5)-C(O)-N(H)-, azidoalkyl($C_1$ à $C_{10}$)oxy, cyanoalkyl($C_1$ à $C_{10}$)oxy, P.G. -HN-CH(Q5)-C(O)-O-, guanidino-alkyl($C_1$ à $C_{10}$)oxy et guanidino-alkyl($C_1$ à $C_{10}$)carboxy ;

à la condition encore que le composé ait au moins l'une des caractéristiques suivantes: (a) au moins l'une des paires suivantes est supprimée et la valence des atomes de carbone des cycles au niveau de ces positions supprimées est complétée par une double liaison : $R_5$ et $R_9$ ; $R_8$ et $R_{10}$ ; et $R_{13}$ et $R_{14}$ ; ou (b) chacun de $R_3$, $R_7$ et $R_{12}$ est choisi indépendamment dans le groupe constitué par -O-C(O)-HC(Q5)-NH2, où Q5 est H ; ou (c) $R_{17}$ est un groupe de liaison alkyl($C_1$ à $C_{10}$)oxy-alkyle en $C_1$ à $C_{10}$ fixé à un second stéroïde et le second stéroïde est un composé de formule I,

ou un sel pharmaceutiquement acceptable de celui-ci.

**2.** Composé ayant la formule :

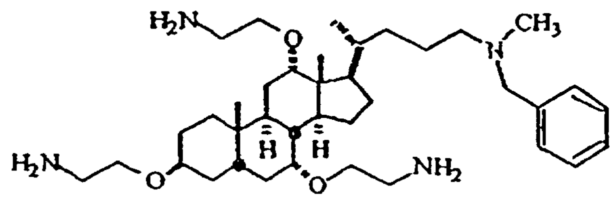

**3.** Composé ayant la formule :

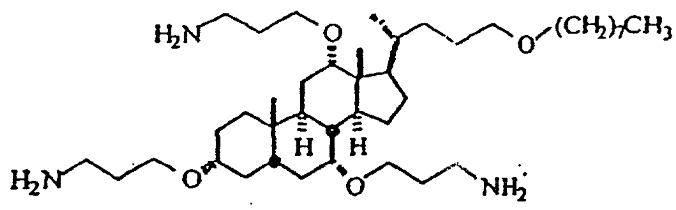

**4.** Composé ayant la formule :

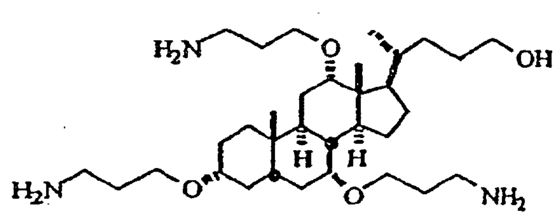

**5.** Composé ayant la formule :

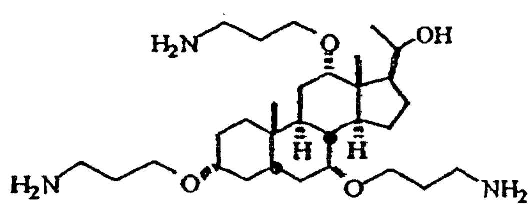

11

**6.** Composé ayant la formule :

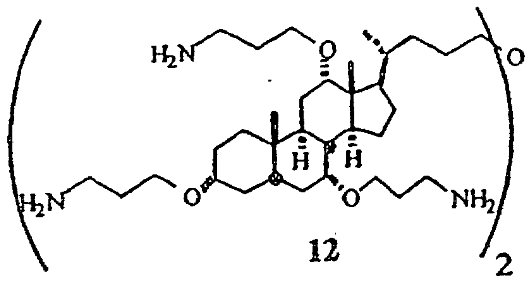

12

7. Procédé de préparation d'un composé de formule I tel que défini dans la revendication 1, le procédé comprenant :

la mise en contact d'un composé de formule IV,

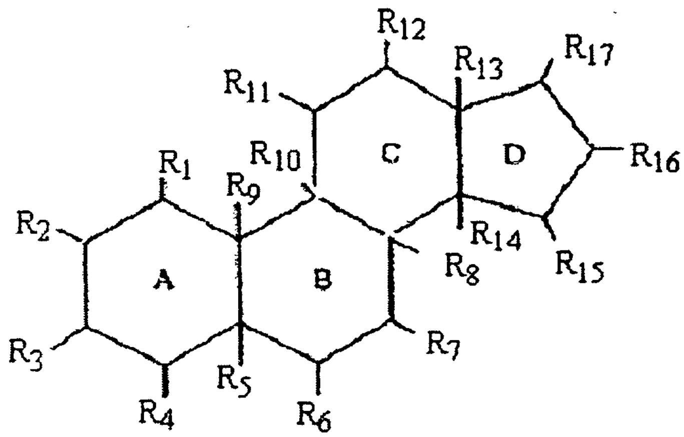

IV

où au moins deux de $R_1$ à $R_{14}$ sont un groupe hydroxyle et les radicaux restants sur les cycles fusionnés A, B, C et D sont définis pour la formule I, avec un acide aminé protégé pour produire un composé d'acide aminé protégé de formule IV où au moins deux de $R_1$ à $R_{14}$ sont (P.G.-HN-HC(Q5)-C(O)-O-), où Q est H et P.G. est un groupe protecteur d'amino ; et

l'élimination du groupe protecteur du composé d'acide aminé protégé pour former un composé de formule I.

8. Composition pharmaceutique comprenant une quantité efficace d'un composé selon l'une quelconque des revendications 1 à 6.

9. Composition pharmaceutique selon la revendication 8, dans laquelle la composition comprend des antibiotiques additionnels.

10. Utilisation d'un composé selon l'une quelconque des revendications 1 à 6 dans la fabrication d'un médicament destiné au traitement d'une infection microbienne d'un hôte.

11. Utilisation selon la revendication 10, dans laquelle l'hôte est un homme.

12. Utilisation selon la revendication 10 ou la revendication 11, dans laquelle le composé est sous la forme d'une composition antimicrobienne qui comprend en outre une seconde substance antimicrobienne à délivrer dans une cellule microbienne.

13. Utilisation selon la revendication 12, dans laquelle la seconde substance antimicrobienne est un antibiotique.

14. Utilisation selon l'une quelconque des revendications 10 à 13, dans laquelle l'infection est une infection bactérienne.

**15.** Utilisation selon la revendication 14, dans laquelle l'infection est une infection à bactérie Gram négatif.

**16.** Utilisation selon la revendication 14, dans laquelle l'infection bactérienne est une infection avec une bactérie **caractérisée par** une membrane externe comprenant un pourcentage substantiel de lipide A.

**17.** Utilisation d'un composé selon l'une quelconque des revendications 1 à 6 dans la fabrication d'un médicament destiné à l'augmentation de la perméabilité cellulaire.

**18.** Utilisation selon la revendication 17, dans laquelle le composé est utilisé conjointement avec une substance à introduire dans la cellule.

**19.** Utilisation selon la revendication 17 ou la revendication 18, dans laquelle la cellule est une bactérie.

**20.** Utilisation selon la revendication 19, dans laquelle la bactérie est une bactérie Gram négatif.

**21.** Utilisation selon la revendication 19, dans laquelle la bactérie est **caractérisée par** une membrane externe comprenant un pourcentage substantiel de lipide A.

**22.** Utilisation selon la revendication 17, dans laquelle la cellule est une cellule spermatique et le composé fait partie d'une composition spermicide.

**23.** Procédé d'identification de composés efficaces contre un microbe comprenant l'administration d'un composé candidat et d'un composé selon les revendications 1 à 6 au microbe et la détermination de si le composé candidat a un effet statique ou toxique sur le microbe.

**24.** Procédé selon la revendication 23, dans lequel le microbe est une bactérie.

**25.** Procédé selon la revendication 24, dans lequel la bactérie est une bactérie Gram négatif.

**26.** Procédé selon la revendication 24, dans lequel la bactérie est **caractérisée par** une membrane externe comprenant plus de 40% de lipide A.

**27.** Procédé de contrôle de la croissance microbienne comprenant la mise en contact d'un microbe avec une quantité efficace d'une composition antimicrobienne comprenant un composé selon l'une quelconque des revendications 1 à 6.

**28.** Procédé selon la revendication 27, dans lequel la composition antimicrobienne comprend en outre une substance antimicrobienne.

**29.** Procédé selon la revendication 28, dans lequel la substance antimicrobienne est un désinfectant, un antibiotique, un désinfectant.

**30.** Composition de matière comprenant le composé selon l'une quelconque des revendications 1 à 6 en combinaison avec une substance à introduire dans une cellule.

**31.** Composition selon la revendication 30, dans ,laquelle la substance à introduire dans une cellule est une substance antimicrobienne.

**32.** Composition selon la revendication 30 ou la revendication 31, dans laquelle le composé et la substance sont mélangés avec un vecteur pharmaceutiquement acceptable.

**33.** Composition selon l'une quelconque des revendications 30 à 32, dans laquelle la substance antimicrobienne est un àntibiotique.

**34.** Composé selon l'une quelconque des revendications 1 à 6 pour une utilisation en tant que médicament.